(19) Europäisches Patentamt
European Patent Office
Office européen des brevets

(11) Veröffentlichungsnummer: **0 169 812**
**B1**

(12) # EUROPÄISCHE PATENTSCHRIFT

(45) Veröffentlichungstag der Patentschrift: **23.08.89**

(51) Int. Cl.⁴: **C 07 F 9/09**, C 07 F 9/10, A 61 K 31/66

(21) Anmeldenummer: **85810332.8**

(22) Anmeldetag: **19.07.85**

(54) **Phosphatidylverbindungen, Verfahren zu ihrer Herstellung und ihre Verwendung.**

(30) Priorität: **25.07.84 CH 3598/84**

(43) Veröffentlichungstag der Anmeldung: **29.01.86 Patentblatt 86/5**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 072 111**
**EP-A-0 072 286**
**EP-A-0 099 578**
**CH-A-641 811**
**US-A-4 235 792**

**CHEMICAL ABSTRACTS, Band 66, Nr. 21, 22. Mai 1967, Seite 8957, Abstrakt Nr. 95388g, Columbus, Ohio, US; A. MIROPOL'SKAYA et al.: "Synthesis of phosphatidopeptides", & DOKL. AKAD. NAUK SSSR 1966, 171(3), 627-629**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Baschang, Gerhard, Dr., Bückenweg 7, CH- 4126 Bettingen (CH)**
Erfinder: **Fechtig, Bruno, Dr., Hinterlindenweg 1, CH- 4153 Reinach (CH)**
Erfinder: **Hartmann, Albert, Dr., Steingasse 21A, D-7889 Grenzach (DE)**
Erfinder: **Lukas, Bohumir, Dr., Rheinfelderstrasse 21, CH- 4058 Basel (CH)**
Erfinder: **Wacker, Oskar, Dr., Löwenbergstrasse 60, CH- 4059 Basel (CH)**

**EP 0 169 812 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft Phosphatidylverbindungen, diese enthaltende pharmazeutische Präparate, ihre Verwendung zur Herstellung von Arzneimitteln zur Prophylaxe und Therapie von Virusinfektionen und Verfahren zu ihrer Herstellung.

In Chem. Abstracts 66, Seite 8957, Referat Nr. 95388g (1967) wird die Herstellung von Glycin-2-(1,2-dipalmitoyl-sn-glycero-3-hydroxyphosphoryloxy)-ethylamid und dem Alanin-Analogen davon beschrieben.

Die Erfindung betrifft Phosphatidylverbindungen der Formel I,

```
             O  W
             ‖  |
R¹-T-Y-O-P-O-CH                 (I)
             |  |
             OH Z
```

worin

$R^1$ $C_{3-14}$-Alkanoyl, Benzoyl, den Acylrest einer α-Amino-carbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxy-ethyl-, 2-Hydroxy-ethyl- oder Hydroxy-methylgruppe, in der mindestens eine der Hydroxygruppen mit einer aliphatischen $C_{8-30}$-Carbonsäure verestert oder mit einem aliphatischen $C_{8-30}$-Alkohol verethert ist,

oder W und Z je eine Hydroxymethylgruppe, die mit einer aliphatischen $C_{8-30}$-Carbonsäure verestert oder mit einem aliphatischen $C_{8-30}$-Alkohol verethert ist, bedeuten,

und ihre Salze sowie Verfahren zu ihrer Herstellung.

Die Erfindung betrifft weiter die obengenannten neuen Verbindungen der Formel I zuzüglich bestimmter Verbindungen, die zwar zum Stand der Technik gehören, deren Anwendung in einem Verfahren zur therapeutischen Behandlung des menschlichen oder tierischen Körpers aber nicht zum Stand der Technik gehört, d.h. Phosphatidylverbindungen der Formel Ia,

```
              O  W
              ‖  |
R¹ᵃ-T-Y-O-P-O-CH                (Ia)
              |  |
              OH Z
```

worin

$R^{1a}$ $C_{2-22}$-Alkanoyl, das durch Carboxy substituiert sein kann, Propenoyl, 2-Methyl-propenoyl, Benzoyl, den Acylrest einer α-Amino-carbonsäure, deren α-Amino-Gruppe durch Niederalkyl, Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure bedeutet, und

die übrigen Substituenten die obengenannten Bedeutungen haben, und ihre Salze zur Anwendung in einem Verfahren zur therapeutischen Behandlung des menschlichen oder tierischen Körpers und zur Herstellung von pharmazeutischen Präparaten sowie zur industriellen Herstellung und Konfektionierung eines Arzneimittels mit antiviraler Wirkung.

Die Erfindung betrifft ferner die Verwendung der obengenannten Verbindungen der Formeln I und Ia zuzüglich derjenigen, worin $R^{1a}$ für $C_3$-$C_{18}$-Alkenoyl steht, zur Prophylaxe und Therapie von Virusinfektionen.

$C_{3-14}$-Alkanoyl $R^1$ ist insbesondere geradkettiges $C_{3-14}$-Alkanoyl, z. B. Propionyl, n-Butyryl, Lauroyl oder Myristoyl.

Der Acylrest $R^1$ einer α-Amino-carbonsäure, die von Glycin, L-Alanin und deren Derivaten mit substituierter Aminogruppe verschieden ist, ist insbesondere von Glycin, L-Alanin und deren Derivaten mit substituierter Aminogruppe verschiedenes α-Amino-niederalkanoyl, das durch eine weitere Amino-, Niederalkanoylamino-, Niederalkoxycarbonylamino- oder Benzyloxycarbonylaminogruppe oder durch Guanidino, Carboxy, Phenyloxycarbonyl, Benzyloxycarbonyl, Benzhydryloxycarbonyl, Niederalkoxycarbonyl, Carbamoyl, Hydroxy, Niederalkoxy, Niederalkanoyloxy, Niederalkoxycarbonyloxy, Mercapto, Niederalkylthio, (2-Amino-2-carboxy-ethyl)-dithio, Phenyl, 4-Hydroxy-phenyl, Imidazol-4-yl oder Indol-3-yl substituiert sein kann, oder unsubstituiertes oder durch Hydroxy, insbesondere γ-Hydroxy, Niederalkoxy, Niederalkanoyloxy oder Niederalkoxycarbonyloxy substituiertes Pyrrolidin-α-carbonyl. Vorzugsweise leitet er sich von einer in der Natur vorkommenden (L)-α-Aminocarbonsäure, mit Ausnahme von Glycin und L-Alanin, oder von deren (D)-Isomeren ab, insbesondere von L-Valin, L-Isoleucin, L-Phenylalanin, L-Threonin, L-Cystein, L-Methionin, L-Tryptophan, L-Glutamin, L-Isoglutamin, L-Arginin, L-γ-Hydroxy-prolin oder in allererster Linie von L-Tyrosin, L-Lysin, L-Asparaginsäure, L-Asparagin, L-Histidin, L-Glutaminsäure, L-Serin, L-Prolin, L-Leucin oder D-Alanin.

Somit kann $R^1$ z. B. den Acylrest von L-Tyrosin oder von N-Niederalkoxycarbonyl- oder N-Niederalkanoyl-L-Tyrosin, den Acylrest von L-Leucin oder von N-Niederalkoxycarbonyl- oder N-Niederalkanoyl-L-leucin, den Acylrest von L-Lysin oder von $N^{\alpha}$-Benzyloxycarbonyl-$N^{\varepsilon}$-niederalkoxycarbonyl-L-lysin, den Acylrest von L-Glutaminsäure oder von N-Benzyloxycarbonyl-L-glutaminsäure-α- oder -γ-niederalkylester, den Acylrest von L-Serin oder von N-Niederalkanoyl- oder N-Niederalkoxycarbonyl-L-serin oder den Acylrest von L-Prolin oder von N-Niederalkoxycarbonyl-L-prolin bedeuten.

Der Acylrest $R^1$ einer β-Amino-carbonsäure ist vorzugsweise β-Aminoniederalkanoyl, insbesondere der Acylrest von β-Alanin.

Der Acylrest einer α- oder β-Hydroxy-carbonsäure ist insbesondere α- oder β-Hydroxy-niederalkanoyl, das durch Carboxy und/oder Hydroxy substituiert sein kann, und leitet sich vorzugsweise von einer in der Natur vorkommenden α- oder β-Hydroxy-carbonsäure, wie hauptsächlich Milchsäure und vor allem Citronensäure, ab.

Die Carboxylgruppe als Substituent des Restes Y befindet sich vorzugsweise in α-Stellung zum Rest T. Die Konfiguration an dem diese Carboxylgruppe tragenden C-Atom ist hauptsächlich (L).

Verethertes Carboxy als Substituent von Y ist in erster Linie Niederalkoxycarbonyl oder Benzyloxycarbonyl. Amidiertes Carboxy als Substituent von Y ist z. B. Carbamoyl.

In einem 1,2-Dihydroxy-ethyl-Rest Z ist z. B. nur die endständige, d.h. die 2-Hydroxy-Gruppe, oder sind vorzugsweise beide Hydroxygruppen verethert oder vorzugsweise verestert.

Eine aliphatische $C_{8-38}$-Carbonsäure hat vorzugsweise eine gerade Anzahl von C-Atomen, in erster Linie 10 - 24, z. B. 12 - 24, 16 - 24 oder 16 - 22, hauptsächlich 12 - 18 C-Atome, ist verzweigt oder vorzugsweise geradkettig und ist insbesondere eine substituierte oder vorzugsweise unsubstituierte Alkan- oder Alkensäure, wobei letztere vorzugsweise 18 C-Atome hat und 1 - 3 isolierte Doppelbindungen enthält, z. B. eine in der Natur vorkommende Fettsäure, wie insbesondere Palmitin-, Öl- oder Stearinsäure, oder auch Linol- oder Linolensäure. Daneben seien als Beispiele für eine aliphatische $C_{8-30}$-Carbonsäure Caprin-, Laurin-, Nervon- oder Lignocerinsäure genannt.

Ein aliphatischer $C_{8-30}$-Alkohol hat vorzugsweise eine gerade Anzahl von C-Atomen, in erster Linie 12 - 24, vor allem 16 - 24, hauptsächlich 16 - 22, C-Atome, ist verzweigt oder vorzugsweise geradkettig und ist insbesondere ein substituiertes oder in erster Linie unsubstituiertes Alkanol oder Alkenol, wobei als Substituenten z. B. Hydroxy, Amino, Alkanoylamino oder Alkenoylamino genannt seien.

$C_{2-22}$-Alkanoyl $R^{1a}$ ist insbesondere geradkettiges $C_{2-22}$-Alkanoyl, vor allem geradkettiges $C_{2-18}$-Alkanoyl, z. B. Acetyl, Propionyl, n-Butyryl, Lauroyl, Myristoyl, Palmitoyl oder Stearoyl. Monomethylamino, Trimethylamino oder Carboxy substituiertes Alkanoyl $R^{1a}$ ist vorzugsweise entsprechend substituiertes Niederalkanoyl, z. B. der Acylrest von Sarkosin, Betain, Bernsteinsäure oder Glutarsäure.

$C_{3-18}$-Alkenoyl $R^{1a}$ ist z. B. Propenoyl oder 2-Methyl-propenoyl.

Der Acylrest einer α-Aminocarbonsäure oder einer α- oder β-Hydroxycarbonsäure $R^{1a}$ ist definiert, wie oben für $R^1$ angegeben, und kann zusätzlich den Acylrest von Glycin, L-Alanin oder von deren Derivaten mit durch Niederalkyl, Niederalkanoyl, Niederalkoxycarbonyl oder Benzyloxycarbonyl substituierter α-Aminogruppe bedeuten. Ein Glycinderivat mit durch Niederalkyl substituierter Aminogruppe ist z. B. Betain.

Die vor- oder nachstehend verwendeten Allgemeinbegriffe haben, wenn nicht für den spezifischen Fall anders angegeben, vorzugsweise die folgenden Bedeutungen:

Das vor- und nachstehend verwendete Präfix "Nieder" bezeichnet Reste bis und mit 7, insbesondere bis und mit 4, Kohlenstoffatomen.

Niederalkanoyl ist z. B. Propionyl, Butyryl oder Hexanoyl, in erster Linie Acetyl.

Niederalkyl ist z. B. n-Propyl, n-Butyl, Isobutyl, sek.-Butyl, tert.-Butyl, ferner n-Pentyl, n-Hexyl oder n-Heptyl, in erster Linie Methyl, Ethyl oder Isopropyl.

Verestertes Hydroxy ist durch eine organische Carbonsäure oder eine starke anorganische Säure, z. B. eine Mineralsäure, verestertes Hydroxy, insbesondere Halogen oder, vorzugsweise, Niederalkanoyloxy.

Verethertes Carboxy ist insbesondere Niederalkoxycarbonyl oder jeweils im aromatischen Ring unsubstituiertes oder substituiertes Phenyloxycarbonyl, Benzyloxycarbonyl oder Benzhydryloxycarbonyl.

Amidiertes Carboxy ist insbesondere Carbamoyl oder unsubstituiertes oder im Niederalkylteil durch Carboxy oder Niederalkoxycarbonyl substituiertes N-Niederalkyl-Carbamoyl.

Halogen ist insbesondere Chlor, Brom oder Iod oder, vorzugsweise als Phenylsubstituent, Fluor.

Niederalkoxy ist z. B. Ethoxy, Propoxy oder, vorzugsweise, Methoxy sowie in bestimmten Fällen tert.-Butoxy.

Da das über Sauerstoff an das Phosphoratom gebundene Proton sauer ist, bilden die Verbindungen der Formel I leicht Salze. Die Verbindungen der Formel I, die eine basische Gruppe, z. B. Amino, enthalten, können bei entsprechendem pH-Wert als innere Salze, d.h. in zwitterionischer Form, vorliegen. Die Verbindungen der Formel I, die mehr als eine basische Gruppe enthalten, können zusätzlich Säureadditionssalze mit externen Säuren, z. B. mit anorganischen Säuren, wie Mineralsäuren, z. B. Salzsäure, Schwefelsäure oder Phosphorsäure, oder mit geeigneten organischen Carbon- oder Sulfonsäuren, z. B. Trifluoressig-, Essig-, Malein-, Fumar-, Wein-, Zitronen-, Methansulfon- oder 4-Toluolsulfonsäure, sowie mit Aminosäuren, wie Arginin und Lysin, bilden.

Verbindungen der Formel I, die keine basische Gruppe enthalten oder die mehr saure als basische Gruppen enthalten, z. B. wenn neben der Phosphorsäuregruppe noch Carboxylgruppen im Molekül vorhanden sind, können Metall- oder Ammoniumsalze bilden, wie Alkalimetall- und Erdalkalimetall-, z. B. Natrium-, Kalium-,

Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z. B. Triethylamin, Hydroxyniederalkylamine, z. B. 2-Hydroxyethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z. B. 4-Aminobenzoesäure-2-diethylaminoethylester, Niederalkylenamine, z. B. 1-Ethylpiperidin, Cycloalkylamine, z. B. Dicyclohexylamin, oder Benzylamine, z. B. N,N'-Dibenzylethylendiamin, ferner Basen vom Pyridintyp, z. B. Pyridin, Collidin oder Chinolin.

Zur Isolierung oder Reinigung können auch pharmazeutisch ungeeignete Salze Verwendung finden. Zur therapeutischen Anwendung gelangen jedoch nur die pharmazeutisch verwendbaren, nicht toxischen Salze, die deshalb bevorzugt werden.

Die neuen Verbindungen der vorliegenden Erfindung weisen wertvolle pharmakologische Eigenschaften auf.

Erfindungsgemäß wurde überraschenderweise gefunden, daß die obengenannten Phosphatidylverbindungen der Formel I und ihre pharmazeutisch verwendbaren Salze sowohl zur Prophylaxe als auch zur Therapie von Virusinfektionen hervorragend geeignet sind, wie sich z. B. aus Tierversuchen, wie sie im Beispielteil exemplifiziert sind, ergibt. In diesen Tierversuchen werden Tiere, wie Mäuse oder Meerschweinchen, mit den verschiedensten Virusarten in einer Dosis, die für alle oder die große Mehrzahl der unbehandelten (Kontroll)-Tiere letal ist, z. B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Verbindungen oder einem Salz davon behandelt werden.

Dabei zeigt sich, daß ein prophylaktischer Effekt bei Verabreichung der Verbindungen der Formel I schon mehrere Tage bis zu einigen, z. B. vier, Wochen vor der Infektion, und ein therapeutischer Effekt noch bei Verabreichung mehrere Tage, z. B. 1 Woche, nach der Infektion, eintritt.

Bemerkenswert ist auch das breite virale Spektrum, gegen das die obengenannten Verbindungen wirksam sind.

Die Verbindungen der Formel I können insbesondere zur Prophylaxe und Therapie von Krankheiten verwendet werden, die durch die nachstehend näher bezeichneten Viren hervorgerufen werden [zur Nomenklatur vgl. J.L. Melnick, Prog. med. Virol. 26, (1980) 214 - 232 und 28, (1982)] 208 - 221:

DNA-Viren mit kubischer Symmetrie und nacktem Nukleokapsid, DNA-Viren mit umhülltem Virion sowie RNA-Viren mit kubischer und solche mit helikaler Symmetrie des Kapsids.

Bevorzugterweise verwendet man die Verbindungen der Formel I im Falle von DNA-Viren mit umhülltem Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z. B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, wie Varicellaviren, z. B. menschlichen Varicella-Zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster Linie Apthoviren von Paarhufern, wie hauptsächlich von Rindern, sowie im Falle von Paramyxoviridae, wie in erster Linie Pneumoviren, z. B. respiratorischen Syncitialviren des Menschen, und wie daneben Morbilliviren oder Paramyxoviren, wie Parainfluenzaviren, z. B. menschlichen Parainfluenzaviren, einschließlich der Sendaiviren sowie im Falle von Arboviren oder Vesiculoviren, z. B. Vesicular stomatitis Viren.

In allererster Linie verwendet man die Verbindungen der Formel I im Falle von Simplexviren, z. B. menschlichen Herpes simplex Viren der Typen 1 und 2, im Falle von menschlichen Encephalomyocarditisviren, im Falle von Influenzaviren, wie hauptsächlich Influenza A und Influenza B Viren und ganz besonders im Falle der in den Beispielen genannten Viren.

Die Verbindungen der Formel I können erfindungsgemäß verwendet werden, indem man sie enteral oder parenteral, in erster Linie zusammen mit geeigneten Hilfs- oder Trägerstoffen, appliziert. Bevorzugterweise appliziert man sie auf die Schleimhaut, z. B. intranasal, rektal, vaginal oder auf die Bindehaut des Auges, oder oral. Der antivirale Effekt tritt jedoch auch bei Applikation auf anderen Wegen, z. B. subkutan, intravenös, intramuskulär oder bei Applikation auf die normale Haut ein.

Die Dosierung des Wirkstoffes hängt u.a. von der Warmblüterspezies, der Abwehrlage des Organismus, der Applikationsweise und der Art des Virus ab. Die Dosis-Wirkungsbeziehung ist relativ schwach ausgeprägt.

Zur Vorbeugung appliziert man eine einmalige Dosis von etwa 0,01 mg bis etwa 25 mg, vorzugsweise 0,05 bis 7 mg, z. B. 0,5 mg, Wirkstoff an einen Warmblüter von etwa 70 kg Körpergewicht, z. B. den Menschen. Die prophylaktische Wirkung dieser Dosis erstreckt sich über mehrere Wochen. Bei Bedarf, z. B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 50 mg, vorzugsweise zwischen 1 und 10 mg, z. B. bei 5 mg, insbesondere bei oraler Applikation. Die Dosierung bei topischer, insbesondere intranasaler Applikation, liegt bis zum Faktor 10 niedriger. Bei Bedarf kann man die

Verabreichung der Verbindungen der Formel I bis zum Eintritt einer Besserung der Erkrankung wiederholen. Oft genügt jedoch eine einmalige Applikation.

Die Erfindung betrifft insbesondere die Phosphatidylverbindungen der Formel I, worin T die Gruppe NH bedeutet und/oder worin W Wasserstoff und Z eine 1,2-Dihydroxy-ethylgruppe, in der mindestens eine der Hydroxygruppen mit einer aliphatischen $C_{8-30}$-Carbonsäure verestert ist, bedeutet, und ihre Salze.

Bevorzugt sind Verbindungen der Formel I, worin Y unsubstituiertes oder durch Carboxy, Niederalkoxycarbonyl oder Carbamoyl substituiertes Dimethylen, W Wasserstoff und Z 1,2-Dihydroxy-ethyl, worin entweder nur die 2-Hydroxy-Gruppe oder beide Hydroxygruppen mit einer $C_{12-24}$-Alkan- oder Alkensäure verestert sind, oder W und Z je eine mit einer $C_{12-24}$-Alkan- oder Alkensäure veresterte Hydroxymethylgruppe bedeuten, und ihre Salze.

Sehr bevorzugt sind die obengenannten Verbindungen der Formel I, worin $R^1$ den Acylrest einer durch α-Amino sowie gegebenenfalls Amino, ein oder zwei Carboxy, Hydroxy, Mercapto, Niederalkylthio, Phenyl, 4-Hydroxy-phenyl, Carbamoyl, Guanidino, 3-Indolyl, 4-Imidazolyl oder (2-Amino-2-carboxy-ethyl)-dithio substituierten Alkansäure mit bis zu 12 C-Atomen, ausgenommen die Acylreste von Glycin, L-Alanin und deren Derivaten mit substituierter Aminogruppe, oder einen Benzoyl- oder Prolylrest bedeutet, und ihre Salze.

Besonders bevorzugt sind die obengenannten Verbindungen der Formel I, worin $R^1$ den Acylrest einer jener 20 Aminosäuren, die regelmässig in Proteinen vorkommen, mit Ausnahme von Glycin und L-Alanin, oder den Acylrest von Milchsäure oder Citronensäure bedeutet, und ihre Salze.

Ganz besonders bevorzugt sind die obengenannten Verbindungen der Formel I, worin $R^1$ den Acylrest von L-Valin, L-Serin, L-Threonin, L-Cystein, L-Methionin, L-Tyrosin oder Citronensäure, T die Gruppe NH, Y unsubstituiertes oder durch Carboxy substituiertes Dimethylen, W Wasserstoff und Z 1,2-Dihydroxy-ethyl, worin beide Hydroxygruppen mit einer geradkettigen $C_{16-24}$-Alkan- oder Alkensäure mit gerader Anzahl C-Atome verestert sind, bedeuten, und ihre Salze.

Hauptsächlich bevorzugt sind die obengenannten Verbindungen der Formel I, worin $R^1$ Benzoyl oder den Acylrest von L-Tyrosin, L-Lysin, L-Asparaginsäure, L-Asparagin, L-Histidin, L-Glutaminsäure, L-Serin, L-Prolin, L-Leucin oder D-Alanin, wobei in diesen Acylresten vorhandene Aminogruppen am Stickstoff durch tert.-Butyloxycarbonyl, Benzyloxycarbonyl oder Niederalkanoyl substituiert und vorhandene Carboxygruppen mit einem Niederalkanol verestert sein können, T die Gruppe NH, Y unsubstituiertes oder durch Carboxy oder Niederalkoxycarbonyl substituiertes Dimethylen, W Wasserstoff und Z eine 1,2-Dihydroxy-ethyl-, 2-Hydroxy-ethyl- oder Hydroxymethylgruppe, in der mindestens eine der Hydroxygruppen mit einer $C_{10-18}$-Alkansäure oder einer $C_{18}$-Alkensäure verestert ist, oder W und Z je eine Hydroxymethylgruppe, die mit einer $C_{10-18}$-Alkansäure oder einer $C_{18}$-Alkensäure verestert ist, bedeuten, und ihre Salze.

Bevorzugt sind auch solche Verbindungen der Formel I, worin W und Z je eine Hydroxymethylgruppe, die mit einer $C_{18}$-Alkensäure oder mit einer geradkettigen $C_{12}$-$C_{18}$-Alkansäure verestert ist, bedeuten, und ihre Salze.

In erster Linie bevorzugt sind die pharmazeutisch verwendbaren Salze der obengenannten Verbindungen der Formel I.

In allererster Linie bevorzugt sind die in den Beispielen beschriebenen Verbindungen der Formel I und ihre Salze.

Die Phosphatidylverbindungen der Formel I und ihre Salze werden nach an sich bekannten Methoden hergestellt. Sie werden z. B. erhalten, indem man

a) eine Verbindung der Formel II,

```
          O   W
          ‖   |
H-T-Y-O-P-O-CH          (II)
          |   |
          OH  Z
```

worin die Substituenten die obengenannten Bedeutungen haben, wobei in einer Verbindung der Formel II vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel III,

$$R^1-OH \qquad (III)$$

worin $R^1$ die obengenannte Bedeutung hat, wobei in einer Verbindung der Formel III vorhandene funktionelle Gruppen mit Ausnahme der an der Reaktion teilnehmenden Gruppe erforderlichenfalls in geschützter Form vorliegen, oder mit einem reaktionsfähigen Carbonsäurederivat davon umsetzt und vorhandene Schutzgruppen abspaltet, oder

b) eine Verbindung der Formel IV,

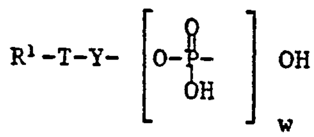

(IV)

worin w für 0 oder 1 steht und $R^1$, T und Y die obengenannten Bedeutungen haben, wobei in einer Verbindung der Formel IV vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel V,

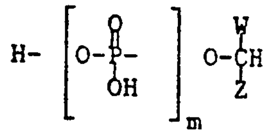

(V)

worin W und Z die obengenannten Bedeutungen haben, wobei in einer Verbindung der Formel V vorhandene funktionelle Gruppen mit Ausnahme der an der Reaktion teilnehmenden Gruppe erforderlichenfalls in geschützter Form vorliegen, und m für 1 steht, wenn im Reaktionspartner der Formel IV w für 0 steht, oder m für 0 steht, wenn w für 1 steht, oder mit einem reaktionsfähigen Derivat einer Verbindung der Formel V umsetzt und vorhandene Schutzgruppen abspaltet, oder

c) eine Verbindung der Formel VI,

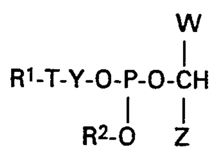

(VI)

worin $R^2$ für Wasserstoff oder eine Schutzgruppe steht und die übrigen Substituenten die obengenannten Bedeutungen haben, wobei in einer Verbindung der Formel VI vorhandene funktionelle Gruppen mit Ausnahme der an der Reaktion teilnehmenden Gruppe erforderlichenfalls in geschützter Form vorliegen, oder ein Tautomeres einer Verbindung der Formel VI mit einem Oxidationsmittel oxidiert und vorhandene Schutzgruppen abspaltet, oder

d) eine Verbindung der Formel VII,

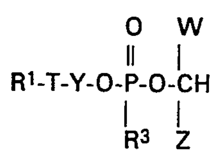

(VII)

worin $R^3$ Halogen bedeutet und die übrigen Substituenten die obengenannten Bedeutungen haben, wobei in einer Verbindung der Formel VII vorhandene funktionelle Gruppen mit Ausnahme der an der Reaktion teilnehmenden Gruppe erforderlichenfalls in geschützter Form vorliegen, hydrolysiert und vorhandene Schutzgruppen abspaltet, oder

e) eine Verbindung der Formel VIII,

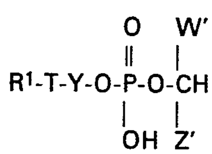

(VIII)

worin W' Wasserstoff und Z' 1,2-Dihydroxy-ethyl, 2-Hydroxy-ethyl, Hydroxymethyl oder 1,2-Dihydroxy-ethyl, worin eine der beiden Hydroxygruppen mit einer aliphatischen $C_{8-30}$-Carbonsäure verestert oder mit einem aliphatischen $C_{8-30}$-Alkohol verethert ist, oder einer der Reste W' und Z' Hydroxymethyl und der andere der Reste W' und Z' freies Hydroxymethyl oder Hydroxymethyl, das mit einer aliphatischen $C_{8-30}$-Carbonsäure verestert oder mit einem aliphatischen $C_{8-30}$-Alkohol verethert ist, und $R^1$, T und Y die obengenannten Bedeutungen haben, wobei in einer Verbindung der Formel VIII vorhandene funktionelle Gruppen mit Ausnahme der an der Reaktion teilnehmenden Gruppe erforderlichenfalls in geschützter Form vorliegen, oder ein reaktionsfähiges Derivat davon, mit einer aliphatischen $C_{8-30}$-Carbonsäure oder einem reaktionsfähigen

Derivat davon verestert oder mit einem aliphatischen $C_{8-30}$-Alkohol oder einem reaktionsfähigen Derivat davon verethert und vorhandene Schutzgruppen abspaltet, oder

f) eine Verbindung der Formel IX,

```
        O   W
        ||  |
X-Y-O-P-O-CH          (IX)
        |   |
        OH  Z
```

worin X für eine nucleophile Abgangsgruppe steht und die übrigen Substituenten die obengenannten Bedeutung haben, wobei in einer Verbindung der Formel IX vorhandene funktionelle Gruppen mit Ausnahme der an der Reaktion teilnehmenden Gruppe erforderlichenfalls in geschützter Form vorliegen, mit einer Verbindung der Formel X,

$R^1$-T-H (X)

worin die Substituenten die obengenannten Bedeutungen haben, wobei in einer Verbindung der Formel X vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzt und vorhandene Schutzgruppen abspaltet, oder

g) in einer Verbindung der Formel I, worin die Substituenten die obengenannten Bedeutungen haben, wobei in einer Verbindung der Formel I mindestens eine funktionelle Gruppe durch eine leicht abspaltbare Schutzgruppe geschützt sein muß, die Schutzgruppe(n) abspaltet, oder

h) zur Herstellung einer Verbindung der Formel I, worin W für Wasserstoff und Z für eine 1,2-Dihydroxy-ethylgruppe steht, worin die 2-Hydroxygruppe mit einer aliphatischen $C_{8-30}$-Carbonsäure verestert ist, eine Verbindung der Formel I, worin W für Wasserstoff und Z für eine 1,2-Dihydroxy-ethyl-gruppe steht, worin beide Hydroxygruppen mit einer aliphatischen $C_{8-30}$-Carbonsäure verestert sind, mit einem zur regioselektiven Abspaltung des die 1-Hydroxygruppe veresternden Restes geeigneten Enzym umsetzt

und nach Ausführung einer der obengenannten Verfahrensvarianten a - h) zur Herstellung eines Salzes erforderlichenfalls eine Verbindung der Formel I in ein Salz überführt oder zur Überführung einer Verbindung der Formel I oder eines Salzes davon in eine andere Verbindung der Formel I oder in ein Salz davon eine im Rest $R^1$ enthaltene Amino- oder Hydroxygruppe acyliert oder eine im Rest $R^1$ oder Y enthaltene Carboxylgruppe verestert oder amidiert.

Die obengenannten Verfahrensvarianten werden im folgenden näher erläutert:

Verfahren a:

Die an der Reaktion teilnehmende Gruppe in einer Verbindung der Formel II ist die Gruppe H-T. Falls T für gegebenenfalls durch Niederalkyl substituiertes NH steht, ist bei geeigneter Reaktionsführung lediglich ein Schutz von weiteren im Molekül der Formel II oder III befindlichen Aminogruppen sowie gegebenenfalls Carboxylgruppen erforderlich. Ein Schutz von Hydroxygruppen oder Mercaptogruppen ist fakultativ.

Falls T für Sauerstoff steht, werden zweckmäßigerweise alle weiteren im Molekül der Formel II oder III vorhandenen Hydroxygruppen sowie freies Amino, Niederalkylamino, Mercapto und Carboxy und gegebenenfalls weitere funktionelle Gruppen geschützt.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis", John Wiley & Sons, New York 1981. Charakteristisch für Schutzgruppen ist, daß sie leicht, d.h. ohne daß unerwünschte Nebenreaktionen stattfinden, z. B. solvolytisch, reduktiv, photolytisch oder auch unter physiologischen Bedingungen abspaltbar sind.

Hydroxyschutzgruppen sind z. B. Acylreste, wie gegebenenfalls, z. B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z. B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z. B. tert.-Butyl, 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z. B. Methoxymethyl, 1-Methoxyethyl, 1-Ethoxy-ethyl, Methylthiomethyl, 1-Methylthioethyl oder 1-Ethylthioethyl, oder 2-Oxa- oder 2-Thiacycloalkyl mit 5 - 6 Ringatomen, z. B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z. B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z. B. tert.-Butyloxycarbonyl, Arylmethoxycarbonyl mit einem oder zwei Arylresten, wobei diese gegebenenfalls z. B. durch Niederalkyl, wie tert.-Niederalkyl, z. B. tert.-Butyl, Niederalkoxy, wie Methoxy, Hydroxy, Halogen, z. B. Chlor, und/oder Nitro, mono- oder polysubstituierte Phenylreste darstellen, wie gegebenenfalls, z. B. wie oben erwähnt, substituiertes Benzyloxycarbonyl, z. B. 4-Methoxybenzyloxycarbonyl, oder 4-Nitrobenzyloxycarbonyl, oder gegebenenfalls, z. B. wie oben erwähnt, substituiertes Diphenylmethoxycarbonyl, z. B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z. B. durch Halogen, wie Brom, substituiertes Benzoyl darstellt, z. B. Phenacyloxycarbonyl, 2-Halogenniederalkoxycarbonyl, z. B. 2,2,2-Trichlorethoxycarbonyl, 2-Bromethoxycarbonyl oder 2-lodethoxycarbonyl, oder 2-(trisubstituiertes Silyl)-ethoxycarbonyl, worin die Substituenten unabhängig voneinander je einen gegebenenfalls substituierten, z. B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z. B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butylsilyl, oder entsprechend substituiertes Stannyl, z. B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z. B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z. B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z. B. bis zu 18 Kohlenstoffatomen, insbesondere einer gegebenenfalls, z. B. durch Halogen oder Aryl, substituierten Alkancarbonsäure oder gegebenenfalls, z. B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-lod-, 2,2,2-Trifluor- oder 2,2,2-Trichloracetyl, gegebenenfalls, z. B. durch Halogen, Niederalkoxy oder Nitro substituiertes Benzoyl, z. B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z. B. tert.-Butyloxycarbonyl, Arylmethoxycarbonyl mit eine oder zwei Arylresten, die vorzugsweise gegebenenfalls, z. B. durch Niederalkyl, insbesondere tert.-Niederalkyl, wie tert.-Butyl, Niederalkoxy, wie Methoxy, Hydroxy, Halogen, z. B. Chlor, und/oder Nitro, mono- oder polysubstituiertes Phenyl darstellen, wie gegebenenfalls substituiertes Benzyloxycarbonyl, z. B. 4-Nitro-benzyloxycarbonyl, oder substituiertes Diphenylmethoxycarbonyl, z. B. Benzhydryloxycarbonyl oder Di-(4-methoxyphenyl)-methoxycarbonyl, Aroylmethoxycarbonyl, worin die Aroylgruppe vorzugsweise gegebenenfalls, z. B. durch Halogen, wie Brom, substituiertes Benzoyl darstellt, z. B. Phenacyloxycarbonyl, 2-Bromethoxycarbonyl oder 2-lodethoxycarbonyl, oder 2-(trisubstituiertes Silyl)-ethoxycarbonyl, worin die Substituenten unabhängig voneinander je einen gegebenenfalls substituierten, z. B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z. B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl).

Weitere, als Aminoschutzgruppen in Frage kommende Acylreste sind auch entsprechende Reste organischer Phosphor-, Phosphon- oder Phosphinsäuren, wie Diniederalkylphosphoryl, z. B. Dimethylphosphoryl, Diethylphosphoryl, Di-n-propylphosphoryl oder Diisopropylphosphoryl, Dicycloalkylphosphoryl, z. B. Dicyclohexylphosphoryl, gegebenenfalls substituiertes Diphenylphosphoryl, z. B. Diphenylphosphoryl, gegebenenfalls, z. B. durch Nitro substituiertes Di-(phenylniederalkyl)-phosphoryl, z. B. Dibenzylphosphoryl oder Di-(4-nitrobenzyl)-phosphoryl, gegebenenfalls substituiertes Phenyloxy-phenyl-phosphonyl, z. B. Phenyloxyphenyl-phosphonyl, Diniederalkylphosphinyl, z. B. Diethylphosphinyl, oder gegebenenfalls substituiertes Diphenylphosphinyl, z. B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z. B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z. B. 4-Nitrophenylthio.

In einem als Aminoschutzgruppe verwendbaren 2-Acyl-niederalk-1-enl-yl-rest ist Acyl z. B. der entsprechende Rest einer Niederalkancarbonsäure, einer gegebenenfalls, z. B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z. B. 1-Acetyl-prop-1-en-2-yl, oder 1-Niederalkoxycarbonyl-prop-1-en-2-yl, z. B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z. B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z. B. wie angegeben, substituiertes Benzyloxycarbonyl, z. B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Eine Mercaptogruppe, wie z. B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden. Bevorzugte Mercaptoschutzgruppen sind z. B. gegebenenfalls im Phenylrest, z. B. durch Methoxy oder Nitro, substituiertes Benzyl, wie 4-Methoxybenzyl, gegebenenfalls im Phenylteil, z. B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl, Benzoyl, Benzyloxycarbonyl oder Aminocarbonyl, wie Ethylaminocarbonyl.

Vorzugsweise wird die Reaktion so durchgeführt, daß man die Verbindung der Formel III in Form eines aktivierten Carbonsäurederivats mit der Verbindung der Formel II umsetzt, wobei die Aktivierung der Carbonsäure der Formel III auch in situ in Gegenwart der Verbindung der Formel II erfolgen kann.

Aktivierte Carbonsäurederivate einer Verbindung der Formel III sind in erster Linie reaktionsfähige aktivierte Ester oder reaktionsfähige Anhydride, ferner reaktionsfähige cyclische Amide; dabei können reaktionsfähige Derivate von Säuren der Formel III auch in situ gebildet werden.

Aktivierte Ester von Säuren sind insbesondere am Verknüpfungskohlenstoffatom des veresternden Restes ungesättigte Ester, z. B. vom Vinylester-Typ, wie eigentliche Vinylester (die man z. B. durch Umesterung eines entsprechenden Esters mit Vinylacetat erhalten kann; Methode des aktivierten Vinylesters), Carbamoylvinylester (die man z. B. durch Behandeln der entsprechenden Säure mit einem Isoxazoliumreagens erhalten kann; 1,2-Oxazolium- oder Woodward-Methode), oder 1-Niederalkoxyvinylester (die man z. B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z. B. durch Behandeln der entsprechenden Säure mit einem geeigneten N,N'-disubstituierten Carbodiimid, z. B. N,N'-Dicyclohexylcarbodiimid, erhalten kann; Carbodiimid-Methode), oder N,N-disubstituierte Amidinoester (die man z. B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z. B. durch Behandeln der entsprechenden Säure mit einem geeignet substituierten Phenol, z. B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z. B. durch Behandeln der entsprechenden Säure mit Chloracetonitril in Gegenwart einer Base erhalten kann; Cyanmethylester-Methode), Thioester, insbesondere gegebenenfalls, z. B. durch Nitro, substituierte Phenyl-thioester (die man z. B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino- oder Amidoester (die man z. B. durch Behandeln der entsprechenden Säure mit einer N-Hydroxy-amino- bzw. N-Hydroxy-amido-Verbindung, z. B. N-Hydroxy-succinimid, N-Hydroxy-piperidin, N-Hydroxyphthalimid oder 1-Hydroxy-benztriazol, z. B. nach der Anhydrid- oder Carbodiimid-Methode, erhalten kann; Methode der aktivierten N-Hydroxyester) oder Silylester (die man z. B. durch Behandeln der entsprechenden Säure mit einem Silylierungsmittel, z. B. Hexamethyldisilazan, erhalten kann und die leicht mit Hydroxy-, nicht aber mit Aminogruppen reagieren).

Anhydride von Säuren der Formel III können symmetrische oder vorzugsweise gemischte Anhydride dieser Säuren sein, so z. B. Anhydride mit anorganischen Säuren, wie Säurehalogenide, insbesondere Säurechloride (die man z. B. durch Behandeln der entsprechenden Säure mit Thionylchlorid, Phosphorpentachlorid oder Oxalylchlorid erhalten kann; Säurechloridmethode), Azide (die man z. B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z. B. Kohlensäureniederalkylhalbestern (die man z. B. durch Behandeln der entsprechenden Säure mit Halogen-, wie Chlorameisensäureniederalkylestern oder mit einem 1-Niederalkoxycarbonyl-2-niederalkoxy-1,2-dihydro-chinolin, z. B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z. B. durch

Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z. B. durch Behandeln der entsprechenden Säure mit einem gegebenenfalls substituierten Niederalkan- oder Phenylalkancarbonsäurehalogenid, z. B. Phenylessigsäure-, Pivalinsäure- oder Trifluoressigsäurechlorid erhalten kann; Methode der gemischten Carbonsäureanhydride) oder mit organischen Sulfonsäuren (die man z. B. durch Behandeln eines Salzes, wie eines Alkalimetallsalzes, der entsprechenden Säure, mit einem geeigneten organischen Sulfonsäurehalogenid, wie Niederalkan- oder Aryl-, z. B. Methan- oder p-Toluolsulfonsäurechlorid, erhalten kann; Methode der gemischten Sulfonsäureanhydride), sowie symmetrische Anhydride (die man z. B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z. B. Imidazol (die man z. B. durch Behandeln der entsprechenden Säure mit N,N'-Carbonyldiimidazol erhalten kann; Imidazolid-Methode), oder Pyrazolen, z. B. 3,5-Dimethyl-pyrazol (die man z. B. über das Säurehydrazid durch Behandeln mit Acetylaceton erhalten kann; Pyrazolid-Methode).

Wie erwähnt können Derivate von Säuren der Formel III auch in situ gebildet werden. So kann man z. B. N,N'-disubstituierte Amidinoester in situ bilden, indem man das Gemisch des Ausgangsmaterials der Formel II und der Säure der Formel III in Gegenwart eines geeigneten N,N-disubstituierten Carbodiimids, z. B. N,N'-Dicyclohexylcarbodiimid, zur Reaktion bringt. Ferner kann man Amino- oder Amidoester von Säuren der Formel III in Gegenwart des zu acylierenden Ausgangsmaterials der Formel II bilden, indem man das Gemisch der entsprechenden Säure- und Amino-Ausgangsstoffe in Gegenwart eines N,N'-disubstituierten Carbodiimids, z. B. N,N'-Dicyclohexyl-carbodiimid, und eines N-Hydroxy-amins oder N-Hydroxy-amids, z. B. N-Hydroxysuccinimid, gegebenenfalls in Anwesenheit einer geeigneten Base, z. B. 4-Dimethylamino-pyridin, umsetzt.

Alternativ kann Verfahrensvariante a) auch so durchgeführt werden, daß man die Säure III mit einem reaktionsfähigen Derivat einer Verbindung der Formel II umsetzt.

Ein Derivat einer Verbindung der Formel II, worin der Rest H-T für die Gruppe NH steht und worin die an der Reaktion teilnehmende Aminogruppe in reaktionsfähiger Form vorliegt, kann z. B. durch Umsetzung mit einem Phosphit, z. B. Diethylchlorphosphit, 1,2-Phenylen-chlorphosphit, Ethyl-dichlor-phosphit, Ethylen-chlor-phosphit oder Tetraethylpyrophosphit, hergestellt werden. Eine reaktionsfähige Form einer Verbindung der Formel II ist z. B. auch ein Carbaminsäurehalogenid oder ein Isocyanat, wobei die an der Reaktion teilnehmende Aminogruppe in einer Verbindung der Formel II an Halogencarbonyl, z. B. Chlorcarbonyl, gebunden ist beziehungsweise als Isocyanatgruppe vorliegt, wobei im letzteren Falle nur Verbindungen der Formel I zugänglich sind, die am Stickstoffatom der durch die Reaktion gebildeten Amidgruppe ein Wasserstoffatom tragen.

Ein Derivat einer Verbindung der Formel II, worin die Gruppe H-T für in reaktionsfähiger Form vorliegendes Hydroxy steht, ist z. B. ein Halogenid. In diesem Fall kann man z. B. auch ein Metallsalz, wie Alkalimetall, vorzugsweise Cäsiumsalz, einer Carbonsäure der Formel III mit dem genannten Halogenid umsetzen.

Die Reaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z. B., wenn die an der Reaktion teilnehmende Carboxylgruppe als Anhydrid vorliegt, auch ein Säure-bindendes Mittel sein kann, unter Kühlen oder Erwärmen, z. B. in einem Temperaturbereich von etwa -30°C bis etwa +150°C, in einem geschlossenen Reaktionsgefäß und/oder in der Atmosphäre eines Inertgases, z. B. Stickstoff. Übliche Kondensationsmittel sind z. B. Carbodiimide, beispielsweise N,N'-Diethyl-, N,N'-Dipropyl-, N,N'-Dicyclohexyl- oder N-Ethyl-N'-(3-dimethylaminopropyl)carbodiimid, geeignete Carbonylverbindungen, beispielsweise Carbonyldiimidazol, oder 1,2-Oxazoliumverbindungen, z. B. 2-Ethyl-5-phenyl-1,2-oxazolium-3'-sulfonat und 2-tert.-Butyl-5-methyl-isoxazoliumperchlorat, oder eine geeignete Acylaminoverbindung, z. B. 2-Ethoxy-1-ethoxycarbonyl-1,2-dihydrochinolin. Übliche säurebindende Kondensationsmittel sind z. B. Alkalimetallcarbonate oder -hydrogencarbonate, z. B. Natrium- oder Kaliumcarbonat oder -hydrogencarbonat (üblicherweise zusammen mit einem Sulfat), oder organische Basen, wie üblicherweise sterisch gehinderte Triniederalkylamine, z. B. N,N-Diisopropyl-N-ethylamin.

Die Abspaltung der Schutzgruppen, z. B. der Carboxyl-, Amino-, Hydroxy- oder Mercaptoschutzgruppen, erfolgt in an sich bekannter Weise, z. B. mittels Solvolyse, insbesondere Hydrolyse, Alkoholyse oder Acidolyse, oder mittels Reduktion, insbesondere Hydrogenolyse oder chemische Reduktion, gegebenenfalls Stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z. B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z. B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z. B. durch Behandeln mit einem Alkalimetall-, z. B. Natriumdithionit, oder mit einem reduzierenden Metall, z. B. Zink, oder Metallsalz, wie einem Chrom-II-salz, z. B. Chrom-II-chlorid, üblicherweise in Gegenwart eines Wasserstoff-abgebenden Mittels, das zusammen mit

dem Metall nascierenden Wasserstoff zu erzeugen vermag, wie einer Säure, in erster Linie einer geeigneten Carbonsäure, wie einer gegebenenfalls, z. B. durch Hydroxy, substituierten Niederalkancarbonsäure, z. B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwasserstoffsäure, wie einem Alkalimetallfluorid, z. B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z. B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z. B. durch Behandeln mit einem geeigneten chemischen Reduktionsmittel, wie Zink in Gegenwart einer geeigneten Carbonsäure, wie wäßriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z. B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z. B. Ameisen- oder Trifluoressigsäure, gegebenenfalls substituiertes Benzyloxycarbonylamino z. B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z. B. durch behandeln mit einer Säure, wie Mineralsäure, z. B. Chlorwasserstoffsäure, oder einer organischen Säure, z. B. Ameisen-, Essig- oder Trifluoressigsäure, gegebenenfalls in Gegenwart von Wasser, und eine mit einer organischen Silylgruppe geschützte Aminogruppe z. B. mittels Hydrolyse oder Alkoholyse freigesetzt werden. Eine durch 2-Halogenacetyl, z. B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschließende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z. B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z. B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z. B. durch basische Hydrolyse, eine durch tert.-Niederalkyl oder durch einen 2-oxa- oder 2-thiaaliphatischen oder -cycloaliphatischen Kohlenwasserstoffrest veretherte Hydroxy- bzw. Mercaptogruppe durch Acidolyse, z. B. durch Behandeln mit einer Mineralsäure oder einer starken Carbonsäure, z. B. Trifluoressigsäure, freigesetzt. Zwei Hydroxygruppen, die zusammen mittels einer vorzugsweise substituierten Methylengruppe, wie durch Niederalkyliden, z. B. Isopropyliden, Cycloalkyliden, z. B. Cyclohexyliden, oder Benzyliden, geschützt sind, können durch saure Solvolyse, besonders in Gegenwart einer Mineralsäure oder einer starken organischen Säure, freigesetzt werden.

Bevorzugt werden beim Vorhandensein von mehreren geschützten funktionellen Gruppen die Schutzgruppen so gewählt, daß gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator.

Verfahren b:

Ein reaktionsfähiges Derivat einer Verbindung der Formel IV, worin w für 1 steht, oder einer Verbindung der Formel V, worin m für 1 steht, ist z. B. ein Mono- oder Bisanhydrid mit einer starken Säure, insbesondere einer Mineralsäure, wie vornehmlich einer Halogenwasserstoffsäure, wie hauptsächlich Chlorwasserstoffsäure. Die

zweite saure Phosphorsäuregruppe kann als solche, als Anhydrid wie oben geschildert oder in veresterter Form vorliegen, wobei als veresternde Reste solche bevorzugt sind, die nach erfolgter Reaktion zwischen den Verbindungen IV und V regioselektiv wieder abgespalten werden können, z. B. die Methylestergruppe, die z. B. durch alkalische Verseifung oder vorzugsweise mittels Lithiumbromid abgespalten werden kann, oder insbesondere hydrogenolytisch abspaltbare Reste, z. B. Benzyl- oder Phenylesterreste, wobei Benzylesterreste z. B. in Gegenwart von Palladiumkatalysatoren, wie Palladium auf Kohlenstoff, und Phenylestergruppen z. B. in Gegenwart von Platin- oder gemischten Platin-Palladium-Katalysatoren abgespalten werden können.

Die Bildung reaktionsfähiger Phosphorsäurederivate kann auch in situ in Gegenwart von Verbindungen erfolgen, die mit Phosphorsäure oder deren Monoestern zumindest intermediär reaktionsfähige Verbindungen mit anhydrid- oder enolesterartigem Charakter einzugehen vermögen, z. B. in Gegenwart von p-Toluolsulfonsäurechlorid, Cyanurchlorid, N-Alkyl-5-phenylisoxazoliumsalzen, Ethoxyacetylen oder bevorzugterweise Trichloracetonitril oder insbesondere einem Carbodiimid, wie hauptsächlich Dicyclohexylcarbodiimid. Beispielsweise kann man einen Phosphorsäuremonoester der Formeln IV oder V, worin w bzw. m für 1 stehen, mit überschüssigem Alkohol der Formeln IV bzw. V, worin w bzw m für 0 stehen in Gegenwart der mehrfachen z. B. fünffachen, molaren Menge von Dicyclohexylcarbodiimid in An- oder Abwesenheit eines tertiären Amins umsetzen.

Wenn in einem Phosphorsäuremonoester beide sauren Gruppen als Anhydrid mit einer Halogenwasserstoffsäure vorliegen, kann man zunächst neben dem Triester auch Phosphorsäurediester-halogenide erhalten, die anschließend durch Wasser, wasserabgebende Mittel oder durch Erhitzen mit tertiären Alkoholen, wie tert.-Butanol oder Tetrahydropyranol, zu Diestern hydrolysiert werden können.

Wenn man von einem Phosphorsäuremonoester-dihalogenid, z. B. einem Phosphorsäuremonoester-dichlorid, ausgeht, führt man die Umsetzung bevorzugterweise in Anwesenheit eines tertiären Amins, wie Pyridin, Lutidin oder Chinolin durch, wobei eine zusätzliche Aktivierung des Esterchlorids durch Dimethylformamid bewirkt wird.

Eine bevorzugte Ausführungsform des Verfahrens b ist die Umsetzung eines Phosphorsäuremonoesterdichlorids mit dem entsprechenden Alkohol in Gegenwart eines tertiären Amins, gefolgt von der Hydrolyse des zunächst erhaltenen Phosphorsäurediesterhalogenids.

In einem reaktionsfähigen Derivat einer Verbindung der Formeln IV oder V, worin w bzw. m für 0 stehen, liegt die an der Reaktion teilnehmende Hydroxygruppe in reaktionsfähiger, veresterter Form vor.

Reaktionsfähiges verestertes Hydroxy ist z. B. mit einer starken anorganischen oder organischen Säure verestertes Hydroxy, wie mit einer Mineralsäure, z. B. Halogenwasserstoffsäure, wie Chlorwasserstoff-, Bromwasserstoff- oder Iodwasserstoffsäure, ferner Schwefelsäure, oder Halogenschwefelsäure, z. B. Fluorschwefelsäure, oder mit einer starken organischen Sulfonsäure, wie einer gegebenenfalls, z. B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom, und/oder Nitro substituierten Benzolsulfonsäure, z. B. einer Methansulfon-, Trifluormethansulfon- oder p-Toluolsulfonsäure verestertes Hydroxy, bevorzugterweise ein Chlorid, Bromid oder Iodid.

Die Reaktion kann so durchgeführt werden, daß man ein reaktionsfähiges Phosphorsäurederivat der Formeln IV oder V mit einem Alkohol der Formeln V bzw. IV in unaktivierter Form umsetzt, oder indem man einen reaktionsfähigen veresterten Alkohol der Formeln IV oder V mit einem Phosphorsäurederivat der Formeln V bzw. IV in unaktivierter Form oder einem reaktionsfähigen Salz davon umsetzt.

Als Salze von Verbindungen der Formeln IV oder V verwendet man im Hinblick auf die beabsichtigte nucleophile Substitutionsreaktion besonders reaktionsfähige Salze, z. B. Salze, wie Silbersalze, die mit der nucleophilen Abgangsgruppe im Reaktionspartner, z. B. einem der obengenannten Halogenidionen, einen schwerlöslichen Niederschlag zu bilden vermögen, oder Salze mit großem Kation, z. B. Cäsiumsalze, in denen die Nucleophilie des Phosphatrestes erhöht ist. Zur Erhöhung der Nucleophilie des Phosphatrestes kann das Gegenion auch räumlich entfernt werden, z. B. durch Zugabe von Komplexbildnern, wie Kronenethern, z. B. 18-Krone-6. Bei Verwendung von 18-Krone-6 kann man die Reaktion mit einem Kaliumsalz durchführen.

Eine bevorzugte Ausführungsform des Verfahrens b ist die Umsetzung des Silbersalzes eines Phosphorsäuremonoesters der Formeln IV oder V, worin eine der beiden sauren Gruppen durch eine leicht abspaltbare Schutzgruppe, z. B. eine der oben beschriebenen, geschützt ist, z. B. als Benzyl- oder Phenylester, mit einem reaktionsfähigen Alkohol der Formel V bzw. IV, worin die OH-Gruppe durch Chlor, Brom oder Iod ersetzt ist. Nach erfolgter Umsetzung wird die Schutzgruppe abgespalten, z. B. eine Benzyl- oder Phenylesterschutzgruppe durch Hydrierung wie oben beschrieben.

Verfahren c:

Die Verbindungen der Formel VI, worin $R^2$ für Wasserstoff steht, liegen zum überwiegenden Teil in der tautomeren Form vor, worin ein Proton direkt an Phosphor gebunden ist. Die Oxidation kann z. B. mit wässerigem Kaliumpermanganat bei Temperaturen um 0°C durchgeführt werden. In wässerigem Medium sind auch Alkalijodate, -perjodate und -hypochlorite, Peressigsäure, N-Chlor-4-methyl-benzol-sulfonsäureamid u.a. als Oxidationsmittel geeignet.

Schutzgruppen $R^2$ sind z. B. die bei Verfahren b genannten.

Verfahren d:

In einer Verbindung der Formel VII steht $R^3$ für ein Halogen, wie Brom oder Iod, in allererster Linie aber für Chlor.

Die Hydrolyse wird mit Wasser oder einem wasserabgebenden Mittel, vorzugsweise bei erhöhter Temperatur, z. B. zwischen 30 und 95° C, durchgeführt.

Die Ausgangsstoffe sind z. B. wie bei Verfahren b beschrieben oder durch Chlorierung der entsprechenden Phosphorigsäurediester, z. B. mit elementarem Chlor, erhältlich.

Verfahren e:

Als Schutzgruppen oder als reaktionsfähige Carbonsäurederivate können z. B. die bei Verfahren a genannten dienen. Als reaktionsfähige Alkoholderivate verwendet man Alkohole, deren Hydroxygruppe in reaktionsfähiger, veresterter Form vorliegt, z. B. wie bei Verfahren b beschrieben. Die Reaktion wird z. B. durchgeführt, indem man eine Verbindung der Formel VIII in unaktivierter Form mit dem reaktionsfähigen Carbonsäure- oder Alkoholderivat umsetzt, wobei die Aktivierung der Carbonsäure auch in situ, in Gegenwart der Verbindung der Formel VIII, erfolgen kann, z. B. analog wie bei Verfahren a beschrieben. Alternativ kann die Reaktion durchgeführt werden, indem man eine Verbindung der Formel VIII, worin die an der Reaktion teilnehmende(n) Hydroxygruppe(n) in reaktionsfähiger, veresterter Form vorliegt (vorliegen), z. B. als Halogenid, mit einer Carbonsäure oder einem Alkohol in jeweils unaktivierter Form oder mit einem reaktionsfähigen Carbonsäuresalz umsetzt, z. B. analog wie bei Verfahren a beschrieben.

Verfahren f:

Eine nucleophile Abgangsgruppe X ist insbesondere eine mit geeigneten Säuren veresterte Hydroxygruppe, z. B. reaktionsfähiges verestertes Hydroxy wie bei Verfahren b beschrieben.

Wenn in der Verbindung der Formel X der Rest T für NH steht, wird die Reaktion am besten mit Hilfe einer Base durchgeführt, wobei man vorzugsweise äquimolare Mengen dieser Base verwendet. Als Basen verwendet man insbesondere z. B. Metallhydroxide, -carbonate oder -alkoholate, wie Alkalimetall- oder Erdalkalimetallhydroxide oder -alkoholate, z. B. Natrium- oder Kaliumhydroxid oder Natrium-tert.-butylat, oder Alkalimetallcarbonate oder Salze, insbesondere Alkalimetallsalze, sekundärer Amide, z. B. 2-Oxo-pyrrolidin-natrium, oder starkbasische Ionenaustauscher, z. B. Dowex 1 (eingetragenes Warenzeichen), daneben auch Hydride oder Amide, z. B. Alkalimetallhydride oder -amide, wie Natriumhydrid oder -amid oder Lithiumdiisopropylamid.

Die Reaktion wird vorzugsweise in einem inerten organischen Lösungsmittel, wenn erwünscht oder notwendig, unter Kühlen oder Erwärmen durchgeführt, z. B. in einem Temperaturbereich von etwa -80° C bis etwa +150° C, hauptsächlich zwischen 0° C und 100° C.

Die Aktivierung mit Hilfe der Base kann bei der gleichen oder einer anderen Temperatur erfolgen als die eigentliche Kupplungsreaktion.

Insbesondere bei Verwendung einer sehr starken Base, z. B. Lithiumdiisopropylamid, erfolgt die Aktivierung mit der Base ("Metallierung") bei tieferer Temperatur (z. B. -80° C) als die Kupplungsreaktion. In diesem Fall kann man z. B. das Reaktionsgefäß nach erfolgter Metallierung sich langsam aufwärmen lassen.

Bei der Wahl des Lösungsmittels muß auch die Art der zu verwendenden Base berücksichtigt werden. Reaktionen unter Verwendung von Alkalimetallhydroxiden werden vorzugsweise in Dimethylsulfoxid oder in Alkoholen, wie Niederalkanolen, z. B. wasserfreiem Ethanol bei Siedetemperatur, Reaktionen unter Verwendung von Alkoholaten werden vorzugsweise in Ethern, insbesondere cyclischen Ethern, durchgeführt, bei Verwendung von Natrium-tert.-butylat z. B. in Dioxan bei Raumtemperatur. Reaktionen mit z. B. 2-Oxo-pyrrolidin-natrium werden unter anderem in einem Gemisch aus einem cyclischen Ether und einem aromatischen Kohlenwasserstoff, z. B. einem Dioxan-Toluol-Gemisch, bei einer Temperatur zwischen Raumtemperatur und 60° C durchgeführt.

Die erfindungsgemäße Reaktion mit Hilfe eines Ionenaustauschers nimmt man insbesondere in einem Alkohol, z. B. Niederalkanol, z. B. Ethanol, bei Raumtemperatur vor.

Ein reaktionsfähiges Derivat einer Verbindung der Formel X, worin der Rest T für Sauerstoff steht, ist z. B. ein geeignetes Carbonsäuresalz, z. B. ein Cäsiumsalz mit hoher Nucleophilie des Carboxylatanions oder ein Silbersalz, wobei das Silberion mit einem Halogenid X einen schwerlöslichen Niederschlag bildet. Zur Erhöhung der Nucleophilie des Carboxylatanions kann das Gegenion auch räumlich entfernt werden, z. B. durch Zugabe von Komplexbildnern, wie Kronenethern, z. B. 18-Krone-6. Bei Verwendung von 18-Krone-6 kann man die Reaktion mit einem Kaliumsalz durchführen.

Mögliche Schutzgruppen und ihre Abspaltung sind bei Verfahren a beschrieben.

Verfahren g:

Funktionelle Gruppen in einer Verbindung der Formel I, die gegebenenfalls durch eine leicht abspaltbare Schutzgruppe geschützt sind, sind insbesondere die Phosphorsäuregruppe, eine Hydroxygruppe im Rest Z, freies Carboxy im Rest Y, oder freies Amino, Hydroxy, Carboxy oder Mercapto im Rest $R^1$. Geeignete Schutzgruppen und ihre Abspaltung sind z. B. bei Verfahren a bzw. b beschrieben.

Verfahren h:

Ein geeignetes Enzym ist z. B. Phospholipase $A_2$, das im Handel erhältlich ist (z. B. Boehringer AG, Mannheim, Bundesrepublik Deutschland).

Die zur Ausführung der obengenannten Verfahren benötigten Ausgangsstoffe sind bekannt oder können nach an sich bekannten Verfahren, z. B. analog einem der vorstehend beschriebenen Verfahren, hergestellt werden.

Die Acylierung einer im Rest $R^1$ enthaltenen Amino- oder Hydroxygruppe wird analog wie bei Verfahren a beschrieben durchgeführt.

Die Veresterung einer im Rest $R^1$ enthaltenen Carboxylgruppe wird z. B. folgendermaßen durchgeführt: Geeignete Mittel sind beispielsweise entsprechende Diazoverbindungen, wie gegebenenfalls substituierte Diazoniederalkane, z. B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z. B. Methylenchlorid, oder eines Ethers, wie eines Diniederalkylethers, z. B. Diethylether, oder eines cyclischen Ethers, z. B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäß und/oder unter einer Inertgas-, z. B. Stickstoffatmosphäre, zur Anwendung gebracht.

Weitere geeignete Mittel sind Ester entsprechender Alkohole, in erster Linie solche mit starken anorganischen oder organischen Säuren, wie Mineralsäuren, z. B. Halogenwasserstoffsäuren, wie Chlorwasserstoff-, Bromwasserstoff- oder Iodwasserstoffsäure, ferner Schwefelsäure, oder Halogen-schwefelsäure, z. B. Fluorschwefelsäure, oder starken organischen Sulfonsäuren, wie gegebenenfalls, z. B. durch Halogen, wie Fluor, substituierten Niederalkansulfonsäuren, oder aromatischen Sulfonsäuren, wie z. B. gegebenenfalls durch Niederalkyl, wie Methyl, Halogen, wie Brom, und/oder Nitro substituierten Benzolsulfonsäuren, z. B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z. B. Fluorsulfonsäuremethylester, oder gegebenenfalls Halogen-substituierte Methansulfonsäure-niederalkylester, z. B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z. B. Methylenchlorid, eines Ethers, wie Dioxan oder Tetrahydrofuran, oder eines Gemisches davon verwendet. Dabei wendet man vorzugsweise geeignete Kondensationsmittel, wie Alkalimetallcarbonate oder -hydrogencarbonate, z. B. Natrium- oder Kaliumcarbonat oder -hydrogencarbonat (üblicherweise zusammen mit einem Sulfat), oder organische Basen, wie üblicherweise sterisch gehinderte Triniederalkylamine, z. B. N,N-Diisopropyl-N-ethyl-amin (vorzugsweise zusammen mit Halogensulfonsäureniederalkylestern oder gegebenenfalls halogensubstituierten Methansulfonsäureniederalkylestern) an, wobei unter Kühlen, bei Raumtemperatur oder unter Erwärmen, z. B. bei Temperaturen von etwa -20°C bis etwa 50°C, und, wenn notwendig, in einem geschlossenen Gefäß und/oder in einer Inertgas-, z. B. Stickstoffatmosphäre, gearbeitet wird.

Weitere Mittel sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z. B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z. B. Diethylether, Tetrahydrofuran oder Methylenchlorid, oder in einem Gemisch davon, wenn notwendig, in Gegenwart einer Base, wie einer organischen Base, z. B. eines, vorzugsweise sterisch gehinderten, Triniederalkylamins, z. B. N,N-Diisopropyl-N-ethyl-amin, und unter Kühlen, bei Raumtemperatur oder unter leichtem Erwärmen, z. B. bei etwa -20°C bis etwa 50°C, wenn notwendig, in einem geschlossenen Gefäß und/oder in einer Inertgas-, z. B. Stickstoffatmosphäre.

Die Umwandlung von freiem Carboxyl in einer Verbindung der Formel (I) in verestertes Carboxyl, kann weiterhin beispielsweise erfolgen, indem man eine Verbindung der Formel (I), worin andere, gegebenenfalls vorhandene funktionelle Gruppen gegebenenfalls in geschützter Form vorliegen, oder ein reaktionsfähiges funktionellen Carboxyderivat, inkl. ein Salz davon, mit einem entsprechenden Alkohol umsetzt, z. B. analog wie bei Verfahren a beschrieben.

Die oben beschriebenen Verfahren, inklusive die Verfahren zur Abspaltung von Schutzgruppen und die zusätzlichen Verfahrensmassnahmen, werden in an sich bekannter Weise, z. B. in An- oder Abwesenheit von Lösungs- und Verdünnungsmitteln, wenn notwendig, in Anwesenheit von Kondensationsmitteln oder Katalysatoren, bei erniedrigter oder erhöhter Temperatur, z. B. in einem Temperaturbereich von etwa -20°C bis etwa 150°C, in einem geschlossenen Gefäß und/oder in einer Inertgas-, z. B. Stickstoffatmosphäre, durchgeführt.

Dabei sind unter Berücksichtigung aller im Molekül befindlichen Substituenten, wenn erforderlich, z. B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff

unter den Reaktionsbedingungen bildet oder in Form eines reaktionsfähigen Derivats oder Salzes verwendet. Dabei geht man vorzugsweise von solchen Ausgangsstoffen aus, die verfahrensgemäß zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, daß man zu den in dieser Anmeldung als besonders bevorzugt aufgeführten Verbindungen gelangt.

Gemische von Isomeren können in an sich bekannter Weise, z. B. durch fraktionierte Kristallisation, Chromatographie etc. in die einzelnen Isomeren, Racemate z. B. unter Bildung von Derivaten mit optisch aktiven Verbindungen und Trennung der so erhältlichen Diastereomerengemische in die optisch aktiven Antipoden, aufgetrennt werden.

Die Erfindung betrifft auch pharmazeutische Präparate, die eine zur Prophylaxe oder Therapie von Virusinfektionen wirksame Menge der Aktivsubstanz, gegebenenfalls zusammen mit pharmazeutisch verwendbaren Trägerstoffen enthalten, die sich zur topischen, z. B. intranasalen, enteralen, z. B. oralen oder rektalen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z. B. Lactose, Dextrose, Sukrose, Mannitol, Sorbitol, Cellulose und/oder Glycerin, und/oder Schmiermitteln, z. B. Kieselerde, Talk, Stearinsäure oder Salze davon, wie Magnesium- oder Calciumstearat, und/oder Polyethylenglykol, enthalten. Tabletten können ebenfalls Bindemittel, z. B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süßmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wäßrige Lösungen oder Suspensionen, wobei diese z. B. bei lyophilisierten Präparaten, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z. B. mittels konventioneller Misch-, Granulier-, Dragier-, Lösungs- oder Lyophilisierungsverfahren, hergestellt und enthalten von etwa 0,001 % bis 99 %, insbesondere von etwa 0,01 % bis etwa 10 %, in erster Linie zwischen 0,1 % und 5 %, des bzw. der Aktivstoffe, wobei eine Wirkstoffkonzentration unterhalb von 1 % insbesondere für topisch zu applizierende Präparate geeignet ist.

Als topisch zu applizierende Darreichungsformen sind die folgenden bevorzugt: Crèmes, Salben oder Pasten mit einem Wirkstoffgehalt von 0,001 % bis 1 %, insbesondere von 0,01 % bis 0,1 %, z. B. 0,05 %, z. B. Salben zur intranasalen Applikation oder Lippenstifte, oder wäßrige Lösungen mit einem Wirkstoffgehalt von 0,001 % bis 1 %, insbesondere 0,05 % bis 0,5 %, z. B. 0,1 %, vorzugsweise isotonische, sterile und physiologisch verträgliche Lösungen, z. B. Augentropfen, vorzugsweise in Mikrobehältern zur einmaligen Verwendung, oder Sprays zur Anwendung im Mund- und Rachenraum.

Besonders geeignet sind die in den Beispielen beschriebenen pharmazeutischen Präparate.

Crèmes sind Öl-in-Wasser-Emulsionen, die mehr als 50 % Wasser aufweisen. Als ölige Grundlage verwendet man in erster Linie Fettalkohole, z. B. Lauryl-, Cetyl- oder Stearylalkohole, Fettsäuren, z. B. Palmitin- oder Stearinsäure, flüssige bis feste Wachse, z. B. Isopropylmyristat, Wollwachs oder Bienenwachs, und/oder Kohlenwasserstoffe, z. B. Vaseline (Petrolatum) oder Paraffinöl. Als Emulgatoren kommen oberflächenaktive Substanzen mit vorliegend hydrophilen Eigenschaften in Frage, wie entsprechende nichtionische Emulgatoren, z. B. Fettsäureester von Polyalkoholen oder Ethylenoxidaddukte davon, wie Polyglycerinfettsäureester oder Polyoxyethylensorbitan-fettsäureester (Tweens), ferner Polyoxyethylen-fettalkoholether oder -fettsäureester, oder entsprechende ionische Emulgatoren, wie Alkalimetallsalze von Fettalkoholsulfaten, z. B. Natriumlaurylsulfat, Natriumcetylsulfat oder Natriumstearylsulfat, die man üblicherweise in Gegenwart von Fettalkoholen, z. B. Cetylalkohol oder Stearylalkohol, verwendet. Zusätze zur Wasserphase sind u.a. Mittel, welche die Austrocknung der Crèmes vermindern, z. B. Polyalkohol, wie Glycerin, Sorbit, Propylenglykol und/oder Polyethylenglykole, ferner Konservierungsmittel und Riechstoffe.

Salben sind Wasser-in-Öl-Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z. B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z. B. Cetylalkohol oder Wollwachsalkohole, bzw. Wollwachs, enthalten.

Emulgatoren sind entsprechende lipophile Substanzen, wie Sorbitanfettsäureester (Spans), z. B. Sorbitanoleat und/oder Sorbitanisostearat. Zusätze zur Wasserphase sind z. B. Feuchthaltungsmittel, wie Polyalkohole, z. B. Glycerin, Propylenglykol, Sorbit und/oder Polyethylenglykol, sowie Konservierungsmittel und Riechstoffe.

Fettsalben sind wasserfrei und enthalten als Grundlage insbesondere Kohlenwasserstoffe, z. B. Paraffin, Vaseline und/oder flüssige Paraffine, ferner natürliche oder partialsynthetische Fette, z. B. Kokosfettsäuretriglycerid, oder vorzugsweise gehärtete Öle, z. B. hydriertes Erdnuß oder Rizinusöl, ferner Fettsäurepartialester

des Glycerins, z. B. Glycerinmono- und -distearat, sowie z. B. die im Zusammenhang mit den Salben erwähnten, die Wasserstoffaufnahmefähigkeit steigernden Fettalkohole, Emulgatoren und/oder Zusätze.

Pasten sind Crèmes und Salben mit sekretabsorbierenden Puderbestandteilen, wie Metalloxiden, z. B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Öl-in-Wasser-Emulsionen, wobei halogenierte Kohlenwasserstoffe, wie Chlorfluorniederalkane, z. B. Dichlordifluormethan und Dichlortetrafluorethan, als Treibmittel verwendet werden. Als Ölphase verwendet man u.a. Kohlenwasserstoffe, z. B. Paraffinöl, Fettalkohole, z. B. Cetylalkohol, Fettsäureester, z. B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel.

Tinkturen und Lösungen weisen meistens eine wäßrigethanolische Grundlage auf, der u.a. Polyalkohole, z. B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wäßrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z. B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die Erfindung, ohne sie in irgendeiner Form einzuschränken. Die $R_f$-Werte werden auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

**Abkürzungen:**

Boc = tert.-Butyloxycarbonyl
i.Vak. = im Vakuum
Me = Methyl
MeOH = Methanol
RT = Raumtemperatur
Smp. = Schmelzpunkt
Zers. = Zersetzung

**Beispiel 1:**

29 g N-Boc-L-tyrosin-2-(1,2-dipalmitoyl-sn-glycero-3-hydroxyphosphoryloxy)-ethylamid · 0,67 $H_2O$ (teilweise in Form des Natriumsalzes) löst man in einer Mischung aus 40 ml Trifluoressigsäure und 80 ml Methylenchlorid und läßt 2,5 Stunden bei RT stehen. Man dampft die Lösung i.Vak. zur Trockne, verreibt den Rückstand 3-mal mit Eiswasser und erhält eine farblose Suspension, die man absaugt. Den Niederschlag kristallisiert man zweimal aus Ethylmethylketon-Isopropylalkohol-Wasser (1 : 1 : 1) um. Man erhält L-Tyrosin-2-(1,2-dipalmitoyl-sn-glycero-3-hydroxyphosphoryloxy)-ethylamid in Form farbloser Kristalle vom Zers.-Punkt 185 - 200°;
$[\alpha]^{20}_D = +39°$ (c = 1,007; $CHCl_3$ : MeOH : $H_2O$ = 80 : 10 : 2), $R_f$ = 0,40 ($CHCl_3$ : MeOH : $H_2O$ = 80 : 20 : 2).

Das Ausgangsmaterial erhält man folgendermaßen:

Stufe 1.1: Zu 2 g 2-(1,2-Dipalmitoyl-sn-glycero-3-hydroxyphosphoryloxy)-ethylamin (Kephalin), gelöst in 30 ml $CHCl_3$-MeOH-$H_2O$ (80 : 20 : 2) gibt man bei RT 1,52 g (1,5 Äquivalente) N-Boc-L-tyrosin-N-hydroxysuccinimidester und 0,422 ml Triethylamin. Nach 18 Stunden bei RT dampft man die Lösung i.Vak. zur Trockne, nimmt den Rückstand mit Essigsäureethylester-Tetrahydrofuran (7 : 2) auf und schüttelt einmal mit 5-%-iger $NaHCO_3$-Lösung und zweimal mit gesättigter NaCl-Lösung aus. Die vereinigten wäßrigen Phasen extrahiert man einmal mit Essigsäureethylester-Tetrahydrofuran (7 : 2). Nach Trocknen ($Na_2SO_4$) und Eindampfen der organischen Phase erhält man ein Rohprodukt, das durch Chromatographie an Kieselgel mit $CHCl_3$-MeOH-$H_2O$ (90 : 10 : 0,5) gereinigt wird. Man erhält N-Boc-L-tyrosin-2-(1,2-dipalmitoyl-sn-glycero-3-hydroxyphoshporyloxy)-ethylamid · 0,67 $H_2O$, das teilweise in Form des Natriumsalzes vorliegt;
Smp. 110 - 112°,

$[\alpha]^{20}_D$, +8° (c = 0,786; Methanol),
$R_f$ = 0,64 ($CHCl_3$ : MeOH : $H_2O$ = 80 : 20 : 2).
Die vollständige Umwandlung in das Natriumsalz gelingt nach dem im Beispiel 3 beschriebenen Filtrationsverfahren.

**Beispiel 2:**

Aus N-Boc-glycin-2-(1,2-dipalmitoyl-sn-glycero-3-hydroxyphosphoryloxy)-ethylamid-natriumsalz · 0,87 $H_2O$ erhält man analog Beispiel 1 durch Spaltung mit Trifluoressigsäure in Methylenchlorid Glycin-2-(1,2-dipalmitoyl-sn-glycero-3-hydroxyphosphoryloxy)-ethylamid · 0,13 $H_2O$; Smp. 220 - 227° (Zers.), $R_f$, 0,16 ($CHCl_3$ : MeOH : $H_2O$ = 80 : 20 : 2).

Das Ausgangsmaterial erhält man folgendermaßen:

Stufe 2.1: Aus 2-(1,2-Dipalmitoyl-sn-glycero-3-hydroxyphosphoryloxy)-ethylamin und N-Boc-glycin-N-hydroxy-succinimidester erhält man analog Stufe 1.1 N-Boc-glycin-2-(1,2-dipalmitoyl-sn-glycero-3-hydroxyphosphoryloxy)-ethylamid-natriumsalz · 0,87 $H_2O$;
Smp. 60 - 65°,
$[\alpha]^{20}_D$ = +5,9° (c = 0,945, $CHCl_3$ : MeOH = 1 : 1),
$R_f$ = 0,37 ($CHCl_3$ : MeOH : $H_2O$ = 80 : 20 : 2).

**Beispiel 3:**

4,7 g 2-(1,2-Dipalmitoyl-sn-glycero-3-hydroxyphosphoryloxy)-ethylamin-natriumsalz löst man in 80 ml Chloroform-Methanol (9 : 1). Dazu gibt man eine Lösung von 1,21 g Citronensäureanhydrid in 16 ml absolutem Dimethylacetamid und anschließend 1,9 ml Triethylamin. Nach 2 Stunden bei RT dampft man die Lösung bei 35° i.Vak. zur Trockne, verrührt den Rückstand mit 20 ml einnormaler Salzsäure und saugt die entstehenden farblosen Kristalle ab. Dies wiederholt man, wäscht die Kristalle mit einnormaler Salzsäure, suspendiert das Nutschgut in 200 ml Wasser und stellt den pH-Wert unter Kontrolle mit einer Elektrode auf 6,8. Die Substanz geht dabei völlig in Lösung. Man filtriert in einer Rührzelle über ein Ultrafilter mit einer Ausschlußgrenze von 10'000 dalton (Hersteller: Amicon Corporation, Danvers, Massachusetts, 01932 USA, Modell 402, Ultrafilter PM 10/76 mm Ø, inerte, nicht-ionische Polymere auf Polysulfonbasis, mittlere Porengröße 10 Å), bis der Durchlauf chloridfrei ist. Die Lösung über dem Filter, die die Substanz enthält, wird mit 2 bar Druck auf 50 ml konzentriert und gefriergetrocknet. Man erhält so 3,4-Dicarboxy-3-hydroxy-buttersäure-[2-(1,2-dipalmitoyl-sn-glycero-3-hydroxyphosphoryloxy)-ethyl]-amid-trinatriumsalz · 2 $H_2O$ als farbloses Produkt mit Smp. 80 - 82° (Zers.);
$[\alpha]^{20}_D$ = +8,3° (c = 1,385; Wasser),
$R_f$ = 0,21 ($CHCl_3$ : MeOH : Phosphatpuffer [Dinatriumhydrogenphosphat-Kaliumdihydrogenphosphat; pH = 7] = 70 : 30 : 5).

**Beispiel 4:**

Gruppen von 30 weiblichen MF-2f SPF-Mäusen mit einem Körpergewicht von 14 - 16 g werden unter leichter Narkose mit einem Gemisch aus gleichen Teilen Diethylether, Ethanol und Chloroform mit letalen Dosen (ungefähr eine $LD_{80-90}$; 1 - 4 plaque forming Units [PFU]) in Form von je 0,05 ml einer Suspension von Influenza A/Texas/l/77 (Maus-adaptierter Stamm) Viren intranasal infiziert.

10 von diesen Mäusen werden zum unten angegebenen Zeitpunkt [Tage] bezogen auf den Tag der Infektion einmal (Einzeldosis) die in Tabelle 1 genannten Mengen der jeweiligen Wirksubstanz in 0,05 bzw. in 0,2 ml einer 0,005 gew.-%-igen Lösung von Carboxymethylcellulosenatriumsalz in doppelt destilliertem, pyrogenfreiem Wasser im Falle von intranasaler bzw. oraler Applikation auf die in Tabelle 1 genannte Art appliziert.

Die restlichen der obengenannten Mäuse, d.h. 20 dienen zur Kontrolle, d.h. sie erhalten ein Placebo (0,005 gew.-%-ige Lösung von Carboxymethylcellulose-natriumsalz).

Die intranasale Applikation der Wirksubstanz wird unter leichter Narkose mit einem Gemisch aus gleichen Teilen Diethylether, Ethanol und Chloroform durchgeführt.

**Tabelle 1**

| Wirksubstanz | Applikations-Art | Applikationszeit [Tage] | Prozentsatz der 23 Tage nach der Infektion noch lebenden Mäuse in Abhängigkeit von der Wirksubstanzmenge [mg/kg], statistische Signifikanz * $P \leqslant 0{,}05$, ** $P \leqslant 0{,}01$ (Vierfelder-Test) | | | | |
|---|---|---|---|---|---|---|---|
| | | | 10 | 1 | 0,1 | 0,01 | 0=Kontrolle |
| I | oral | +7 | | 70 | 60 | 80* | 30 |
| | intranasal | -7 | | 60 | 80** | 60 | 20 |
| II | oral | +7 | | 40 | 70 | 100** | 30 |
| | intranasal | -7 | | 60 | 90** | 90** | 20 |
| III | oral | +7 | 50 | 70* | 60 | | 20 |
| IV | oral | +7 | 90** | 90** | 90** | | 30 |
| | intranasal | -7 | | 100* | 80** | 50 | 20 |

Verbindung I = Glycin-2-(1,2-dipalmitoyl-sn-glycero-3-hydroxyphosphoryloxy)-ethylamid · 0,13 $H_2O$;
Verbindung II = L-Tyrosin-2-(1,2-dipalmitoyl-sn-glycero-3-hydroxyphosphoryloxy)-ethylamid;
Verbindung III = Citronensäure-mono-2-(1,2-dipalmitoyl-sn-glycero-3-hydroxyphosphoryloxy)-ethylamid-trinatriumsalz · 2 $H_2O$;
Verbindung IV = L-Alanin-2-(1,2-dipalmitoyl-sn-glycero-3-hydroxyphosphoryloxy)-ethylamid.

**Beispiel 5:**

3,80 g (3,53 mMol) $N_\alpha$-Benzyloxycarbonyl-$N_\varepsilon$-tert.butyloxycarbonyl-L-lysin-2-(1,2-dipalmitoyl-sn-glycero-3-hydroxyphosphoryloxy)-ethylamid-triethylammoniumsalz werden in 15 ml absolutem Dioxan gelöst und nach Abkühlen auf 5° durch Zugabe von 24 ml 33-%-iger Bromwasserstoffsäure in Eisessig gespalten. Die sich bildende rötliche Suspension wird 30 Minuten bei 5° gerührt, dann eingeengt und nach Zugabe von 50 ml absolutem Dioxan lyophilisiert. Der Rückstand wird durch zweimalige Chromatographie an Kieselgel (Merck, Typ 60, Korngröße 0,063 - 0,200 mm; 1 : 130, 5 ml Fraktionen) in Chloroform-Methanol-Wasser (70 : 30 : 5) gereinigt. Nach Eindampfen der reinen Fraktionen wird der Rückstand in 60 ml Chloroform-Methanol-Wasser (70 : 30 : 5) gelöst, durch ein Millipore-Filter (Hersteller: Millipore Corporation, Bedford, Massachusetts, 01730 USA, Teflon®, Typ FGLP 0,2 µm) sterilfiltriert und durch Zugabe von 100 ml absolutem Dioxan zur Kristallisation gebracht. Man erhält L-Lysin-2-[1,2-dipalmitoyl-sn-glycero-3-hydroxyphosphoryloxy]-ethylamid-monohydrobromid · 0,9 $H_2O$ in Form nadelförmiger Kristalle;

Smp. 210 - 212°,
$[\alpha]^{20}_D = +2{,}9°$ und $[\alpha]^{20}_{546\ nm} = +4°$ (jeweils c = 0,700; Chloroform : Methanol : Wasser = 70 : 30 : 5),
$R_f$ = 0,35 (Chloroform : Methanol : Wasser : Essigsäure = 70 : 40 : 10 : 0,5),
$R_f$ = 0,13 (Chloroform : Methanol : Wasser = 70 : 30 : 5).

Das Ausgangsmaterial erhält man wie folgt:

<u>Stufe 5.1</u>: 5,53 g (7,99 mMol) 2-(1,2-Dipalmitoyl-sn-glycero-3-hydroxyphosphoryloxy)-ethylamin werden in 120 ml Chloroform-Methanol-Wasser (70 : 30 : 5) suspendiert und durch Zutropfen von 1,23 ml (8,80 mMol) Triethylamin in Lösung gebracht.

Zu dieser Lösung werden 4,67 g (10,38 mMol) $N_\alpha$-Benzyloxycarbonyl-$N_\varepsilon$-tert.-butyloxycarbonyl-L-lysin-p-nitrophenylester, gelöst in 60 ml Dimethylformamid, zugetropft. Nach 16-stündigem Rühren bei RT wird die leicht gelbliche Suspension zur Trockene eingedampft. Der amorphe Rückstand wird durch Kieselgel (Merck, Typ 60, Korngröße 0,063 - 0,200 mm; 1 : 50; 5 ml Fraktionen) zuerst mit Chloroform, dann mit Chloroform-Methanol (9 : 1) von der Säule eluiert und die reinen Fraktionen werden gesammelt. Man erhält $N_\alpha$-Benzyloxycarbonyl-$N_\varepsilon$-tert.-butyloxycarbonyl-L-lysin-2-(1,2-dipalmitoyl-sn-glycero-3-hydroxyphosphoryloxy)-ethylamid--triethylammoniumsalz · 0,7 $H_2O$ als farbloses Pulver;

$[\alpha]^{20}_D = +2{,}2°$ und $[\alpha]^{20}_{546\ nm} = +2{,}7°$ (jeweils c = 0,555; Chloroform),
$R_f$ = 0,61 (Chloroform : Methanol : Wasser = 70 : 30 : 5),
$R_f$ = 0,82 (Essigsäureethylester : n-Butanol : Pyridin-Essigsäure : Wasser = 42 : 21 : 21 : 6 : 10).

**Beispiel 6:** <u>Nicht-wässrige Einzeldosis zur Nasenapplikation</u>

<u>Zusammensetzung</u>:

| | |
|---|---|
| L-Alanin-2-(1,2-dipalmitoyl-sn-glycero-3-hydroxy-phosphoryloxy)-ethylamid | 0,03 mg |
| Miglyol 812 | 30,00 mg |

Herstellung:

0,03 mg des Wirkstoffs werden unter aseptischen Bedingungen in 29,97 mg Miglyol gelöst.

Diese Lösung wird in einen handelsüblichen Einmalnasenapplikator, z. B. einen gemäß US-Patent Nr. 3 739 951, abgefüllt, der vor der Anwendung auf eine Treibstoffdose aufgesetzt wird.

**Beispiel 7:** Nasentropfen

| Zusammensetzung: | I | II |
|---|---|---|
| L-Alanin-2-(1,2-dipalmitoyl-sn-glycero-3-hydroxy-phosphoryloxy)-ethylamid | 0,15 mg | 0,10 mg |
| Thiomersal | 0,02 mg | - |
| Natriummonohydrogenphosphat · $12H_2O$ | 0,30 mg | 0,30 mg |
| Natriumdihydrogenphosphat · $2H_2O$ | 10,10 mg | 10,10 mg |
| Benzalkoniumchlorid | - | 0,10 mg |
| Ethylendiamintetraessigsäure-di-natriumsalz (EDTA) | 0,50 mg | 0,50 mg |
| Natriumchlorid | 3,70 mg | 4,50 mg |
| Entmineralisiertes Wasser | 988,30 mg | 987,60 mg |
| pH-Wert: | 5,0 ± 0,3 | 5,0 ± 0,3 |
| Gefrierpunktserniedrigung | -0,51° C | -0,56° C |

Herstellung:

In einem Teil der obengenannten Menge von entmineralisiertem Wasser werden unter Rühren Natriumdihydrogenphosphat, Dinatriumhydrogenphosphat, Natriumchlorid, Thiomersal und EDTA-Dinatriumsalz bei Raumtemperatur gelöst.

In dieser Lösung löst man anschließend den Wirkstoff auf und ergänzt mit dem restlichen entmineralisierten Wasser.

Die Lösung oder ein Teil oder ein Vielfaches davon wird durch einen Membranfilter filtriert und in gereinigte Behälter abgefüllt. Geeignete Behälter sind z. B.

a) Glas- oder Kunststoffbehälter (à 5 ml oder 10 ml), welche eine Pipette aus Glas oder Kunststoff mit einem elastomeren Pipettensauger besitzen,

b) Knautschflaschen aus Kunststoff mit einem Steigrohr und einem Sprühkopf aus Kunststoff,

c) Einzeldosisbehälter aus Kunststoff (Inhalt 2 - 3 Tropfen) oder

d) Glas- oder Kunststoffflaschen, die mit einem normierten Pumpdosierspray aus Kunststoff versehen sind (ohne Treibgas).

**Beispiel 8:** Nasensalbe

| Zusammensetzung: | |
|---|---|
| L-Alanin-2-(1,2-dipalmitoyl-sn-glycero-3-hydroxyphosphoryloxy)-ethylamid | 0,03 g |
| Paraffinöl dickflüssig | 20,00 g |
| weißes Vaselin | 30,00 g |
| Wollfett, wasserfrei | 40,00 g |
| entmineralisiertes Wasser | 19,97 g |

Herstellung:

Die Fettphase, bestehend aus Paraffinöl, Vaselin und Wollfett, wird zusammengeschmolzen. Die wässrige Lösung des Wirkstoffs wird bei ca. 50° C in die Fettphase eingearbeitet.

**Beispiel 9:** Herstellung von 1000 Tabletten, enthaltend 0,5 % Wirkstoff

| Zusammensetzung pro 1000 Tabletten: | |
|---|---|
| L-Alanin-2-(1,2-dipalmitoyl-sn-glycero-3-hydroxyphosphoryloxy)-ethylamid | 0,5 g |
| Lactose gemahlen | 43,0 g |
| Maisstärke | 52,0 g |
| Pharmacoat 603® (Hydroxypropylmethylcellulose, enthaltend 28 - 30 % | |

| | |
|---|---|
| Methoxylgruppen, geliefert von Shinetsu Chemical Company, Tokio, Japan) | 3,0 g |
| Aerosil® (kolloidales Siliziumdioxid, geliefert von Degussa, Frankfurt, Bunderepublik Deutschland) | 1,0 g |
| Magnesiumstearat | 0,5 g |

Herstellung:

Der Wirkstoff und 15 g Lactose werden vorgemischt. Die so erhaltene Vormischung wird mit 28 g Lactose und 47 g Maisstärke zusammengemischt. Mit der so erhaltenen Mischung und einer wässerigen Lösung des Pharmacoat wird eine granulierfertige Masse hergestellt, die granuliert, getrocknet und gemahlen wird. Hierzu mischt man 5 g Maisstärke, Aerosil und Magnesiumstearat und komprimiert zu 1000 Tabletten mit einem Gewicht von je 100 mg.

Die Presslinge können auf an sich bekannte Weise magensaftresistent lackiert werden.

**Beispiel 10:**

2 g N-Benzyloxycarbonyl-L-asparaginsäure-α-benzylester-2-(1,2-dipalmitoyl-sn-glycero-3-hydroxyphosphoryloxy)-ethylamid-natriumsalz löst man in Chloroform-Methanol-Wasser (60 : 40 : 2) und hydriert mit 2 g $Pd/BaSO_4$ bei Normaldruck. Man filtriert vom Katalysator und dampft das Filtrat im Vakuum zur Trockne. Den Rückstand nimmt man mit 100 ml Wasser auf und bringt den pH-Wert mit gesättigter $NaHCO_3$-Lösung auf 7. Die so erhaltene Lösung filtriert man analog Beispiel 3 in einer Amiconzelle Typ 402 über einen Ultrafilter Diaflo® PM10 unter einem $N_2$-Druck von 2 bar und konzentriert den Filterüberstand auf etwa 50 ml. Dann gibt man 300 ml 10-%-ige NaCl-Lösung zu und filtriert unter Zusatz von Wasser bis das Filtrat von Chlorid frei ist. Man erhält so nach Gefriertrocknen des Überstandes L-Asparaginsäure-β-2-(1,2-dipalmitoyl-sn-glycero-3-hydroxyphosphoryloxy)-ethylamid-natriumsalz, das man durch Digerieren mit warmem Dimethoxyethan rein erhält;

Smp. 197 - 200° (Zers.),

$R_f$ = 0,36, ($CHCl_3$ : MeOH : $H_2O$ = 60 : 40 : 2),

$R_f$ = 0,11 ($CHCl_3$ : MeOH : $H_2O$ = 80 : 20 : 2).

Das Ausgangsmaterial erhält man wie folgt:

Analog Stufe 1.1 läßt man Dipalmitoylkephalin mit dem β-N-Hydroxysuccinimidester von N-Benzyloxycarbonyl-asparaginsäure-α-benzylester reagieren. Nach Aufarbeitung analog Beispiel 3 und Chromatographie an Kieselgel in $CHCl_3$ - MeOH (9 : 1) erhält man N-Benzyloxycarbonyl-L-asparaginsäure-α-benzylester-2-(1,2-dipalmitoyl-sn-glycero-3-hydroxyphosphoryloxy)-ethylamid-natriumsalz, das noch Spuren von N-Benzyloxycarbonyl-asparaginsäure-α-benzylester enthält und direkt in die Hydrierung eingesetzt werden kann;

$R_f$ = 0,54 ($CHCl_3$ : MeOH : $H_2O$ = 80 : 20 : 2).

**Beispiel 11:**

Durch katalytische Hydrierung analog Beispiel 10 erhält man aus N-Benzyloxycarbonyl-L-asparagin-α-2-(1,2-dilauroyl-sn-glycero-3-hydroxyphosphoryloxy)-ethylamid-natriumsalzL-Asparagin-α-2-(1,2-dilauroyl-sn-glycero-3-hydroxyphosphoryloxy)-ethylamid.

Das Ausgangsmaterial erhält man analog Stufe 1.1 aus N-Benzyloxycarbonylasparagin-N'-hydroxysuccinimidester und Dilauroylkephalin und Aufarbeitung gemäß Beispiel 3.

**Beispiel 12:**

Aus N-Boc-D-alanin-2-(1,2-dipalmitoyl-sn-glycero-3-hydroxyphosphoryloxy)-ethylamid-natriumsalz erhält man analog Beispiel 1 D-Alanin-2-(1,2-dipalmitoyl-sn-glycero-3-hydroxyphosphoryloxy)-ethylamid als Hydrat;

$R_f$ = 0,61 ($CHCl_3$ : MeOH : $H_2O$ = 70 : 30 : 5),

$R_f$ = 0,53 ($CHCl_3$ : MeOH : Essigsäure : $H_2O$ = 75 : 27 : 0,5 : 5),

$[\alpha]^{20}_D$ = 14,1° ± 1,2° (c = 0,836; $CHCl_3$ : MeOH : $H_2O$ = 70 : 30 : 2).

Das Ausgangsmaterial erhält man analog Stufe 1.1 und Beispiel 3 aus Dipalmitoylkephalin und N-Boc-D-alanin-N-hydroxysuccinimidester als Natriumsalz;

$R_f$ = 0,5 ($CHCl_3$ : MeOH : $H_2O$ = 80 : 20 : 2).

**Beispiel 13:**

Analog Beispiel 1 erhält man aus $N^{\alpha}$,$N^{im}$-Di-Boc-L-histidin-2-(1,2-didecanoyl-sn-glycero-3-hydroxyphosphoryloxy)-ethylamid-natriumsalz mit Trifluoressigsäure in Methylenchlorid und nach Aufarbeitung gemäß Beispiel 3 das L-Histidin-2-(1,2-didecanoyl-sn-glycero-3-hydroxyphosphoryloxy)-ethylamid als Hydrat.

Das Ausgangsmaterial erhält man analog Stufe 1.1 und Beispiel 3 aus $N^{\alpha}$,$N^{im}$-Di-Boc-L-histidin-N-hydroxysuccinimidester und 2-(1,2-Didecanoyl-sn-glycero-3-hydroxyphosphoryloxy)-ethylamin.

**Beispiel 14:**

Zu einer Lösung von 9,69 g (0,01 Mol) N-Boc-L-tyrosin-2-(1,2-dipalmitoyl-sn-glycero-3-methoxyphosphoryloxy)-ethylamid in 100 ml Aceton gibt man 3,47 g (0,04 Mol) Lithiumbromid (purum, Fluka) und erhitzt das Ganze 3 Stunden unter Rühren und Rückfluß.

Das nach Abkühlen im Eisbad ausgefallene Produkt wird abgesaugt und mit kaltem Aceton gewaschen.

Man erhält N-Boc-L-tyrosin-2-(1,2-dipalmitoyl-sn-glycero-3-hydroxyphosphoryloxy)-ethylamid-lithiumsalz als farbloses Pulver, das noch 0,7 Mol Wasser enthält;

$R_f$ = 0,68 (Chloroform : Methanol : Wasser, 80 : 20 : 2).

Das Ausgangsmaterial erhält man wie folgt:

Analog Beispiel 1 erhält man aus 7,06 g (0,01 Mol) 2-(1,2-Dipalmitoyl-sn-glycero-3-methoxyphosphoryloxy)-ethylamin (Kephalinmethylester) und 5,46 g (1,5 Äquivalente) N-Boc-L-tyrosin-N-hydroxysuccinimidester N-Boc-L-tyrosin-2-(1,2-dipalmitoyl-sn-glycero-3-methoxyphosphoryloxy)-ethylamid;

$R_f$ = 0,85 (Chloroform : Methanol : Wasser = 80 : 20 : 2).

**Beispiel 15:**

Aus Phosphorsäure-[2-(N-Boc-L-tyrosylamido)-ethyl]-monoester-natriumsalz erhält man in bekannter Weise [H.-J. Rüger, P. Kertscher und P. Nuhn, Pharmazie 34, (1979) 287; R. Aneja, J.S. Chadha und A.P. Davies, Tetrahedron Letters 48, (1969) 4183] durch Umsetzung mit 1,2-Dipalmitoyl-sn-glycerin in absolutem Pyridin in Gegenwart von 2,4,6-Triisopropylbenzolsulfonsäurechlorid (TPS) als Kondensationsmittel N-Boc-L-tyrosin-2-(1,2-dipalmitoyl-sn-glycero-3-hydroxyphosphoryloxy)-ethylamid-natriumsalz;

Smp. = 110 - 112°,

$R_f$ = 0,64 ($CHCl_3$ : MeOH : $H_2O$ = 80 : 20 : 2).

**Beispiel 16:**

Aus N-Boc-L-tyrosin-2-(1,2-dipalmitoyl-sn-glycero-3-hydroxyphosphoryloxy)-ethylamid-natriumsalz (4,27 mMol) erhält man durch Spaltung mit Phospholipase $A_2$ (0,1 mg; aus Schweinenieren; Boehringer, Mannheim, Deutschland) bei 50° und pH 8 in Gegenwart von 5,26 mMol Calciumchlorid-dihydrat in einer gut gerührten Mischung aus 369 ml Wasser und 331 ml Diisopropylether N-Boc-L-tyrosin-2-(1-palmitoyl-sn-glycero-3-hydroxyphosphoryloxy)-ethylamid-calciumsalz (· 1/2 $Ca^{2+}$), das in bekannter Weise mit anderen Fettsäuren nach der Steglich-Methode [G. Höfle, W. Steglich und H. Vorbrüggen, Angew. Chem. 90, (1978) 602; Dicyclohexyl-carbodiimid/4-Dimethylamino-pyridin] in 2-Stellung verestert werden kann.

**Beispiel 17:**

0,5 g N-Benzyloxycarbonyl-L-glutaminsäure-γ-tertiär-butylester-α-2-(1,3-dipalmitoyl-glycero-2-hydroxyphosphoryloxy)-ethylamid-natriumsalz werden in einem Gemisch von 4 ml Trifluoressigsäure und 6 ml Methylenchlorid gelöst und 2 Stunden bei 22° stehen gelassen. Man dampft im Vakuum ein und digeriert den Rückstand mehrmals mit Petrolether-Diethylether (2 : 1). Der erhaltene pulverige Rückstand wird in 10 ml $CHCl_3$-MeOH-$H_2O$ (75 : 25 : 5) gelöst und in Gegenwart von Palladium auf Bariumsulfat hydriert. Nach beendeter Wasserstoffaufnahme filtriert man den Katalysator ab und dampft das Filtrat im Vakuum ein. Nach Digerieren mit Eiswasser erhält man pulveriges L-Glutaminsäure-α-2-(1,3-dipalmitoyl-glycero-2-hydroxyphosphoryloxy)-ethylamid.

Das Ausgangsmaterial wird wie folgt erhalten:

Stufe 17.1: Zu einer Lösung von 2 g 2-(1,3-Dipalmitoyl-glycero-2-hydroxyphosphoryloxy)-ethylamin in 30 ml $CHCl_3$-MeOH-$H_2O$ (80 : 20 : 2) werden bei 22° 1,3 g N-Benzyloxycarbonyl-L-glutaminsäure-α-1-hydroxybenzotriazolester-γ-tertiär-butylester und 0,42 ml Triethylamin zugegeben. Nach 17 Stunden bei 22° dampft man im

Vakuum ein, nimmt den Rückstand in Essigsäureethylester-Aceton (4 : 1) auf und schüttelt mit 5-%-iger Natriumhydrogencarbonatlösung. Man trennt die Phasen und wäscht die organische Phase 2-mal mit gesättigter Natriumchloridlösung. Die wäßrigen Phasen werden nochmals mit Essigsäureethylester-Aceton (4 : 1) extrahiert, die organischen Phasen getrocknet (Natriumsulfat) und eingedampft. Den Rückstand chromatographiert man an Kieselgel. Die mit $CHCl_3$-MeOH-$H_2O$ (85 : 15 : 0,5) eluierten Fraktionen enthalten N-Carboxy-L-glutaminsäure-γ-tertiär-butylester-α-2-(1,3-dipalmitoyl-glycero-2-hydroxyphosphoryloxy)-ethylamid.

**Beispiel 18:**

Entsprechend Beispiel 17 werden in N-Benzyloxycarbonyl-L-glutaminsäure-α-tertiär-butylester-γ-2-(1,3-dilauroylglycero-2-hydroxyphosphoryloxy)-ethylamid-natriumsalz die Schutzgruppen abgespalten, worauf man L-Glutaminsäure-γ-2-(1,3-dilauroylglycero-2-hydroxyphosphoryloxy)-ethylamid erhält.

Das Ausgangsmaterial wird analog Stufe 17.1 aus 2-(1,3-Dilauroylglycero-2-hydroxyphosphoryloxy)-ethylamin und N-Benzyloxycarbonylglutaminsäure-α-tertiär-butylester-γ-1-hydroxybenztriazolester hergestellt.

**Beispiel 19:**

Analog Beispiel 17 wird in N-Benzyloxycarbonyl-L-glutaminsäure-γ-methylester-α-2-(1,2-dipalmitoyl-sn-glycero-3-hydroxyphosphoryloxy)-ethylamid-natriumsalz die Benzyloxycarbonylgruppe abhydriert und L-Glutaminsäure-γ-methylester-α-2-(1,2-dipalmitoyl-sn-glycero-3-hydroxyphosphoryloxy)-ethylamid erhalten.

Das Ausgangsmaterial stellt man entsprechend Stufe 17.1 aus 2-(1,2-Dipalmitoyl-sn-glycero-3-hydroxyphosphoryloxy)-ethylamin und N-Benzyloxycarbonyl-L-glutaminsäure-α-1-hydroxybenztriazolester-γ-methylester her.

**Beispiel 20:**

Aus N-Boc-L-tyrosin-2-(1-palmitoyloxy-propyl-3-oxy-hydroxyphosphoryloxy)-ethylamid-natriumsalz erhält man beim Abspalten der Boc-Gruppe analog Beispiel 1 L-Tyrosin-2-(1-palmitoyloxy-propyl-3-oxy-hydroxyphosphoryloxy)-ethylamid.

Das Ausgangsmaterial wird entsprechend Stufe 1.1 aus 2-(1-Palmitoylpropyl-3-oxy-hydroxyphosphoryloxy)-ethylamin und N-Boc-L-tyrosin-N-hydroxysuccinimidester erhalten.

**Beispiel 21:**

Analog Beispiel 1 erhält man aus N-Boc-L-serin-2-(1,2-dihexadecyl-sn-glycero-3-hydroxyphosphoryloxy)-ethylamid-natriumsalz durch Behandeln mit Trifluoressigsäure das L-Serin-2-(1,2-dihexadecyl-sn-glycero-3-hydroxyphosphoryloxy)-ethylamid.

Das Ausgangsmaterial wird wie folgt hergestellt:

Stufe 21.1: 2 g Boc-L-serin, 6,6 g 2-(1,2-Dihexadecyl-sn-glycero-3-hydroxyphosphoryloxy)-ethylamin und 1,3 ml Triethylamin werden in 35 ml N,N-Dimethylformamid gelöst und mit 2,7 g 1-Hydroxybenztriazol sowie bei 0° mit 2,3 g N,N'-Dicyclohexylcarbodiimid versetzt. Man rührt 2 Stunden bei 0° und läßt über Nacht bei Raumtemperatur stehen. Man filtriert ab, dampft das Filtrat im Vakuum ein und arbeitet wie in Stufe 17.1 mit Essigsäureethylester-Aceton (4 : 1) auf. Man erhält das N-Boc-L-serin-2-(1,2-dihexadecyl-sn-glycero-3-hydroxyphoshoryloxy)-ethylamid.

**Beispiel 22:**

Beispiel 1 entsprechend wird beim Behandeln von N-Boc-L-prolin-2-(1,2-didecanoyl-sn-glycero-3-hydroxyphosphoryloxy)-ethylamid-natriumsalz mit Trifluoressigsäure das L-Prolin-2-(1,2-didecanoyl-sn-glycero-3-hydroxyphosphoryloxy)-ethylamid erhalten.

Das Ausgangsmaterial wird analog Stufe 17.1 aus N-Boc-L-prolin-1-hydroxybenztriazolester und 2-(1,2-Didecanoyl-sn-glycero-3-hydroxyphosphoryloxy)-ethylamin erhalten.

**Beispiel 23:**

Zu 3,1 g (0,005 Mol) 0-(1,3-Dilauroyl-glycero-2-hydroxyphosphoryl)-L-serin in 50 ml Chloroform gibt man 0,8 ml Triethylamin und 2,56 g (1,9 Äquivalente) N-Acetyl-L-leucin-N'-hydroxysuccinimidester in 10 ml Chloroform. Nach 3 Stunden bei Raumtemperatur dampft man die Lösung im Vakuum ein und arbeitet analog Beispiel 3 auf. Man erhält so das Natriumsalz von N-Acetyl-L-leucyl-O-(1,3-dilauroyl-glycero-2-hydroxyphosphoryl)-L-serin als farbloses Pulver.

**Beispiel 24:**

Zu 3,45 g (0,005 Mol) 0-(1,2-Dioleoyl-sn-glycero-3-hydroxyphosphoryl)-L-serin in 50 ml Chloroform gibt man 1 ml Triethylamin und 3 g N-Boc-L-leucin-N-hydroxysuccinimidester bei Raumtemperatur. Nach 4 Stunden dampft man im Vakuum ein und arbeitet gemäß Beispiel 3 zum Natriumsalz von N-Boc-L-leucyl-O-(1,2-dioleoyl-sn-glycero-3-hydroxyphosphoryl)-L-serin auf.

Diese Verbindung wird gemäß Beispiel 1 mit Trifluoressigsäure in Methylenchlorid zum L-Leucin-0-(1,2-dioleoyl-sn-glycero-3-hydroxyphosphoryl)-L-serin gespalten.

**Beispiel 25:**

Eine Suspension von 0,8 g 2-(1,2-Dipalmitoyl-sn-glycero-3-hydroxyphosphoryloxy)-ethylamin in 200 ml $CHCl_3$ wird bei 22° mit 0,5 ml Triethylamin und nach 15-minütigem Rühren mit 0,23 ml Benzoylchlorid versetzt. Nach 2 Stunden Rühren bei 22° wird die nunmehr klare Lösung auf ein kleines Volumen eingeengt, mit 3,5 ml einer 3-molaren Lösung von 2-Ethyl-hexansäure-natriumsalz in Methanol versetzt und zur Trockne eingedampft. Man chromatographiert an 100 g Kieselgel und eluiert mit $CHCl_3$-MeOH (7 : 3). Das ölige Produkt wird in 20 ml Aceton bei ca. 40° gelöst und auf 0° gekühlt. Dabei kristallisiert Benzoesäure-2-(1,2-dipalmitoyl-sn-glycero-3-hydroxyphosphoryloxy)-ethylamid-natriumsalz;

Smp. 60 - 61°,

$R_f = 0,68$ ($CHCl_3$ : MeOH : $H_2O$ = 60 : 40 : 3).

**Patentansprüche** für die Vertragsstaaten: BE, CH, DE, FR, GB, IT, LI, LU, NL und SE

1. Phosphatidylverbindungen der Formel I,

$$R^1\text{-T-Y-O-}\overset{\overset{\displaystyle O}{\|}}{\underset{\underset{\displaystyle OH}{|}}{P}}\text{-O-}\overset{\overset{\displaystyle W}{|}}{\underset{\underset{\displaystyle Z}{|}}{CH}} \qquad (I)$$

worin

$R^1$ $C_{3\text{-}14}$-Alkanoyl, Benzoyl, den Acylrest einer α-Amino-carbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxyethyl-, 2-Hydroxy-ethyl- oder Hydroxy-methylgruppe, in der mindestens eine der Hydroxygruppen mit einer aliphatischen $C_{8\text{-}30}$-Carbonsäure verestert oder mit einem aliphatischen $C_{8\text{-}30}$-Alkohol verethert ist,

oder W und Z je eine Hydroxymethylgruppe, die mit einer aliphatischen $C_{8\text{-}30}$-Carbonsäure verestert oder mit einem aliphatischen $C_{8\text{-}30}$-Alkohol verethert ist, bedeuten,

wobei das vorstehend verwendete Präfix "Nieder" Reste bis und mit 7 Kohlenstoffatomen bezeichnet, und ihre Salze.

2. Verbindungen der Formel I nach Anspruch 1, worin $R^1$ den Acylrest einer α-Amino-carbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert ist, bedeutet, und ihre Salze.

3. Verbindungen der Formel I nach Anspruch 1 oder 2, worin W Wasserstoff und Z eine 1,2-Dihydroxy-ethylgruppe, in der mindestens eine der Hydroxygruppen mit einer aliphatischen $C_{8-30}$-Carbonsäure verestert ist, bedeuten, und ihre Salze.

4. Verbindungen der Formel I nach Anspruch 1, worin $R^1$ von Glycyl, L-Alanyl und deren Derivaten mit substituierter Aminogruppe verschiedenes α-Amino-niederalkanoyl, das durch eine weitere Amino-, Niederalkanoylamino-, Niederalkoxycarbonylamino- oder Benzyloxycarbonylaminogruppe oder durch Guanidino, Carboxy, Phenyloxycarbonyl, Benzyloxycarbonyl, Benzhydryloxycarbonyl, Niederalkoxycarbonyl, Carbamoyl, Hydroxy, Niederalkoxy, Niederalkanoyloxy, Niederalkoxycarbonyloxy, Mercapto, Niederalkylthio, (2-Amino-2-carboxy-ethyl)-dithio, Phenyl, 4-Hydroxy-phenyl, Imidazol-4-yl oder Indol-3-yl substituiert sein kann, oder unsubstituiertes oder durch Hydroxy, Niederalkoxy, Niederalkanoyloxy oder Niederalkoxycarbonyloxy substituiertes Pyrrolidin-α-carbonyl, T die Gruppe NH oder Sauerstoff, Y unsubstituiertes oder durch Carboxy, Niederalkoxycarbonyl oder Carbamoyl substituiertes Dimethylen, W Wasserstoff und Z 1,2-Dihydroxy-ethyl, worin entweder nur die 2-Hydroxy-Gruppe oder beide Hydroxygruppen mit einer $C_{12-24}$-Alkan- oder Alkensäure verestert sind, oder W und Z je eine mit einer $C_{12-24}$-Alkan- oder Alkensäure veresterte Hydroxymethylgruppe bedeuten, und ihre Salze.

5. Verbindungen der Formel I nach Anspruch 1 oder 3, worin $R^1$ den von Glycyl, L-Alanyl und deren Derivaten mit substituierter Aminogruppe verschiedenen Acylrest einer durch α-Amino sowie gegebenenfalls eine weitere Aminogruppe, ein oder zwei Carboxy, Hydroxy, Mercapto, Niederalkylthio, Phenyl, 4-Hydroxy-phenyl, Carbamoyl, Guanidino, 3-Indolyl, 4-Imidazolyl oder (2-Amino-2-carboxy-ethyl)-dithio substituierten Alkansäure mit bis zu 12 C-Atomen oder einen Benzoyl- oder Prolylrest bedeutet, und ihre Salze.

6. Verbindungen der Formel I nach Anspruch 1 oder 3, worin $R^1$ den Acylrest einer jener 20 Aminosäuren, die regelmässig in Proteinen vorkommen, mit Ausnahme von Glycin und L-Alanin oder den Acylrest von Milchsäure oder Citronensäure bedeutet, und ihre Salze.

7. Verbindungen der Formel I nach Anspruch 1, worin $R^1$ Benzoyl oder den Acylrest von L-Tyrosin, L-Lysin, L-Asparaginsäure, L-Asparagin, L-Histidin, L-Glutaminsäure, L-Serin, L-Prolin, L-Leucin oder D-Alanin, wobei in diesen Acylresten vorhandene Aminogruppen am Stickstoff durch tert.-Butyloxycarbonyl, Benzyloxycarbonyl oder Niederalkanoyl substituiert und vorhandene Carboxygruppen mit einem Niederalkanol verestert sein können, T die Gruppe NH, Y unsubstituiertes oder durch Carboxy oder Niederalkoxycarbonyl substituiertes Dimethylen, W Wasserstoff und Z eine 1,2-Dihydroxyethyl-, 2-Hydroxy-ethyl- oder Hydroxymethylgruppe, in der mindestens eine der Hydroxygruppen mit einer $C_{10-18}$-Alkansäure oder einer $C_{18}$-Alkensäure verestert ist, oder W und Z je eine Hydroxymethylgruppe, die mit einer $C_{10-18}$-Alkansäure oder einer $C_{18}$-Alkensäure verestert ist, bedeuten, und ihre Salze.

8. Verbindungen der Formel I nach Anspruch 7, worin $R^1$ den Acylrest von L-Tyrosin, L-Lysin, L-Asparaginsäure, L-Asparagin, L-Histidin, L-Glutaminsäure, L-Serin, L-Prolin, L-Leucin oder D-Alanin, wobei in diesen Acylresten vorhandene Aminogruppen am Stickstoff durch tert.-Butyloxycarbonyl, Benzyloxycarbonyl oder Niederalkanoyl substituiert sind, bedeutet, und ihre Salze.

9. Verbindungen der Formel I nach Anspruch 1, worin $R^1$ den Acylrest von L-Valin, L-Serin, L-Threonin, L-Cystein, L-Methionin, L-Tyrosin oder Citronensäure, T die Gruppe NH, Y unsubstituiertes oder durch Carboxy substituiertes Dimethylen, W Wasserstoff und Z 1,2-Dihydroxy-ethyl, worin beide Hydroxygruppen mit einer geradkettigen $C_{16-24}$-Alkan- oder Alkensäure mit gerader Anzahl C-Atome verestert sind, bedeuten, und ihre Salze.

10. Verbindungen der Formel I nach einem der Ansprüche 1 - 8, worin Y durch Carboxy oder Niederalkoxycarbonyl substituiertes Dimethylen bedeutet, und ihre Salze.

11. Verbindungen der Formel I nach einem der Ansprüche 1, 2 und 4 - 8, worin W und Z je eine Hydroxymethylgruppe, die mit einer $C_{18}$-Alkensäure oder mit einer geradkettigen $C_{12}$-$C_{18}$-Alkansäure verestert ist, bedeuten, und ihre Salze.

12. Verbindungen der Formel I nach einem der Ansprüche 1 - 11, worin T für die Gruppe NH steht, und ihre Salze.

13. Verbindungen der Formel I nach einem der Ansprüche 1 - 9, 11 und 12, worin Y unsubstituiertes Dimethylen bedeutet, und ihre Salze.

14. Verbindungen der Formel I nach einem der Ansprüche 1 - 13 in Form ihrer pharmazeutisch verwendbaren Salze.

15. Verbindungen der Formel I nach einem der Ansprüche 1, 3, 4 und 10 - 13, worin $R^1$ den Acylrest von L-Tyrosin oder von N-Niederalkoxycarbonyl- oder N-Niederalkanoyl-L-tyrosin bedeutet, und ihre pharmazeutisch verwendbaren Salze.

16. Verbindungen der Formel I nach einem der Ansprüche 1, 3, 4 und 10 - 13, worin $R^1$ den Acylrest von L-Leucin oder von N-Niederalkoxycarbonyl- oder N-Niederalkanoyl-L-leucin bedeutet, und ihre pharmazeutisch verwendbaren Salze.

17. Verbindungen der Formel I nach einem der Ansprüche 1, 3, 4 und 10 - 13, worin $R^1$ den Acylrest von L-Lysin oder von $N^{\alpha}$-Benzyloxycarbonyl-$N^{\varepsilon}$-niederalkoxycarbonyl-L-lysin bedeutet, und ihre pharmazeutisch verwendbaren Salze.

18. L-Tyrosin-2-(1,2-dipalmitoyl-sn-glycero-3-hydroxyphosphoryloxy)-ethylamid oder ein pharmazeutisch verwendbares Salz davon nach Anspruch 1.

19. 3,4-Dicarboxy-3-hydroxy-buttersäure-2-(1,2-dipalmitoyl-sn-glycero-3-hydroxyphosphoryloxy)-ethylamid oder ein pharmazeutisch verwendbares Salz davon nach Anspruch 1.

20. Eine Verbindung nach Anspruch 1, ausgewählt aus der Gruppe, bestehend aus L-Lysin-2-[1,2-dipalmitoyl-sn-glycero-3-hydroxyphosphoryloxy]-ethylamid, $N^{\alpha}$-Benzyloxycarbonyl-$N^{\varepsilon}$-tert.-butyloxycarbonyl-L-lysin-2-(1,2-dipalmitoyl-sn-glycero-3-hydroxyphosphoryloxy)-ethylamid, N-Boc-L-tyrosin-2-(1,2-dipalmitoyl-sn-glycero-3-hydroxyphosphoryloxy)-ethylamid, N-Benzyloxycarbonyl-L-asparaginsäure-α-benzylester-2-(1,2-dipalmitoyl-sn-glycero-3-hydroxyphosphoryloxy)-ethylamid, L-Asparaginsäure-β-2-(1,2-dipalmitoyl-sn-glycero-3-hydroxyphosphoryloxy)-ethylamid, N-Benzyloxycarbonyl-L-asparagin-α-2-(1,2-dilauroyl-sn-glycero-3-hydroxyphosphoryloxy)-ethylamid, L-Asparagin-α-2-(1,2-dilauroyl-sn-glycero-3-hydroxyphosphoryloxy)-ethylamid, N-Boc-D-alanin-2-(1,2-dipalmitoyl-sn-glycero-3-hydroxyphosphoryloxy)-ethylamid, D-Alanin-2-(1,2-dipalmitoyl-sn-glycero-3-hydroxyphosphoryloxy)-ethylamid, $N^{\alpha}$,$N^{im}$-Di-Boc-L-histidin-2-(1,2-didecanoyl-sn-glycero-3-hydroxyphosphoryloxy)-ethylamid, L-Histidin-2-(1,2-didecanoyl-sn-glycero-3-hydroxyphosphoryloxy)-ethylamid, N-Benzyloxycarbonyl-L-glutaminsäure-γ-tertiär-butylester-α-2-(1,3-dipalmitoyl-glycero-2-hydroxyphosphoryloxy)-ethylamid, L-Glutaminsäure-α-2-(1,3-dipalmitoyl-glycero-2-hydroxyphosphoryloxy)-ethylamid, N-Benzyloxycarbonyl-L-glutaminsäure-α-tertiär-butylester-γ-2-(1,3-dilauroyl-glycero-2-hydroxyphosphoryloxy)-ethylamid, L-Glutaminsäure-γ-2-(1,3-dilauroyl-glycero-2-hydroxyphosphoryloxy)-ethylamid, N-Benzyloxycarbonyl-L-glutaminsäure-γ-methylester-α-2-(1,2-dipalmitoyl-sn-glycero-3-hydroxyphosphoryloxy)-ethylamid, L-Glutaminsäure-γ-methylester-α-2-(1,2-dipalmitoyl-sn-glycero-3-hydroxyphosphoryloxy)-ethylamid, N-Boc-L-tyrosin-2-(1-palmitoyloxypropyl-3-oxyhydroxyphosphoryloxy)-ethylamid, L-Tyrosin-2-(1-palmitoyloxy-propyl-3-oxyhydroxyphosphoryloxy)-ethylamid, N-Boc-L-serin-2-(1,2-dihexadecyl-sn-glycero-3-hydroxyphosphoryloxy)-ethylamid, L-Serin-2-(1,2-dihexadecyl-sn-glycero-3-hydroxyphosphoryloxy)-ethylamid, N-Boc-L-prolin-2-(1,2-didecanoyl-sn-glycero-3-hydroxyphosphoryloxy)-ethylamid, L-Prolin-2-(1,2-didecanoyl-sn-glycero-3-hydroxyphosphoryloxy)-ethylamid, N-Acetyl-L-leucyl-O-(1,3-dilauroyl-glycero-2-hydroxyphosphoryl)-L-serin, N-Boc-L-leucyl-O-(1,2-dioleoyl-sn-glycero-3-hydroxyphosphoryl)-L-serin, L-Leucin-O-(1,2-dioleoyl-sn-glycero-3-hydroxyphosphoryl)-L-serin oder einem pharmazeutisch verwendbaren Salz der obengenannten Verbindungen.

21. Phosphatidylverbindungen der Formel Ia,

```
               O   W
               ||  |
R1a-T-Y-O-P-O-CH          (Ia)
               |   |
               OH  Z
```

worin

R $C_{2-22}$-Alkanoyl, das durch Carboxy substituiert sein kann, Propenoyl, 2-Methyl-propenoyl, Benzoyl, den Acylrest einer α-Aminocarbonsäure, deren α-Amino-Gruppe durch Niederalkyl, Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxy-ethyl-, 2-Hydroxy-ethyl- oder Hydroxy-methylgruppe, in der mindestens eine der Hydroxygruppen mit einer aliphatischen $C_{8-30}$-Carbonsäure verestert oder mit einem aliphatischen $C_{8-30}$-Alkohol verethert ist,

oder W und Z je eine Hydroxymethylgruppe, die mit einer aliphatischen $C_{8-30}$-Carbonsäure verestert oder mit einem aliphatischen $C_{8-30}$-Alkohol verethert ist, bedeuten,

wobei das vorstehend verwendete Präfix "Nieder" Reste bis und mit 7 Kohlenstoffatomen bezeichnet, und ihre pharmazeutisch verwendbaren Salze zur Anwendung in einem Verfahren zur therapeutischen Behandlung des menschlichen oder tierischen Körpers.

22. L-Alanin-2-(1,2-dipalmitoyl-sn-glycero-3-hydroxyphosphoryloxy)-ethylamid oder ein pharmazeutisch verwendbares Salz davon nach Anspruch 21 zur Anwendung in einem Verfahren zur therapeutischen Behandlung des menschlichen oder tierischen Körpers.

23. Eine Verbindung der Formel I nach einem der Ansprüche 1 - 20 oder ein pharmazeutisch verwendbares Salz davon zur Anwendung in einem Verfahren zur therapeutischen Behandlung des menschlichen oder tierischen Körpers.

24. Pharmazeutisches Präparat, enthaltend als Wirkstoff eine zur Prophylaxe oder Therapie einer Virusinfektion wirksame Menge einer Verbindung der Formel I nach einem der Ansprüche 1 - 20 oder ein pharmazeutisch verwendbares Salz davon zusammen mit einer signifikanten Menge eines pharmazeutischen Trägermaterials.

25. Verwendung einer Verbindung der Formel Ia,

```
               O   W
               ||  |
R1a-T-Y-O-P-O-CH          (Ia)
               |   |
               OH  Z
```

worin

$R^{1a}$ $C_{2-22}$-Alkanoyl, das durch Carboxy substituiert sein kann, $C_{3-18}$-Alkenoyl, Benzoyl, den Acylrest einer α-

Amino-carbonsäure, deren α-Amino-Gruppe durch Niederalkyl, Niederalkanoyl, Niederalkoxycarbonyl oder Benzyloxycarbonyl substituiert sein kann, oder den Acylrest einer β-Amino-carbonsäure oder einer α- oder β-Hydroxycarbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxy-ethyl-, 2-Hydroxy-ethyl- oder Hydroxy-methylgruppe, in der mindestens eine der Hydroxygruppen mit einer aliphatischen $C_{8-30}$-Carbonsäure verestert oder mit einem aliphatischen $C_{8-30}$-Alkohol verethert ist,

oder W und Z je eine Hydroxymethylgruppe, die mit einer aliphatischen $C_{8-30}$-Carbonsäure verestert oder mit einem aliphatischen $C_{8-30}$-Alkohol verethert ist, bedeuten,

wobei das vorstehend verwendete Präfix "Nieder" Reste bis und mit 7 Kohlenstoffatomen bezeichnet, oder eines pharmazeutisch verwendbaren Salzes davon zur Herstellung eines Arzneimittels für die Anwendung zur Prophylaxe oder Therapie einer Virusinfektion.

26. Verwendung einer Verbindung der Formel I nach einem der Ansprüche 1 - 20 oder eines pharmazeutisch verwendbaren Salzes davon zur Herstellung eines Arzneimittels für die Anwendung zur Prophylaxe oder Therapie einer Virusinfektion.

27. Verfahren zur Herstellung einer Verbindung der Formel I,

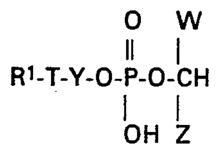

(I)

worin

$R^1$ $C_{3-14}$-Alkanoyl, Benzoyl, den Acylrest einer α-Amino-carbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxy-ethyl-, 2-Hydroxy-ethyl- oder Hydroxy-methylgruppe, in der mindestens eine der Hydroxygruppen mit einer aliphatischen $C_{8-30}$-Carbonsäure verestert oder mit einem aliphatischen $C_{8-30}$-Alkohol verethert ist,

oder W und Z je eine Hydroxymethylgruppe, die mit einer aliphatischen $C_{8-30}$-Carbonsäure verestert oder mit einem aliphatischen $C_{8-30}$-Alkohol verethert ist, bedeuten,

wobei das vorstehend verwendete Präfix "Nieder" Reste bis und mit 7 Kohlenstoffatomen bezeichnet, oder eines Salzes davon, dadurch gekennzeichnet, daß man

a) eine Verbindung der Formel II,

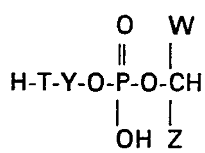

(II)

worin die Substituenten die obengenannten Bedeutungen haben, wobei in einer Verbindung der Formel II vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel III,

$R^1$ - OH (III)

worin $R^1$ die obengenannte Bedeutung hat, wobei in einer Verbindung der Formel III vorhandene funktionelle Gruppen mit Ausnahme der an der Reaktion teilnehmenden Gruppe erforderlichenfalls in geschützter Form vorliegen, oder mit einem reaktionsfähigen Carbonsäurederivat davon umsetzt und vorhandene Schutzgruppen abspaltet, oder

b) eine Verbindung der Formel IV,

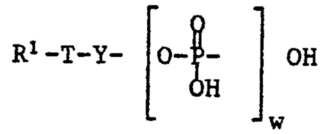 (IV)

worin w für 0 oder 1 steht und $R^1$, T und Y die obengenannten Bedeutungen haben, wobei in einer Verbindung der Formel IV vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel V

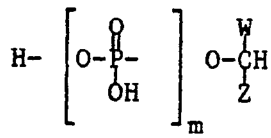 (V)

worin W und Z die obengenannten Bedeutungen haben, wobei in einer Verbindung der Formel V vorhandene funktionelle Gruppen mit Ausnahme der an der Reaktion teilnehmenden Gruppe erforderlichenfalls in geschützter Form vorliegen, und m für 1 steht, wenn im Reaktionspartner der Formel IV w für 0 steht, oder m für 0 steht, wenn w für 1 steht, oder mit einem reaktionsfähigen Derivat einer Verbindung der Formel V umsetzt und vorhandene Schutzgruppen abspaltet, oder

c) eine Verbindung der Formel VI,

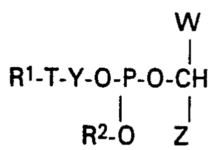 (VI)

worin $R^2$ für Wasserstoff oder eine Schutzgruppe steht und die übrigen Substituenten die obengenannten Bedeutungen haben, wobei in einer Verbindung der Formel VI vorhandene funktionelle Gruppen mit Ausnahme der an der Reaktion teilnehmenden Gruppe erforderlichenfalls in geschützter Form vorliegen, oder ein Tautomeres einer Verbindung der Formel VI mit einem Oxidationsmittel oxidiert und vorhandene Schutzgruppen abspaltet, oder

d) eine Verbindung der Formel VII,

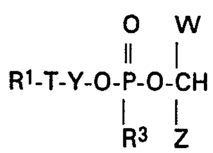 (VII)

worin $R^3$ Halogen bedeutet und die übrigen Substituenten die obengenannten Bedeutungen haben, wobei in einer Verbindung der Formel VII vorhandene funktionelle Gruppen mit Ausnahme der an der Reaktion teilnehmenden Gruppe erforderlichenfalls in geschützter Form vorliegen, hydrolysiert und vorhandene Schutzgruppen abspaltet, oder

e) eine Verbindung der Formel VIII,

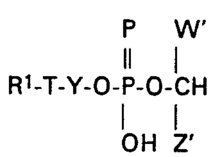 (VIII)

worin W, Wasserstoff und Z' 1,2-Dihydroxy-ethyl, 2-Hydroxy-ethyl, Hydroxymethyl oder 1,2-Dihydroxy-ethyl, worin eine der beiden Hydroxygruppen mit einer aliphatischen $C_{8-30}$-Carbonsäure verestert oder mit einem aliphatischen $C_{8-30}$-Alkohol verethert ist, oder einer der Reste W' und Z' Hydroxymethyl und der andere der Reste W' und Z' freies Hydroxymethyl oder Hydroxymethyl, das mit einer aliphatischen $C_{8-30}$-Carbonsäure verestert oder mit einem aliphatischen $C_{8-30}$-Alkohol verethert ist, und $R^1$, T und Y die obengenannten Bedeutungen haben, wobei in einer Verbindung der Formel VIII vorhandene funktionelle Gruppen mit Ausnahme der an der Reaktion teilnehmenden Gruppe erforderlichenfalls in geschützter Form vorliegen, oder ein reaktionsfähiges Derivat davon, mit einer aliphatischen $C_{8-30}$-Carbonsäure oder einem reaktionsfähigen

Derivat davon verestert oder mit einem aliphatischen $C_{8-30}$-Alkohol oder einem reaktionsfähigen Derivat davon verethert und vorhandene Schutzgruppen abspaltet, oder

f) eine Verbindung der Formel IX,

```
          O  W
          ‖  |
X-Y-O-P-O-CH                                   (IX)
          |  |
          OH Z
```

worin X für eine nucleophile Abgangsgruppe steht und die übrigen Substituenten die obengenannten Bedeutungen haben, wobei in einer Verbindung der Formel IX vorhandene funktionelle Gruppen mit Ausnahme der an der Reaktion teilnehmenden Gruppe erforderlichenfalls in geschützter Form vorliegen, mit einer Verbindung der Formel X,

$R^1$ - T - H (X)

worin die Substituenten die obengenannten Bedeutungen haben, wobei in einer Verbindung der Formel X vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzt und vorhandene Schutzgruppen abspaltet, oder

g) in einer Verbindung der Formel I, worin die Substituenten die obengenannten Bedeutungen haben, wobei in einer Verbindung der Formel I mindestens eine funktionelle Gruppe durch eine leicht abspaltbare Schutzgruppe geschützt sein muß, die Schutzgruppe(n) abspaltet, oder

h) zur Herstellung einer Verbindung der Formel I, worin W für Wasserstoff und Z für eine 1,2-Dihydroxy-ethyl-gruppe steht, worin die 2-Hydroxygruppe mit einer aliphatischen $C_{8-30}$-Carbonsäure verestert ist, eine Verbindung der Formel I, worin W für Wasserstoff und Z für eine 1,2-Dihydroxy-ethyl-gruppe steht, worin beide Hydroxygruppen mit einer aliphatischen $C_{8-30}$-Carbonsäure verestert sind, mit einem zur regioselektiven Abspaltung des die 1-Hydroxygruppe veresternden Restes geeigneten Enzym umsetzt

und nach Ausführung einer der obengenannten Verfahrensvarianten a - h) zur Herstellung eines Salzes erforderlichenfalls eine Verbindung der Formel I in ein Salz überführt oder zur Überführung einer Verbindung der Formel I oder eines Salzes davon in eine andere Verbindung der Formel I oder in ein Salz davon eine im Rest $R^1$ enthaltene Amino- oder Hydroxygruppe acyliert oder eine im Rest $R^1$ oder Y enthaltene Carboxylgruppe verestert oder amidiert.

**Patentansprüche** für den Vertragsstaat: AT

1. Verfahren zur Herstellung einer Verbindung der Formel I.

```
             O  W
             ‖  |
R1-T-Y-O-P-O-CH                                (I)
             |  |
             OH Z
```

worin

$R^1$ $C_{3-14}$-Alkanoyl, Benzoyl, den Acylrest einer α-Amino-carbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert sein kann, oder den Acylrest einer β-Amino-carbonsäure oder einer α- oder β-Hydroxy-carbonsäure,

T die unsubstituierte oder durch Niederalkyl substituierte Gruppe NH oder Sauerstoff,

Y unsubstituiertes oder durch freies, verethertes oder amidiertes Carboxy substituiertes Dimethylen,

W Wasserstoff und

Z eine 1,2-Dihydroxy-ethyl-, 2-Hydroxy-ethyl- oder Hydroxy-methylgruppe, in der mindestens eine der Hydroxygruppen mit einer aliphatischen $C_{8-30}$-Carbonsäure verestert oder mit einem aliphatischen $C_{8-30}$-Alkohol verethert ist,

oder W und Z je eine Hydroxymethylgruppe, die mit einer aliphatischen $C_{8-30}$-Carbonsäure verestert oder mit einem aliphatischen $C_{8-30}$-Alkohol verethert ist, bedeuten, wobei das vorstehend verwendete Präfix "Nieder" Reste bis und mit 7 Kohlenstoffatomen bezeichnet, oder eines Salzes davon, dadurch gekennzeichnet, daß man

a) eine Verbindung der Formel II,

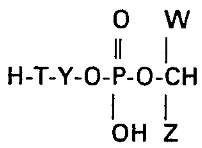

(II)

worin die Substituenten die obengenannten Bedeutungen haben, wobei in einer Verbindung der Formel II vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel III,

$R^1$ - OH (III)

worin $R^1$ die obengenannte Bedeutung hat, wobei in einer Verbindung der Formel III vorhandene funktionelle Gruppen mit Ausnahme der an der Reaktion teilnehmenden Gruppe erforderlichenfalls in geschützter Form vorliegen, oder mit einem reaktionsfähigen Carbonsäurederivat davon umsetzt und vorhandene Schutzgruppen abspaltet, oder

b) eine Verbindung der Formel IV,

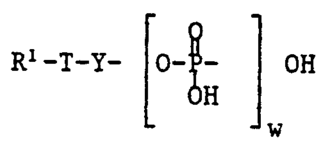

(IV)

worin w für 0 oder 1 steht und $R^1$, T und Y die obengenannten Bedeutungen haben, wobei in einer Verbindung der Formel IV vorhandene funktionelle Gruppen mit Ausnahme der an der Reaktion teilnehmenden Gruppe erforderlichenfalls in geschützter Form vorliegen, oder ein reaktionsfähiges Derivat davon mit einer Verbindung der Formel V

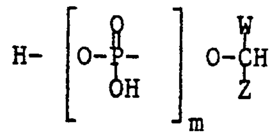

(V)

worin W und Z die obengenannten Bedeutungen haben, wobei in einer Verbindung der Formel V vorhandene funktionelle Gruppen mit Ausnahme der an der Reaktion teilnehmenden Gruppe erforderlichenfalls in geschützter Form vorliegen, und m für 1 steht, wenn im Reaktionspartner der Formel IV w für 0 steht, oder m für 0 steht, wenn w für 1 steht, oder mit einem reaktionsfähigen Derivat einer Verbindung der Formel V umsetzt und vorhandene Schutzgruppen abspaltet, oder

c) eine Verbindung der Formel VI;

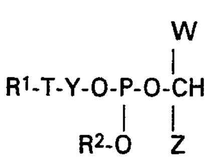

(VI)

worin $R^2$ für Wasserstoff oder eine Schutzgruppe steht und die übrigen Substituenten die obengenannten Bedeutungen haben, wobei in einer Verbindung der Formel VI vorhandene funktionelle Gruppen mit Ausnahme der an der Reaktion teilnehmenden Gruppe erforderlichenfalls in geschützter Form vorliegen, oder ein Tautomeres einer Verbindung der Formel VI mit einem Oxidationsmittel oxidiert und vorhandene Schutzgruppen abspaltet, oder

d) eine Verbindung der Formel VII,

```
           O   W
           ‖   |
R1-T-Y-O-P-O-CH                (VII)
           |   |
           R3  Z
```

worin $R^3$ Halogen bedeutet und die übrigen Substituenten die obengenannten Bedeutungen haben, wobei in einer Verbindung der Formel VII vorhandene funktionelle Gruppen mit Ausnahme der an der Reaktion teilnehmenden Gruppe erforderlichenfalls in geschützter Form vorliegen, hydrolysiert und vorhandene Schutzgruppen abspaltet, oder

e) eine Verbindung der Formel VIII,

```
           O   W'
           ‖   |
R1-T-Y-O-P-O-CH                (VIII)
           |   |
           OH  Z'
```

worin W' Wasserstoff und Z' 1,2-Dihydroxy-ethyl, 2-Hydroxy-ethyl, Hydroxymethyl oder 1,2-Dihydroxy-ethyl, worin eine der beiden Hydroxygruppen mit einer aliphatischen $C_{8-30}$-Carbonsäure verestert oder mit einem aliphatischen $C_{8-30}$-Alkohol verethert ist, oder einer der Reste W' und Z' Hydroxymethyl und der andere der Reste W' und Z' freies Hydroxymethyl oder Hydroxymethyl, das mit einer aliphatischen $C_{8-30}$-Carbonsäure verestert oder mit einem aliphatischen $C_{8-30}$-Alkohol verethert ist, und $R^1$, T und Y die obengenannten Bedeutungen haben, wobei in einer Verbindung der Formel VIII vorhandene funktionelle Gruppen mit Ausnahme der an der Reaktion teilnehmenden Gruppe erforderlichenfalls in geschützter Form vorliegen, oder ein reaktionsfähiges Derivat davon, mit einer aliphatischen $C_{8-30}$-Carbonsäure oder einem reaktionsfähigen Derivat davon verestert oder mit einem aliphatischen $C_{8-30}$-Alkohol oder einem reaktionsfähigen Derivat davon verethert und vorhandene Schutzgruppen abspaltet, oder

f) eine Verbindung der Formel IX,

```
          O   W
          ‖   |
X-Y-O-P-O-CH                   (IX)
          |   |
          OH  Z
```

worin X für eine nucleophile Abgangsgruppe steht und die übrigen Substituenten die obengenannten Bedeutungen haben, wobei in einer Verbindung der Formel IX vorhandene funktionelle Gruppen mit Ausnahme der an der Reaktion teilnehmenden Gruppe erforderlichenfalls in geschützter Form vorliegen, mit einer Verbindung der Formel X,

$R^1$ - T - H (X)

worin die Substituenten die obengenannten Bedeutungen haben, wobei in einer Verbindung der Formel X vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzt und vorhandene Schutzgruppen abspaltet, oder

g) in einer Verbindung der Formel I, worin die Substituenten die obengenannten Bedeutungen haben, wobei in einer Verbindung der Formel I mindestens eine funktionelle Gruppe durch eine leicht abspaltbare Schutzgruppe geschützt sein muß, die Schutzgruppe(n) abspaltet, oder

h) zur Herstellung einer Verbindung der Formel I, worin W für Wasserstoff und Z für eine 1,2-Dihydroxy-ethylgruppe steht, worin die 2-Hydroxygruppe mit einer aliphatischen $C_{8-30}$-Carbonsäure verestert ist, eine Verbindung der Formel I, worin W für Wasserstoff und Z für eine 1,2-Dihydroxy-ethyl-gruppe steht, worin beide Hydroxygruppen mit einer aliphatischen $C_{8-30}$-Carbonsäure verestert sind, mit einem zur regioselektiven Abspaltung des die 1-Hydroxygruppe veresternden Restes geeigneten Enzym umsetzt und nach Ausführung einer der obengenannten Verfahrensvarianten a) - h) zur Herstellung eines Salzes erforderlichenfalls eine Verbindung der Formel I in ein Salz überführt oder zur Überführung einer Verbindung der Formel I oder eines Salzes davon in eine andere Verbindung der Formel I oder in ein Salz davon eine im Rest $R^1$ enthaltene Amino- oder Hydroxygruppe acyliert oder eine im Rest $R^1$ oder Y enthaltene Carboxylgruppe verestert oder amidiert.

2. Verfahren nach Anspruch 1, dadurch gekennzeichnet, daß man die Ausgangsverbindungen so wählt, daß

man eine Verbindung der Formel I oder ein Salz davon herstellt, worin $R^1$ den Acylrest einer α-Aminocarbonsäure, die von Glycin, L-Alanin und deren Derivaten mit substituierter Aminogruppe verschieden ist, und deren α-Amino-Gruppe durch Niederalkanoyl, Niederalkoxycarbonyl oder Benzyloxycarbonyl substituiert ist, bedeutet.

3. Verfahren nach Anspruch 1 oder 2, dadurch gekennzeichnet, daß man die Ausgangsverbindungen so wählt, daß man eine Verbindung der Formel I oder ein Salz davon herstellt, worin W Wasserstoff und Z eine 1,2-Dihydroxy-ethylgruppe, in der mindestens eine der Hydroxygruppen mit einer aliphatischen $C_{8-30}$-Carbonsäure verestert ist, bedeuten.

4. Verfahren nach Anspruch 1, dadurch gekennzeichnet, daß man die Ausgangsverbindungen so wählt, daß man eine Verbindung der Formel I oder ein Salz davon herstellt, worin $R^1$ von Glycyl, L-Alanyl und deren Derivaten mit substituierter Aminogruppe verschiedenes α-Amino-niederalkanoyl, das durch eine weitere Amino-, Niederalkanoylamino-, Niederalkoxycarbonylamino- oder Benzyloxycarbonylaminogruppe oder durch Guanidino, Carboxy, Phenyloxycarbonyl, Benzyloxycarbonyl, Benzhydryloxycarbonyl, Niederalkoxycarbonyl, Carbamoyl, Hydroxy, Niederalkoxy, Niederalkanoyloxy, Niederalkoxycarbonyloxy, Mercapto, Niederalkylthio, (2-Amino-2-carboxy-ethyl)-dithio, Phenyl, 4-Hydroxy-phenyl, Imidazol-4-yl oder Indol-3-yl substituiert sein kann, oder unsubstituiertes oder durch Hydroxy, Niederalkoxy, Niederalkanoyloxy oder Niederalkoxycarbonyloxy substituiertes Pyrrolidin-α-carbonyl, T die Gruppe NH oder Sauerstoff, Y unsubstituiertes oder durch Carboxy, Niederalkoxycarbonyl oder Carbamoyl substituiertes Dimethylen, W Wasserstoff und Z 1,2-Dihydroxyethyl, worin entweder nur die 2-Hydroxy-Gruppe oder beide Hydroxygruppen mit einer $C_{12-24}$-Alkan- oder Alkensäure verestert sind, oder W und Z je eine mit einer $C_{12-24}$-Alkan- oder Alkensäure veresterte Hydroxymethylgruppe bedeuten.

5. Verfahren nach Anspruch 1 oder 3, dadurch gekennzeichnet, daß man die Ausgangsverbindungen so wählt, daß man eine Verbindung der Formel I oder ein Salz davon herstellt, worin $R^1$ den von Glycyl, L-Alanyl und deren Derivaten mit substituierter Aminogruppe verschiedenen Acylrest einer durch α-Amino sowie gegebenenfalls eine weitere Aminogruppe, ein oder zwei Carboxy, Hydroxy, Mercapto, Niederalkylthio, Phenyl, 4-Hydroxy-phenyl, Carbamoyl, Guanidino, 3-Indolyl, 4-Imidazolyl oder (2-Amino-2-carboxy-ethyl)-dithio substituierten Alkansäure mit bis zu 12 C-Atomen oder einen Benzoyl- oder Prolylrest bedeutet.

6. Verfahren nach Anspruch 1 oder 3, dadurch gekennzeichnet, daß man die Ausgangsverbindungen so wählt, daß man eine Verbindung der Formel I oder ein Salz davon herstellt, worin $R^1$ den Acylrest einer jener 20 Aminosäuren, die regelmässig in Proteinen vorkommen, mit Ausnahme von Glycin und L-Alanin oder den Acylrest von Milchsäure oder Citronensäure bedeutet.

7. Verfahren nach Anspruch 1, dadurch gekennzeichnet, daß man die Ausgangsverbindungen so wählt, daß man eine Verbindung der Formel I oder ein Salz davon herstellt, worin $R^1$ Benzoyl oder den Acylrest von L-Tyrosin, L-Lysin, L-Asparaginsäure, L-Asparagin, L-Histidin, L-Glutaminsäure, L-Serin, L-Prolin, L-Leucin oder D-Alanin, wobei in diesen Acylresten vorhandene Aminogruppen am Stickstoff durch tert.-Butyloxycarbonyl, Benzyloxycarbonyl oder Niederalkanoyl substituiert und vorhandene Carboxygruppen mit einem Niederalkanol verestert sein können, T die Gruppe NH, Y unsubstituiertes oder durch Carboxy oder Niederalkoxycarbonyl substituiertes Dimethylen, W Wasserstoff und Z eine 1,2-Dihydroxy-ethyl-, 2-Hydroxy-ethyl- oder Hydroxymethylgruppe, in der mindestens eine der Hydroxygruppen mit einer $C_{10-18}$-Alkansäure oder einer $C_{18}$-Alkensäure verestert ist, oder W und Z je eine Hydroxymethylgruppe, die mit einer $C_{10-18}$-Alkansäure oder einer $C_{18}$-Alkensäure verestert ist, bedeuten.

8. Verfahren nach Anspruch 7, dadurch gekennzeichnet, daß man die Ausgangsverbindungen so wählt, daß man eine Verbindung der Formel I oder ein Salz davon herstellt, worin $R^1$ den Acylrest von L-Tyrosin, L-Lysin, L-Asparaginsäure, L-Asparagin, L-Histidin, L-Glutaminsäure, L-Serin, L-Prolin, L-Leucin oder D-Alanin, wobei in diesen Acylresten vorhandene Aminogruppen am Stickstoff durch tert.-Butyloxycarbonyl, Benzyloxycarbonyl oder Niederalkanoyl substituiert sind, bedeutet.

9. Verfahren nach Anspruch 1, dadurch gekennzeichnet, daß man die Ausgangsverbindungen so wählt, daß man eine Verbindung der Formel I oder ein Salz davon herstellt, worin $R^1$ den Acylrest von L-Valin, L-Serin, L-Threonin, L-Cystein, L-Methionin, L-Tyrosin oder Citronensäure, T die Gruppe NH, Y unsubstituiertes oder durch Carboxy substituiertes Dimethylen, W Wasserstoff und Z 1,2-Dihydroxy-ethyl, worin beide Hydroxygruppen mit einer geradkettigen $C_{16-24}$-Alkan- oder Alkensäure mit gerader Anzahl C-Atome verestert sind, bedeuten.

10. Verfahren nach einem der Ansprüche 1 - 8, dadurch gekennzeichnet, daß man die Ausgangsverbindungen so wählt, daß man eine Verbindung der Formel I oder ein Salz davon herstellt, worin Y durch Carboxy oder Niederalkoxycarbonyl substituiertes Dimethylen bedeutet.

11. Verfahren nach einem der Ansprüche 1, 2 und 4 - 8, dadurch gekennzeichnet, daß man die Ausgangsverbindungen so wählt, daß man eine Verbindung der Formel I oder ein Salz davon herstellt, worin W und Z je eine Hydroxymethylgruppe, die mit einer $C_{18}$-Alkensäure oder mit einer geradkettigen $C_{12}$-$C_{18}$-Alkansäure verestert ist, bedeuten.

12. Verfahren nach einem der Ansprüche 1 - 11, dadurch gekennzeichnet, daß man die Ausgangsverbindungen so wählt, daß man eine Verbindung der Formel I oder ein Salz davon herstellt, worin T für die Gruppe NH steht.

13. Verfahren nach einem der Ansprüche 1 - 9, 11 und 12, dadurch gekennzeichnet, daß man die Ausgangsverbindungen so wählt, daß man eine Verbindung der Formel I oder ein Salz davon herstellt, worin Y unsubstituiertes Dimethylen bedeutet.

14. Verfahren nach einem der Ansprüche 1 - 13, dadurch gekennzeichnet, daß man die Ausgangsverbindungen so wählt, daß man ein pharmazeutisch verwendbares Salz einer Verbindung der Formel I herstellt.

15. Verfahren nach einem der Ansprüche 1, 3, 4 und 10 - 13, dadurch gekennzeichnet, daß man die Ausgangsverbindungen so wählt, daß man eine Verbindung der Formel I oder ein pharmazeutisch verwendbares Salz davon herstellt, worin $R^1$ den Acylrest von L-Tyrosin oder von N-Niederalkoxycarbonyl- oder N-Niederalkanoyl-L-tyrosin bedeutet.

16. Verfahren nach einem der Ansprüche 1, 3, 4 und 10 - 13, dadurch gekennzeichnet, daß man die Ausgangsverbindungen so wählt, daß man eine Verbindung der Formel I oder ein pharmazeutisch verwendbares Salz davon herstellt, worin $R^1$ den Acylrest von L-Leucin oder von N-Niederalkoxycarbonyl- oder N-Niederalkanoyl-L-leucin bedeutet.

17. Verfahren nach einem der Ansprüche 1, 3, 4 und 10 - 13, dadurch gekennzeichnet, daß man die Ausgangsverbindungen so wählt, daß man eine Verbindung der Formel I oder ein pharmazeutisch verwendbares Salz davon herstellt, worin $R^1$ den Acylrest von L-Lysin oder von N-Benzyloxycarbonyl-$N^{\varepsilon}$-niederalkoxycarbonyl-L-lysin bedeutet.

18. Verfahren nach Anspruch 1, dadurch gekennzeichnet, daß man die Ausgangsverbindungen so wählt, daß man L-Tyrosin-2-(1,2-dipalmitoyl-sn-glycero-3-hydroxyphosphoryloxy)-ethylamid oder ein pharmazeutisch verwendbares Salz davon herstellt.

19. Verfahren nach Anspruch 1, dadurch gekennzeichnet, daß man die Ausgangsverbindungen so wählt, daß man 3,4-Dicarboxy-3-hydroxy-buttersäure-2-(1,2-dipalmitoyl-sn-glycero-3-hydroxyphosphoryloxy)-ethylamid oder ein pharmazeutisch verwendbares Salz davon herstellt.

20. Verfahren nach Anspruch 1, dadurch gekennzeichnet, daß man die Ausgangsverbindungen so wählt, daß man eine Verbindung, ausgewählt aus der Gruppe, bestehend aus L-Lysin-2-[1,2-dipalmitoyl-sn-glycero-3-hydroxyphosphoryloxy]-ethylamid, $N^{\alpha}$-Benzyloxycarbonyl-$N^{\varepsilon}$-tert.-butyloxycarbonyl-L-lysin-2-(1,2-dipalmitoyl-sn-glycero-3-hydroxyphosphoryloxy)-ethylamid, N-Boc-L-tyrosin-2-(1,2-dipalmitoyl-sn-glycero-3-hydroxyphosphoryloxy)-ethylamid, N-Benzyloxycarbonyl-L-asparaginsäure-α-benzylester-2-(1,2-dipalmitoyl-sn-glycero-3-hydroxyphosphoryloxy)-ethylamid, L-Asparaginsäure-β-2-(1,2-dipalmitoyl-sn-glycero-3-hydroxyphosphoryloxy)-ethylamid, N-Benzyloxycarbonyl-L-asparagin-α-2-(1,2-dilauroyl-sn-glycero-3-hydroxyphosphoryloxy)-ethylamid, L-Asparagin-α-2-(1,2-dilauroyl-sn-glycero-3-hydroxyphosphoryloxy)-ethylamid, N-Boc-D-alanin-2-(1,2-dipalmitoyl-sn-glycero-3-hydroxyphosphoryloxy)-ethylamid, D-Alanin-2-(1,2-dipalmitoyl-sn-glycero-3-hydroxyphosphoryloxy)-ethylamid, $N^{\alpha}$,$N^{im}$-Di-Boc-L-histidin-2-(1,2-didecanoyl-sn-glycero-3-hydroxyphosphoryloxy)-ethylamid, L-Histidin-2-(1,2-didecanoyl-sn-glycero-3-hydroxyphosphoryloxy)-ethylamid, N-Benzyloxy-carbonyl-L-glutaminsäure-γ-tertiär-butylester-α-2-(1,3-dipalmitoylglycero-2-hydroxyphosphoryloxy)-ethylamid, L-Glutaminsäure-α-2-(1,3-dipalmitoyl-glycero-2-hydroxyphosphoryloxy)-ethylamid, N-Benzyloxycarbonyl-L-glutaminsäure-α-tertiär-butylester-γ-2-(1,3-dilauroylglycero-2-hydroxyphosphoryloxy)-ethylamid, L-Glutaminsäure-γ-2-(1,3-dilauroyl-glycero-2-hydroxyphosphoryloxy)-ethylamid, N-Benzyloxycarbonyl-L-glutaminsäure-γ-methylester-α-2-(1,2-dipalmitoyl-sn-glycero-3-hydroxyphosphoryloxy)-ethylamid, L-Glutaminsäure-γ-methylester-α-2-(1,2-dipalmitoyl-sn-glycero-3-hydroxyphosphoryloxy)-ethylamid, N-Boc-L-tyrosin-2-(1-palmitoyloxypropyl-3-oxyhydroxyphosphoryloxy)-ethylamid, L-Tyrosin-2-(1-palmitoyloxy-propyl-3-oxyhydroxyphosphoryloxy)-ethylamid, N-Boc-L-serin-2-(1,2-dihexadecyl-sn-glycero-3-hydroxyphosphoryloxy)-ethylamid, L-Serin-2-(1,2-dihexadecyl-sn-glycero-3-hydroxyphosphoryloxy)-ethylamid, N-Boc-L-prolin-2-(1,2-didecanoyl-sn-glycero-3-hydroxyphosphoryloxy)-ethylamid, L-Prolin-2-(1,2-didecanoyl-sn-glycero-3-hydroxyphosphoryloxy)-ethylamid, N-Acetyl-L-leucyl-O-(1,3-dilauroyl-glycero-2-hydroxyphosphoryl)-L-serin, N-Boc-L-leucyl-O-(1,2-dioleoyl-sn-glycero-3-hydroxyphosphoryl)-L-serin, L-Leucin-0-(1,2-dioleoyl-sn-glycero-3-hydroxyphosphoryl)-L-serin oder einem pharmazeutisch verwendbaren Salz der obengenannten Verbindungen, herstellt.

**Claims** for the Contracting States: BE, CH, DE, FR, GB, IT, LI, LU, NL and SE

1. Phosphatidyl compounds of the formula I

```
           O   W
           ||  |
R1-T-Y-O-P-O-CH          (I)
           |   |
           OH  Z
```

in which

$R^1$ represents $C_{3-14}$-alkanoyl, benzoyl, the acyl radical of an α-aminocarboxylic acid that is other than glycine, L-alanine and derivatives thereof having a substituted amino group, and of which the α-amino group may be substituted by lower alkanoyl, lower alkoxycarbonyl or by benzyloxycarbonyl, or the acyl radical of a β-aminocarboxylic acid or an α- or β hydroxycarboxylic acid,

T represents a group NH that is unsubstituted or is substituted by lower alkyl, or oxygen,

Y represents dimethylene that is unsubstituted or is substituted by free, etherified or amidated carboxy,

W represents hydrogen, and

Z represents a 1,2-dihydroxyethyl, 2-hydroxyethyl or hydroxymethyl group in which at least one of the hydroxy groups is esterified by an aliphatic $C_{8-30}$-carboxylic acid or is etherified by an aliphatic $C_{8-30}$-alcohol,

or each of W and Z represents a hydroxymethyl group that is esterified by an aliphatic $C_{8-30}$-carboxylic acid or is etherified by an aliphatic $C_{8-30}$-alcohol,

the prefix "lower" used above denoting radicals having up to and including 7 carbon atoms, and their salts.

2. Compounds of the formula I according to claim 1, in which $R^1$ represents the acyl radical of an α-aminocarboxylic acid that is other than glycine, L-alanine and derivatives thereof having a substituted amino group, and of which the α-amino group is substituted by lower alkanoyl, lower alkoxycarbonyl or by benzyloxycarbonyl, and their salts.

3. Compounds of the formula I according to claim 1, or claim 2, in which W represents hydrogen, and Z represents a 1,2-dihydroxyethyl group in which at least one of the hydroxy groups is esterified by an aliphatic $C_{8-30}$-carboxylic acid, and their salts.

4. Compounds of the formula I according to claim 1, in which $R^1$ represents α-amino-lower alkanoyl that is other than glycyl, L-alanyl and derivatives thereof having a substituted amino group and that may be substituted by a further amino, lower alkanoylamino, lower alkoxycarbonylamino or benzyloxycarbonylamino group or by guanidino, carboxy, phenoxycarbonyl, benzyloxycarbonyl, benzhydryloxycarbonyl, lower alkoxycarbonyl, carbamoyl, hydroxy, lower alkoxy, lower alkanoyloxy, lower alkoxycarbonyloxy, mercapto, lower alkylthio, (2-amino-2-carboxyethyl)-dithio, phenyl, 4-hydroxyphenyl, imidazol-4-yl or by indol-3-yl, or pyrrolidine-α-carbonyl that is unsubstituted or is substituted by hydroxy, lower alkoxy, lower alkanoyloxy or by lower alkoxycarbonyloxy, T represents a group NH or oxygen, Y represents dimethylene that is unsubstituted or is substituted by carboxy, lower alkoxycarbonyl or carbamoyl, W represents hydrogen and Z represents 1,2-dihydroxyethyl in which either only the 2-hydroxy group or both hydroxy groups is (are) esterified by a $C_{12-24}$-alkanoic or -alkenoic acid, or each of W and Z represents a hydroxymethyl group esterified by a $C_{12-24}$-alkanoic or -alkenoic acid, and their salts.

5. Compounds of the formula I according to claim 1 or claim 3, in which $R^1$ represents the acyl radical of an alkanoic acid having up to 12 carbon atoms that is substituted by α-amino and that may be substituted by a further amino group, one or two carboxy groups, hydroxy, mercapto, lower alkylthio, phenyl, 4-hydroxyphenyl, carbamoyl, guanidino, 3-indolyl, 4-imidazolyl or (2-amino-2-carboxyethyl)-dithio, with the exception of the acyl radicals glycyl, L-alanyl and the derivatives thereof having a substituted amino group, or represents a benzoyl or prolyl radical, and their salts.

6. Compounds of the formula I according to claim 1 or claim 3, in which $R^1$ represents the acyl radical of one of those 20 amino acids that are regularly present in proteins, with the exception of glycine and L-alanine, or the acyl radical of lactic acid or citric acid, and their salts.

7. Compounds of the formula I according to claim 1, in which $R^1$ represents benzoyl or the acyl radical of L-tyrosine, L-lysine, L-aspartic acid, L-asparagine, L-histidine, L-glutamic acid, L-serine, L-proline, L-leucine or D-alanine, it being possible for amino groups present in these acyl radicals to be substituted at the nitrogen atom by tert.-butoxycarbonyl, benzyloxycarbonyl or lower alkanoyl and for carboxy groups present to be esterified by a lower alkanol, T represents a group NH, Y represents dimethylene that is unsubstituted or is substituted by carboxy or lower alkoxycarbonyl, W represents hydrogen and Z represents a 1,2-dihydroxyethyl, 2-hydroxyethyl or hydroxymethyl group in which at least one of the hydroxy groups is esterified by a $C_{10-18}$-alkanoic acid or a $C_{18}$-alkenoic acid, or each of W and Z represents a hydroxymethyl group that is esterified by a $C_{10-18}$-alkanoic acid or a $C_{18}$-alkenoic acid, and their salts.

8. Compounds of the formula I according to claim 7 in which $R^1$ represents the acyl radical of L-tyrosine, L-lysine, L-aspartic acid, L-asparagine, L-histidine, L-glutamic acid, L-serine, L-proline, L-leucine or D-alanine, amino groups present in those radicals being substituted at the nitrogen atom by tert.-butoxycarbonyl, benzyloxycarbonyl or lower alkanoyl, and their salts.

9. Compounds of the formula I according to claim 1 in which $R^1$ represents the acyl radical of L-valine, L-serine, L-threonine, L-cysteine, L-methionine, L-tyrosine or citric acid, T represents a group NH, Y represents unsubstituted or carboxy-substituted dimethylene, W represents hydrogen and Z represents 1,2-dihydroxyethyl in which each hydroxy group is esterified by a straight-chained $C_{16-24}$-alkanoic or -alkenoic acid having an even number of carbon atoms, and their salts.

10. Compounds of the formula I according to any one of claims 1 to 8 in which Y represents dimethylene substituted by carboxy or lower alkoxycarbonyl, and their salts.

11. Compounds of the formula I according to any one of claims 1, 2 and 4 to 8 in which each of W and Z represents a hydroxymethyl group that is esterified by a $C_{18}$-alkenoic acid or by a straight-chained $C_{12}$-$C_{18}$-alkanoic acid, and their salts.

12. Compounds of the formula I according to any one of claims 1 to 11 in which T represents a group NH, and their salts.

13. Compounds of the formula I according to any one of claims 1 to 9, 11 and 12 in which Y represents unsubstituted dimethylene, and their salts.

14. Compounds of the formula I according to any one of claims 1 to 13 in the form of their pharmaceutically acceptable salts.

15. Compounds of the formula I according to any one of claims 1, 3, 4 and 10 to 13 in which $R^1$ represents the acyl radical of L-tyrosine or of N-lower alkoxycarbonyl- or N-lower alkanoyl-L-tyrosine, and their pharmaceutically acceptable salts.

16. Compounds of the formula I according to any one of claims 1, 3, 4 and 10 to 13 in which $R^1$ represents the acyl radical of L-leucine or of N-lower alkoxycarbonyl- or N-lower alkanoyl-L-leucine, and their pharmaceutically acceptable salts.

17. Compounds of the formula I according to any one of claims 1, 3, 4 and 10 to 13 in which $R^1$ represents the acyl radical of L-lysine or of $N^{\alpha}$-benzyloxycarbonyl-$N^{\varepsilon}$-lower alkoxycarbonyl-L-lysine, and their pharmaceutically acceptable salts.

18. L-tyrosine-2-(1,2-dipalmitoyl-sn-glycero-3-hydroxyphosphoryloxy)-ethylamide or a pharmaceutically acceptable salt thereof according to claim 1.

19. 3,4-dicarboxy-3-hydroxybutyric acid 2-(1,2-dipalmitoyl-sn-glycero-3-hydroxyphosphoryloxy)-ethylamide or a pharmaceutically acceptable salt thereof according to claim 1.

20. A compound according to claim 1 selected from the group consisting of L-lysine-2-[1,2-dipalmitoyl-sn-glycero-3-hydroxyphosphoryloxy]-ethylamide, $N^{\alpha}$-benzyloxycarbonyl-$N^{\varepsilon}$-tert.-butoxycarbonyl-L-lysine-2-(1,2-dipalmitoyl-sn-glycero-3-hydroxyphosphoryloxy)-ethylamide, N-boc-L-tyrosine-2-(1,2-dipalmitoyl-sn-glycero-3-hydroxyphosphoryloxy)-ethylamide, N-benzyloxycarbonyl-L-aspartic acid α-benzyl ester-2-(1,2-dipalmitoyl-sn-glycero-3-hydroxyphosphoryloxy)-ethylamide, L-aspartic acid β-2-(1,2-dipalmitoyl-sn-glycero-3-hydroxyphosphoryloxy)-ethylamide, N-benzyloxycarbonyl-L-asparagine-α-2-(1,2-dilauroyl-sn-glycero-3-hydroxyphosphoryloxy)-ethylamide, L-asparagine-α-2-(1,2-dilauroyl-sn-glycero-3-hydroxyphosphoryloxy)-ethylamide, N-boc-D-alanine-2-(1,2-dipalmitoyl-sn-glycero-3-hydroxyphosphoryloxy)-ethylamide, D-alanine-2-(1,2-dipalmitoyl-sn-glycero-3-hydroxyphosphoryloxy)-ethylamide, $N^{\alpha}$,$N^{im}$-di-boc-L-histidine-2-(1,2-didecanoyl-sn-glycero-3-hydroxyphosphoryloxy)ethylamide, L-histidine-2-(1,2-didecanoyl-sn-glycero-3-hydroxyphosphoryloxy)-ethylamide, N-benzyloxycarbonyl-L-glutamic acid γ-tertiary-butyl ester-α-2-(1,3-dipalmitoylglycero-2-hydroxyphosphoryloxy)-ethylamide, L-glutamic acid α-2-(1,3-dipalmitoylglycero-2-hydroxyphosphoryloxy)-ethylamide, N-benzyloxycarbonyl-L-glutamic acid α-tertiary-butyl ester-γ-2-(1,3-dilauroylglycero-2-hydroxyphosphoryloxy)-ethylamide, L-glutamic acid γ-2-(1,3-dilauroylglycero-2-hydroxyphosphoryloxy)-ethylamide, N-benzyloxycarbonyl-L-glutamic acid γ-methyl ester-α-2-(1,2-dipalmitoyl-sn-glycero-3-hydroxyphosphoryloxy)-ethylamide, L-glutamic acid γ-methyl ester-α-2-(1,2-dipalmitoyl-sn-glycero-3-hydroxyphosphoryloxy)-ethylamide, N-boc-L-tyrosine-2-(1-palmitoyloxypropyl-3-oxyhydroxyphosphoryloxy)-ethylamide, L-tyrosine-2-(1-palmitoyloxypropyl-3-oxy-hydroxyphosphoryloxy)-ethylamide, N-boc-L-serine-2-(1,2-dihexadecyl-sn-glycero-3-hydroxyphosphoryloxy)-ethylamide, L-serine-2-(1,2-dihexadecyl-sn-glycero-3-hydroxyphosphoryloxy)-ethylamide, N-boc-L-proline-2-(1,2-didecanoyl-sn-glycero-3-hydroxyphosphoryloxy)-ethylamide, L-proline-2-(1,2-didecanoyl-sn-glycero-3-hydroxyphosphoryloxy)-ethylamide, N-acetyl-L-leucyl-O-(1,3-dilauroylglycero-2-hydroxyphosphoryl)-L-serine, N-boc-L-leucyl-O-(1,2-dioleoyl-sn-glycero-3-hydroxyphosphoryl)-L-serine, L-leucine-O-(1,2-dioleoyl-sn-glycero-3-hydroxyphosphoryl)-L-serine or a pharmaceutically acceptable salt of the above-mentioned compounds.

21. Phosphatidyl compounds of the formula Ia

```
            O  W
            ‖  |
R1a-T-Y-O-P-O-CH                    (Ia)
            |  |
            OH Z
```

in which $R^{1a}$ represents $C_{2-22}$-alkanoyl that may be substituted by carboxy, propenoyl, 2-methylpropenoyl, benzoyl, the acyl radical of an α-aminocarboxylic acid of which the α-amino group may be substituted by lower alkyl, lower alkanoyl, lower alkoxycarbonyl or benzyloxycarbonyl, or the acyl radical of a β-aminocarboxylic acid or an α- or β-hydroxycarboxylic acid, T represents a group NH that is unsubstituted or is substituted by lower alkyl, or oxygen, Y represents dimethylene that is unsubstituted or is substituted by free, etherified or amidated carboxy, W represents hydrogen, and 2 represents a 1,2-dihydroxyethyl, 2-hydroxyethyl or hydroxymethyl group in which at least one of the hydroxy groups is esterified by an aliphatic $C_{8-30}$-carboxylic acid or is etherified by an aliphatic $C_{8-30}$-alcohol, or each of W and Z represents a hydroxymethyl group that is esterified by an aliphatic $C_{8-30}$-carboxylic acid or is etherified by an aliphatic $C_{8-30}$-alcohol, the prefix "lower" used above denoting radicals having up to and including 7 carbon atoms, and their pharmaceutically acceptable salts, for use in a method for the therapeutic treatment of the human or animal body.

22. L-alanine-2-(1,2-dipalmitoyl-sn-glycero-3-hydroxyphosphoryloxy)-ethylamide or a pharmaceutically acceptable salt thereof according to claim 21 for use in a method for the therapeutic treatment of the human or animal body.

23. A compound of the formula I according to any one of claims 1 to 20 or a pharmaceutically acceptable salt thereof for use in a method for the therapeutic treatment of the human or animal body.

24. Pharmaceutical preparation containing as active ingredient an amount, effective for the prophylaxis or treatment of a viral infection, of a compound of the formula I according to any one of claims 1 to 20 or a pharmaceutically acceptable salt thereof together with a significant amount of a pharmaceutical carrier.

25. Use of a compound of the formula Ia

```
           O   W
           ||  |
R1a-T-Y-O-P-O-CH                                        (Ia)
           |   |
           OH  Z
```

in which $R^{1a}$ represents $C_{2-22}$-alkanoyl that may be substituted by carboxy, $C_{3-18}$-alkenoyl, benzoyl, the acyl radical of an α-aminocarboxylic acid of which the α-amino group may be substituted by lower alkyl, lower alkanoyl, lower alkoxycarbonyl or benzyloxycarbonyl, or the acyl radical of a β-aminocarboxylic acid or an α- or β-hydroxycarboxylic acid, T represents a group NH that is unsubstituted or is substituted by lower alkyl, or oxygen, Y represents dimethylene that is unsubstituted or is substituted by free, etherified or amidated carboxy, W represents hydrogen, and Z represents a 1,2-dihydroxyethyl, 2-hydroxyethyl or hydroxymethyl group in which at least one of the hydroxy groups is esterified by an aliphatic $C_{8-30}$-carboxylic acid or is etherified by an aliphatic $C_{8-30}$-alcohol, or each of W and Z represents a hydroxymethyl group that is esterified by an aliphatic $C_{8-30}$-carboxylic acid or is etherified by an aliphatic $C_{8-30}$-alcohol, the prefix "lower" used above denoting radicals having up to and including 7 carbon atoms, or a pharmaceutically acceptable salt thereof, for the manufacture of a medicament for use in the prophylaxis or treatment of a viral infection.

26. Use of a compound of the formula I according to any one of claims 1 to 20 or of a pharmaceutically acceptable salt thereof for the manufacture of a medicament for use in the prophylaxis or treatment of a viral infection.

27. Process for the manufacture of a compound of the formula I

```
          O   W
          ||  |
R1-T-Y-O-P-O-CH                                         (I)
          |   |
          OH  Z
```

in which $R^1$ represents $C_{3-14}$-alkanoyl, benzoyl, the acyl radical of an α-aminocarboxylic acid that is other than glycine, L-alanine and derivatives thereof having a substituted amino group, and of which the α-amino group may be substituted by lower alkanoyl, lower alkoxycarbonyl or by benzyloxycarbonyl, or the acyl radical of a β-aminocarboxylic acid or an α- or β-hydroxycarboxylic acid, T represents a group NH that is unsubstituted or is substituted by lower alkyl, or oxygen, Y represents dimethylene that is unsubstituted or is substituted by free, etherified or amidated carboxy, W represents hydrogen, and Z represents a 1,2-dihydroxyethyl, 2-hydroxyethyl or hydroxymethyl group in which at least one of the hydroxy groups is esterified by an aliphatic $C_{8-30}$-carboxylic acid or is etherified by an aliphatic $C_{8-30}$-alcohol, or each of W and Z represents a hydroxymethyl group that is esterified by an aliphatic $C_{8-30}$-carboxylic acid or is etherified by an aliphatic $C_{8-30}$-alcohol, the prefix "lower" used above denoting radicals having up to and including 7 carbon atoms, or a salt thereof, characterised in that:

a) a compound of the formula II

```
         O   W
         ||  |
H-T-Y-O-P-O-CH                                          (II)
         |   |
         OH  Z
```

in which the substituents have the meanings given above, functional groups present in a compound of the formula II, with the exception of the group that participates in the reaction, being, if necessary, in protected form, or a reactive derivative thereof, is reacted with a compound of the formula III

$R^1$-OH (III)

in which $R^1$ has the meaning given above, functional groups present in a compound of the formula III, with the exception of the group that participates in the reaction, being, if necessary, in protected form, or with a reactive carboxylic acid derivative thereof, and any protecting groups present are removed, or

b) a compound of the formula IV

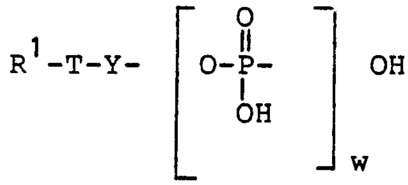

(IV)

in which $\underline{w}$ represents 0 or 1 and $R^1$, T and Y have the meanings given above, functional groups present in a compound of the formula IV, with the exception of the group that participates in the reaction, being, if necessary, in protected form, or a reactive derivative thereof, is reacted with a compound of the formula V

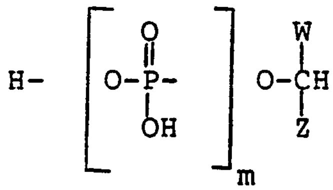

(V)

in which W and Z have the meanings given above, functional groups present in a compound of the formula V, with the exception of the group that participates in the reaction, being, if necessary, in protected form, and, if $\underline{w}$ represents 0 in the reactant of the formula IV, $\underline{m}$ represents 1 or, if $\underline{w}$ represents 1, $\underline{m}$ represents 0, or with a reactive derivative of a compound of the formula V, and any protecting groups present are removed, or

c) a compound of the formula VI

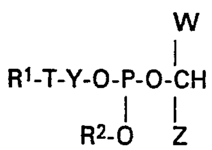

(VI)

in which $R^2$ represents hydrogen or a protecting group and the other substituents have the meanings given above, functional groups present in a compound of the formula VI, with the exception of the group that participates in the reaction, being, if necessary, in protected form, or a tautomer of a compound of the formula VI, is oxidised with an oxidising agent, and any protecting groups present are removed, or

d) a compound of the formula VII

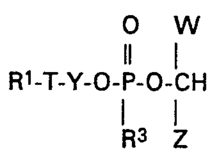

(VII)

in which $R^3$ represents halogen and the other substituents have the meanings given above, functional groups present in a compound of the formula VII, with the exception of the group that participates in the reaction, being, if necessary, in protected form, is hydrolysed, and any protecting groups present are removed, or

e) a compound of the formula III

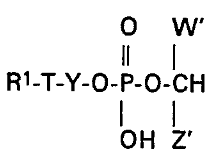

(VIII)

in which W' represents hydrogen and Z' represents 1,2-dihydroxyethyl, 2-hydroxyethyl, hydroxymethyl or 1,2-dihydroxyethyl in which one of the two hydroxy groups is esterified by an aliphatic $C_{8-30}$-carboxylic acid or is etherified by an aliphatic $C_{8-30}$-alcohol, or one of the radicals W' and Z' represents hydroxymethyl and the other radical W' or Z' represents free hydroxymethyl or hydroxymethyl that is esterified by an aliphatic $C_{8-30}$-carboxylic acid or is etherified by an aliphatic $C_{8-30}$-alcohol, and $R^1$, T and Y have the meanings given above,

functional groups present in a compound of the formula VIII, with the exception of the group that participates in the reaction, being, if necessary, in protected form, or a reactive derivative thereof, is esterified by an aliphatic $C_{8-30}$-carboxylic acid or a reactive derivative thereof, or is etherified by an aliphatic $C_{8-30}$-alcohol or a reactive derivative thereof, and any protecting groups present are removed, or

f) a compound of the formula IX

```
         O   W
         ||  |
X-Y-O-P-O-CH
         |   |
         OH  Z
```

(IX)

in which X represents a nucleophilic leaving group and the other substituents have the meanings given above, functional groups present in a compound of the formula IX, with the exception of the group that participates in the reaction, being, if necessary, in protected form, is reacted with a compound of the formula X

$R^1$-T-H (X)

in which the substituents have the meanings given above, functional groups present in a compound of the formula X, with the exception of the group that participates in the reaction, being, if necessary, in protected form, or with a reactive derivative thereof, and any protecting groups present are removed, or

g) in a compound of the formula I in which the substituents have the meanings given above, it being necessary for at least one functional group in a compound of the formula 1 to be protected by a readily removable protecting group, the protecting group(s) is (are) removed, or

h) for the manufacture of a compound of the formula I in which W represents hydrogen and Z represents a 1,2-dihydroxyethyl group in which the 2-hydroxy group is esterified by an aliphatic $C_{8-30}$-carboxylic acid, a compound of the formula I in which W represents hydrogen and Z represents a 1,2-dihydroxyethyl group in which both hydroxy groups are esterified by an aliphatic $C_{8-30}$-carboxylic acid, is reacted with an enzyme that is suitable for the regioselective removal of the radical that esterifies the 1-hydroxy group, and, when one of the above-mentioned process variants a-h) has been carried out, in order to manufacture a salt if necessary a compound of the formula I is converted into a salt, or in order to convert a compound of the formula I or a salt thereof into a different compound of the formula I or into a salt thereof, an amino or hydroxy group contained in the radical $R^1$ is acylated or a carboxy group contained in the radical $R^1$ or Y is esterified or amidated.

**Claims** for the Contracting State: AT

1. Process for the manufacture of a compound of the formula I

```
            O   W
            ||  |
R1-T-Y-O-P-O-CH
            |   |
            OH  Z
```

(I)

in which

$R^1$ represents $C_{3-14}$-alkanoyl, benzoyl, the acyl radical of an α-aminocarboxylic acid that is other than glycine, L-alanine and derivatives thereof having a substituted amino group, and of which the α-amino group may be substituted by lower alkanoyl, lower alkoxycarbonyl or by benzyloxycarbonyl, or the acyl radical of a β-aminocarboxylic acid or an α- or β-hydroxycarboxylic acid,

T represents a group NH that is unsubstituted or is substituted by lower alkyl, or oxygen,

Y represents dimethylene that is unsubstituted or is substituted by free, etherified or amidated carboxy,

W represents hydrogen, and

Z represents a 1,2-dihydroxyethyl, 2-hydroxyethyl or hydroxymethyl group in which at least one of the hydroxy groups is esterified by an aliphatic $C_{8-30}$-carboxylic acid or is etherified by an aliphatic $C_{8-30}$-alcohol,

or each of W and Z represents a hydroxymethyl group that is esterified by an aliphatic $C_{8-30}$-carboxylic acid or is etherified by an aliphatic $C_{8-30}$-alcohol,

the prefix "lower" used above denoting radicals having up to and including 7 carbon atoms, or a salt thereof, characterised in that:

a) a compound of the formula II

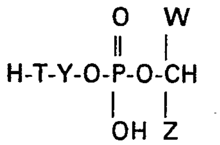

(II)

in which the substituents have the meanings given above, functional groups present in a compound of the formula II, with the exception of the group that participates in the reaction, being, if necessary, in protected form, or a reactive derivative thereof, is reacted with a compound of the formula III

$R^1$-OH (III)

in which $R^1$ has the meaning given above, functional groups present in a compound of the formula III, with the exception of the group that participates in the reaction, being, if necessary, in protected form, or with a reactive carboxylic acid derivative thereof, and any protecting groups present are removed, or

b) a compound of the formula IV

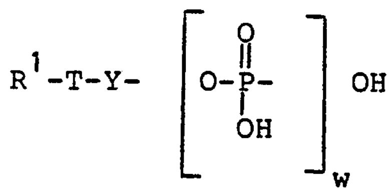

(IV)

in which w represents 0 or 1 and $R^1$, T and Y have the meanings given above, functional groups present in a compound of the formula IV, with the exception of the group that participates in the reaction, being, if necessary, in protected form, or a reactive derivative thereof, is reacted with a compound of the formula V

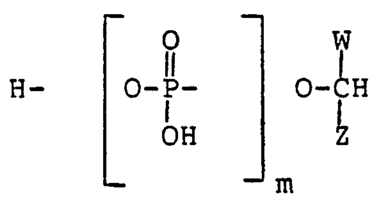

(V)

in which W and Z have the meanings given above, functional groups present in a compound of the formula V, with the exception of the group that participates in the reaction, being, if necessary, in protected form, and, if w represents 0 in the reactant of the formula IV, m represents 1 or, if w represents 1, m represents 0, or with a reactive derivative of a compound of the formula V, and any protecting groups present are removed, or

c) a compound of the formula VI

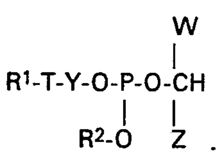

(VI)

in which $R^2$ represents hydrogen or a protecting group and the other substituents have the meanings given above, functional groups present in a compound of the formula VI, with the exception of the group that participates in the reaction, being, if necessary, in protected form, or a tautomer of a compound of the formula VI, is oxidised with an oxidising agent, and any protecting groups present are removed, or

d) a compound of the formula VII

```
           O   W
           ||  |
R1-T-Y-O-P-O-CH                (VII)
           |   |
           R3  Z
```

in which $R^3$ represents halogen and the other substituents have the meanings given above, functional groups present in a compound of the formula VII, with the exception of the group that participates in the reaction, being, if necessary, in protected form, is hydrolysed, and any protecting groups present are removed, or

e) a compound of the formula VIII

```
           O   W'
           ||  |
R1-T-Y-O-P-O-CH                (VIII)
           |   |
           OH  Z'
```

in which W' represents hydrogen and Z' represents 1,2-dihydroxyethyl, 2-hydroxyethyl, hydroxymethyl or 1,2-dihydroxyethyl in which one of the two hydroxy groups is esterified by an aliphatic $C_{8-30}$-carboxylic acid or is etherified by an aliphatic $C_{8-30}$-alcohol, or one of the radicals W' and Z' represents hydroxymethyl and the other radical W' or Z' represents free hydroxymethyl or hydroxymethyl that is esterified by an aliphatic $C_{8-30}$-carboxylic acid or is etherified by an aliphatic $C_{8-30}$-alcohol, and $R^1$, T and Y have the meanings given above, functional groups present in a compound of the formula VIII, with the exception of the group that participates in the reaction, being, if necessary, in protected form, or a reactive derivative thereof, is esterified by an aliphatic $C_{8-30}$-carboxylic acid or a reactive derivative thereof, or is etherified by an aliphatic $C_{8-30}$-alcohol or a reactive derivative thereof, and any protecting groups present are removed, or

f) a compound of the formula IX

```
         O   W
         ||  |
X-Y-O-P-O-CH                   (IX)
         |   |
         OH  Z
```

in which X represents a nucleophilic leaving group and the other substituents have the meanings given above, functional groups present in a compound of the formula IX, with the exception of the group that participates in the reaction, being, if necessary, in protected form, is reacted with a compound of the formula X

$R^1$-T-H (X)

in which the substituents have the meanings given above, functional groups present in a compound of the formula X, with the exception of the group that participates in the reaction, being, if necessary, in protected form, or with a reactive derivative thereof, and any protecting groups present are removed, or

g) in a compound of the formula I in which the substituents have the meanings given above, it being necessary for at least one functional group in a compound of the formula I to be protected by a readily removable protecting group, the protecting group(s) is (are) removed, or

h) for the manufacture of a compound of the formula I in which W represents hydrogen and Z represents a 1,2-dihydroxyethyl group in which the 2-hydroxy group is esterified by an aliphatic $C_{8-30}$-carboxylic acid, a compound of the formula I in which W represents hydrogen and Z represents a 1,2-dihydroxyethyl group in which both hydroxy groups are esterified by an aliphatic $C_{8-30}$-carboxylic acid, is reacted with an enzyme that is suitable for the regioselective removal of the radical that esterifies the 1-hydroxy group,

and, when one of the above-mentioned process variants a-h) has been carried out, in order to manufacture a salt if necessary a compound of the formula I is converted into a salt, or in order to convert a compound of the formula I or a salt thereof into a different compound of the formula I or into a salt thereof, an amino or hydroxy group contained in the radical $R^1$ is acylated or a carboxy group contained in the radical $R^1$ or Y is esterified or amidated.

2. Process according to claim 1, characterised in that the starting compounds are so selected that a compound of the formula I in which $R^1$ represents the acyl radical of an α-aminocarboxylic acid that is other than glycine, L-alanine and derivatives thereof having a substituted amino group, and of which the α-amino group is substituted by lower alkanoyl, lower alkoxycarbonyl or by benzyloxycarbonyl, or a salt thereof, is prepared.

3. Process according to claim 1 or claim 2, characterised in that the starting compounds are so selected that a compound of the formula I in which W represents hydrogen and Z represents a 1,2-dihydroxyethyl group in which at least one of the hydroxy groups is esterified by an aliphatic $C_{8-30}$-carboxylic acid, or a salt thereof, is prepared.

4. Process according to claim 1, characterised in that the starting compounds are so selected that a compound of the formula I in which $R^1$ represents α-amino-lower alkanoyl that is other than glycyl, L-alanyl and derivatives thereof having a substituted amino group and that may be substituted by a further amino, lower alkanoylamino, lower alkoxycarbonylamino or benzyloxycarbonylamino group or by guanidino, carboxy, phenoxycarbonyl, benzyloxycarbonyl, benzhydryloxycarbonyl, lower alkoxycarbonyl, carbamoyl, hydroxy, lower alkoxy, lower alkanoyloxy, lower alkoxycarbonyloxy, mercapto, lower alkylthio, (2-amino-2-carboxyethyl)-dithio, phenyl, 4-hydroxyphenyl, imidazol-4-yl or by indol-3-yl, or pyrrolidine-α-carbonyl that is unsubstituted or is substituted by hydroxy, lower alkoxy, lower alkanoyloxy or by lower alkoxycarbonyloxy, T represents a group NH or oxygen, Y represents dimethylene that is unsubstituted or is substituted by carboxy, lower alkoxycarbonyl or carbamoyl, W represents hydrogen and Z represents 1,2-dihydroxyethyl in which either only the 2-hydroxy group or both hydroxy groups is (are) esterified by a $C_{12-24}$-alkanoic or -alkenoic acid, or each of W and Z represents a hydroxymethyl group esterified by a $C_{12-24}$-alkanoic or -alkenoic acid, or a salt thereof, is prepared.

5. Process according to claim 1 or claim 3, characterised in that the starting compounds are so selected that a compound of the formula I in which $R_1$ represents the acyl radical of an alkanoic acid having up to 12 carbon atoms that is substituted by α-amino and may be substituted by a further amino group, one or two carboxy groups, hydroxy, mercapto, lower alkylthio, phenyl, 4-hydroxyphenyl, carbamoyl, guanidino, 3-indolyl, 4-imidazolyl or (2-amino-2-carboxyethyl)-dithio, with the exception of the acyl radicals glycyl, L-alanyl and the derivatives thereof having a substituted amino group, or represents a benzoyl or prolyl radical, or a salt thereof, is prepared.

6. Process according to claim 1 or claim 3, characterised in that the starting compounds are so selected that a compound of the formula I in which $R^1$ represents the acyl radical of one of those 20 amino acids that are regularly present in proteins, with the exception of glycine and L-alanine, or the acyl radical of lactic acid or citric acid, or a salt thereof, is prepared.

7. Process according to claim 1, characterised in that the starting compounds are so selected that a compound of the formula I in which $R^1$ represents benzoyl or the acyl radical of L-tyrosine, L-lysine, L-aspartic acid, L-asparagine, L-histidine, L-glutamic acid, L-serine, L-proline, L-leucine or D-alanine, it being possible for amino groups present in these acyl radicals to be substituted at the nitrogen atom by tert.-butoxycarbonyl, benzyloxycarbonyl or lower alkanoyl and for carboxy groups present to be esterified by a lower alkanol, T represents a group NH, Y represents dimethylene that is unsubstituted or is substituted by carboxy or lower alkoxycarbonyl, W represents hydrogen and Z represents a 1,2-dihydroxyethyl, 2-hydroxyethyl or hydroxymethyl group in which at least one of the hydroxy groups is esterified by a $C_{10-18}$-alkanoic acid or a $C_{18}$-alkenoic acid, or each of W and Z represents a hydroxymethyl group that is esterified by a $C_{10-18}$-alkanoic acid or a $C_{18}$-alkenoic acid, or a salt thereof, is prepared.

8. Process according to claim 7, characterised in that the starting compounds are so selected that a compound of the formula I in which $R^1$ represents the acyl radical of L-tyrosine, L-lysine, L-aspartic acid, L-asparagine, L-histidine, L-glutamic acid, L-serine, L-proline, L-leucine or D-alanine, amino groups present in those acyl radicals being substituted at the nitrogen atom by tert.-butoxycarbonyl, benzyloxycarbonyl or by lower alkanoyl, or a salt thereof, is prepared.

9. Process according to claim 1, characterised in that the starting compounds are so selected that a compound of the formula I in which $R^1$ represents the acyl radical of L-valine, L-serine, L-threonine, L-cysteine, L-methionine, L-tyrosine or citric acid, T represents a group NH, Y represents unsubstituted or carboxy-substituted dimethylene, W represents hydrogen and Z represents 1,2-dihydroxyethyl in which each hydroxy group is esterified by a straight-chained $C_{16-24}$-alkanoic or alkenoic acid having an even number of carbon atoms, or a salt thereof, is prepared.

10. Process according to any one of claims 1 to 8, characterised in that the starting compounds are so selected that a compound of the formula I in which Y represents dimethylene substituted by carboxy or lower alkoxycarbonyl, or a salt thereof, is prepared.

11. Process according to any one of claims 1, 2 and 4 to 8, characterised in that the starting compounds are so selected that a compound of the formula I in which each of W and Z represents a hydroxymethyl group that is esterified by a $C_{18}$-alkenoic acid or by a straight-chained $C_{12}$-$C_{18}$-alkanoic acid, or a salt thereof, is prepared.

12. Process according to any one of claims 1 to 11, characterised in that the starting compounds are so selected that a compound of the formula I in which T represents a group NH, or a salt thereof, is prepared.

13. Process according to any one of claims 1 to 9, 11 and 12, characterised in that the starting compounds are so selected that a compound of the formula I in which Y represents unsubstituted dimethylene, or a salt thereof, is prepared.

14. Process according to any one of claims 1 to 13, characterised in that the starting compounds are so selected that a pharmaceutically acceptable salt of a compound of the formula I is prepared.

15. Process according to any one of claims 1, 3, 4 and 10 to 13, characterised in that the starting compounds are so selected that a compound of the formula I in which $R^1$ represents the acyl radical of L-tyrosine or of N-lower alkoxycarbonyl- or N-lower alkanoyl-L-tyrosine, or a pharmaceutically acceptable salt thereof, is prepared.

16. Process according to any one of claims 1, 3, 4 and 10 to 13, characterised in that the starting compounds are so selected that a compound of the formula I in which $R^1$ represents the acyl radical of L-leucine or of N-lower alkoxycarbonyl- or N-lower alkanoyl-L-leucine, or a pharmaceutically acceptable salt thereof, is prepared.

17. Process according to any one of claims 1, 3, 4 and 10 to 13, characterised in that the starting compounds are so selected that a compound of the formula I in which $R^1$ represents the acyl radical of L-lysine or of $N^\alpha$-benzyloxycarbonyl-$N^\varepsilon$-lower alkoxycarbonyl-L-lysine, or a pharmaceutically acceptable salt thereof, is prepared.

18. Process according to claim 1, characterised in that the starting compounds are so selected that L-tyrosine-2-(1,2-dipalmitoyl-sn-glycero-3-hydroxyphosphoryloxy)-ethylamide or a pharmaceutically acceptable salt thereof is prepared.

19. Process according to claim 1, characterised in that the starting compounds are so selected that 3,4-dicarboxy-3-hydroxybutyric acid 2-(1,2-dipalmitoyl-sn-glycero-3-hydroxyphosphoryloxy)-ethylamide or a pharmaceutically acceptable salt thereof is prepared.

20. Process according to claim 1, characterised in that the starting compounds are so selected that a compound selected from the group consisting of L-lysine-2-(1,2-dipalmitoyl-sn-glycero-3-hydroxyphosphoryloxy]-ethylamide, $N^\alpha$-benzyloxycarbonyl-$N^\varepsilon$-tert.-butoxycarbonyl-L-lysine-2-(1,2-dipalmitoyl-sn-glycero-3-hydroxyphosphoryloxy)-ethylamide, N-boc-L-tyrosine-2-(1,2-dipalmitoyl-sn-glycero-3-hydroxyphosphoryloxy)-ethylamide, N-benzyloxycarbonyl-L-aspartic acid α-benzyl ester-2-(1,2-dipalmitoyl-sn-glycero-3-hydroxyphosphoryloxy)-ethylamide, L-aspartic acid β-2-(1,2-dipalmitoyl-sn-glycero-3-hydroxyphosphoryloxy)-ethylamide, N-benzyloxycarbonyl-L-asparagine-α-2-(1,2-dilauroyl-sn-glycero-3-hydroxyphosphoryloxy)-ethylamide, L-asparagine-α-2-(1,2-dilauroyl-sn-glycero-3-hydroxyphosphoryloxy)-ethylamide, N-boc-D-alanine-2-(1,2-dipalmitoyl-sn-glycero-3-hydroxyphosphoryloxy)-ethylamide, D-alanine-2-(1,2-dipalmitoyl-sn-glycero-3-hydroxyphosphoryloxy)-ethylamide, $N^\alpha$,$N^{im}$-di-boc-L-histidine-2-(1,2-didecanoyl-sn-glycero-3-hydroxyphosphoryloxy)-ethylamide, L-histidine-2-(1,2-didecanoyl-sn-glycero-3-hydroxyphosphoryloxy)-ethylamide, N-benzyloxycarbonyl-L-glutamic acid γ-tertiary-butyl ester-α-2-(1,3-dipalmitoylglycero-2-hydroxyphosphoryloxy)-ethylamide, L-glutamic acid α-2-ethylamide, D-alanine-2-(1,2-dipalmitoyl-sn-glycero-3-hydroxyphosphoryloxy)-ethylamide, $N^\alpha$,$N^{im}$-di-boc-L-histidine-2-(1,2-didecanoyl-sn-glycero-3-hydroxy-phosphoryloxy)-ethylamide, L-histidine-2-(1,2-didecamoyl-sn-glycero-3-hydroxyphosphoryloxy)-ethylamide, N-benzyloxycarbonyl-L-glutamic acid γtertiary-butyl ester-α-2-(1,3-dipalmitoylglycero-2-hydroxyphosphoryloxy)-ethylamide, L-glutamic acid α-2-(1,3-dipalmitoylglycero-2-hydroxyphosphoryloxy)-ethylamide, N-benzyloxycarbonyl-L-glutamic acid α-tertiary-butyl ester-γ-2-(1,3-dilauroylglycero-2-hydroxyphosphoryloxy)-ethylamide, L-glutamic acid γ-2-(1,3-dilauroylglycero-2-hydroxyphosphoryloxy)-ethylamide, N-benzyloxycarbonyl-L-glutamic acid γ-methyl ester-α-2-(1,2-dipalmitoyl-sn-glycero-3-hydroxyphosphoryloxy)-ethylamide, L-glutamic acid γ-methyl ester-α-2-(1,2-dipalmitoyl-sn-glycero-3-hydroxyphosphoryloxy)-ethylamide, N-boc-L-tyrosine-2-(1-palmitoyloxypropyl-3-oxy-hydroxyphosphoryloxy)-ethylamide, L-tyrosime-2-(1-palmitoyloxypropyl-3-oxy-hydroxyphosphoryloxy)-ethylamide, N-boc-L-serine-2-(1,2-dihexadecyl-sn-glycero-3-hydroxyphosphoryloxy)-ethylamide, L-serine-2-(1,2-dihexadecyl-sn-glycero-3-hydroxyphosphoryloxy)-ethylamide, N-boc-L-proline-2-(1,2-didecanoyl-sn-glycero-3-hydroxyphosphoryloxy)-ethylamide, L-proline-2-(1,2-didecanoyl-sn-glycero-3-hydroxyphosphoryloxy)-ethylamide, N-acetyl-L-leucyl-0-(1,3-dilauroylglycero-2-hydroxyphosphoryl)-L-serine, N-boc-L-leucyl-O-(1,2-dioleoyl-sn-glycero-3-hydroxyphosphoryl)-L-serine, L-leucine-O-(1,2-dioleoyl-sn-glycero-3-hydroxyphosphoryl)-L-serine, or a pharmaceutically acceptable salt of the above-mentioned compounds, is prepared.

**Revendications** pour les Etats Contractants: BE, CH, DE, FR, GB, IT, LI, LU, NL, SE

1. Dérivés phosphatidyliques de formule I

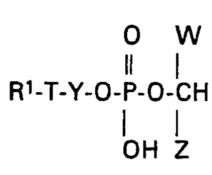

(I)

dans laquelle

R représente un groupe alcanoyle en $C_3$-$C_{14}$, un groupe benzoyle, le radical acyle d'un acide alpha-aminocarboxylique autre que la glycine, la L-alanine et leurs dérivés à groupe amino substitué, et dont le groupe alpha-amino peut être substitué par un groupe alcanoyle inférieur, (alcoxy inférieur)-carbonyle ou benzyloxycarbonyle, ou bien le radical acyle d'un acide bêta-aminocarboxylique ou d'un acide alpha- ou bêta-hydroxycarboxylique,

T représente le groupe NH non substitué ou substitué par un groupe alkyle inférieur, ou l'oxygène, Y représente un groupe diméthylène non substitué ou substitué par un groupe carboxy libre, éthérifié ou amidé, W représente l'hydrogène, et Z un groupe 1,2-dihydroxyéthyle, 2-hydroxyéthyle ou hydroxyméthyle dans

lequel l'un au moins des groupes hydroxy est estérifié par un acide carboxylique aliphatique en $C_8$-$C_{30}$ ou éthérifié par un alcool aliphatique en $C_8$-$C_{30}$, ou bien W et Z représentent chacun un groupe hydroxyméthyle estérifié par un acide carboxylique aliphatique en $C_8$-$C_{30}$ ou éthérifié par un alcool aliphatique en $C_8$-$C_{30}$, l'expression "inférieur" s'appliquant à des groupes qui contiennent jusqu'à 7 atomes de carbone inclus, et leurs sels.

2. Composés de formule I selon la revendication 1, dans lesquels R représente le radical acyle d'un acide alpha-aminocarboxylique autre que la glycine, la L-alanine et leurs dérivés à groupe amino substitué, et dont le groupe alpha-amino est substitué par un groupe alcanoyle inférieur, (alcoxy inférieur)-carbonyle ou benzyloxycarbonyle, et leurs sels.

3. Composés de formule I selon la revendication 1 ou 2, dans lesquels W représente l'hydrogène et Z un groupe 1,2-dihydroxyéthylène dans lequel l'un au moins des groupes hydroxy est estérifié par un acide carboxylique aliphatique en $C_8$-$C_{30}$, et leurs sels.

4. Composés de formule I selon la revendication 1, dans lesquels $R^1$ représente un groupe alpha-aminoalcanoyle inférieur autre que le groupe glycyle, le groupe L-alanyle et leurs dérivés à groupe amino substitué, qui peut être substitué par un autre groupe amino, alcanoylamino inférieur, (alcoxy inférieur)-carbonylamino ou benzyloxycarbonylamino ou par un groupe guanidino, carboxy, phényloxycarbonyle, benzyloxycarbonyle, benzhydryloxycarbonyle, (alcoxy inférieur)-carbonyle, carbamoyle, hydroxy, alcoxy inférieur, alcanoyloxy inférieur, (alcoxy inférieur)-carbonyloxy, mercapto, alkylthio inférieur, (2-amino-2-carboxyéthyl)-dithio, phényle, 4-hydroxyphényle, imidazole-4-yle ou indole-3-yle, ou un groupe pyrrolidine-alpha-carbonyle non substitué ou substitué par un groupe hydroxy, alcoxy inférieur, alcanoyloxy inférieur ou (alcoxy inférieur)-carbonyloxy, T représente le groupe NH ou l'oxygène, Y représente un groupe diméthylène non substitué ou substitué par un groupe carboxy, (alcoxy inférieur)-carbonyle ou carbamoyle, W représente l'hydrogène et Z un groupe 1,2-dihydroxyéthyle dans lequel le groupe 2-hydroxy, seul, ou les deux groupes hydroxy, sont estérifiés par un acide alcanoïque ou alcénoïque en $C_{12}$-$C_{24}$, ou bien W et Z représentent chacun un groupe hydroxyméthyle estérifié par un acide alcanoïque ou alcénoïque en $C_{12}$-$C_{24}$, et leurs sels.

5. Composés de formule I selon la revendication 1 ou 3, dans lesquels R est le radical acyle, autre qu'un radical glycyle, L-alanyle et leurs dérivés à groupe amino substitué, d'un acide alcanoïque contenant jusqu'à 12 atomes de carbone substitué par un groupe alpha-amino et le cas échéant un autre groupe amino, un ou deux groupes carboxy, hydroxy, mercapto, alkylthio inférieurs, phényle, 4-hydroxyphényle, carbamoyle, guanidino, 3-indolyle, 4-imidazolyle ou (2-amino-2-carboxyéthyl)-dithio, ou un radical benzoyle ou prolyle, et leurs sels.

6. Composés de formule I selon la revendication 1 ou 3, dans lesquels $R^1$ représente le radical acyle de l'un des 20 aminoacides rencontrés régulièrement dans les protéines, à l'exception de la glycine et de la L-alanine, ou le radical acyle de l'acide lactique ou de l'acide citrique, et leurs sels.

7. Composés de formule I selon la revendication 1, dans lesquels $R^1$ représente un groupe benzoyle ou le radical acyle de la L-tyrosine, de la L-lysine, de l'acide L-aspartique, de la L-asparagine, de la L-histidine, de l'acide L-glutamique, de la L-sérine, de la L-proline, de la L-leucine ou de la D-alanine, les groupes amino présents dans ces radicaux acyle pouvant être substitués à l'azote par un groupe tert.-butyloxycarbonyle, benzyloxycarbonyle ou alcanoyle inférieur, et les groupes carboxy présents dans ces radicaux acyle pouvant être estérifiés par un alcanol inférieur, T représente le groupe NH, Y représente un groupe diméthylène non substitué ou substitué par un groupe carboxy ou (alcoxy inférieur)carbonyle, W représente l'hydrogène et Z un groupe 1,2-dihydroxyéthyle, 2-hydroxyéthyle ou hydroxyméthyle dans lequel l'un au moins des groupes hydroxy est estérifié par un acide alcanoïque en $C_{10}$-$C_{18}$ ou un acide alcénoïque en $C_{18}$, ou bien W et Z représentent chacun un groupe hydroxyméthyle qui est estérifié par un acide alcanoïque en $C_{10}$-$C_{18}$ ou un acide alcénoïque en $C_{18}$, et leurs sels.

8. Composés de formule I selon la revendication 7, dans lesquelle $R^1$ représente le radical acyle de la L-tyrosine, de la L-lysine, de L-aspartique de la L-asparagine, de la L-histidine, de l'acide L-glutamique, de la L-sérine, de la L-proline, de la L-leucine ou de la D-alanine, les groupes amino présents dans ces radicaux acyle étant substitués à l'azote par un groupe tert.-butyloxycarbonyle, benzyloxycarbonyle ou alcanoyle inférieur, et leurs sels.

9. Composés de formule I selon la revendication 1, dans lesquels $R^1$ représente le radical acyle de la L-valine, de la L-sérine, de la L-thréonine, de la L-cystéine, de la L-méthionine, de la L-tyrosine ou de l'acide citrique, T représente le groupe NH, Y représente un groupe diméthylène non substitué ou substitué par un groupe carboxy, W représente l'hydrogène et Z un groupe 1,2-dihydroxyéthyle dans lequel les deux groupes hydroxy sont estérifiés par un acide alcanoïque ou alcénoïque à chaîne droite et à nombre pair d'atomes de carbone en $C_{16}$-$C_{24}$, et leurs sels.

10. Composés de formule I selon l'une des revendications 1 à 8, dans lesquels Y représente un groupe diméthylène substitué par un groupe carboxy ou (alcoxy inférieur)-carbonyle, et leurs sels.

11. Composés de formule I selon l'une des revendications 1, 2 et 4 à 8, dans lesquels W et Z représentent chacun un groupe hydroxyméthyle qui est estérifié par un acide alcénoïque en $C_{18}$ ou par un acide alcanoïque à chaîne droite en $C_{12}$-$C_{18}$, et leurs sels.

12. Composés de formule I selon l'une des revendications 1 à 11, dans lesquels T représente le groupe NH, et leurs sels.

13. Composés de formule I selon l'une des revendications 1 à 9, 11 et 12, dans lesquels Y représente le groupe diméthylène non substitué, et leurs sels.

14. Composés de formule I selon l'une des revendications 1 à 13, à l'état de sels acceptables pour l'usage pharmaceutique.

15. Composés de formule I selon l'une des rerevendications 1, 3, 4 et 10 à 13, dans lesquels $R^1$ représente le radical acyle de la L-tyrosine ou d'une N-(alcoxy inférieur)-carbonyl- ou d'une N-(alcanoyle inférieur)-L-tyrosine, et leurs sels acceptables pour l'usage pharmaceutique.

16. Composés de formule I selon l'une des revendications 1, 3, 4 et 10 à 13, dans lesquels $R^1$ représente le radical acyle de la L-leucine ou d'une N-(alcoxy inférieur)-carbonyl- ou d'une N-(alcanoyle inférieur)-L-leucine, et leurs sels acceptables pour l'usage pharmaceutique.

17. Composés de formule I selon l'une des revendications 1, 3, 4 et 10 à 13, dans lesquels $R^1$ représente le radical acyle de la L-lysine ou d'une N-(alcoxy inférieur)-carbonyl- ou d'une N-(alcanoyle inférieur)-L-tyrosine, et le leurs sels acceptables pour l'usage pharmaceutique.

18. Le L-tyrosine-2-(1,2-dipalmitoyl-sn-glycéro-3-hydroxyphosphoryloxy)-éthylamide ou un sel de ce composé acceptable pour l'usage pharmaceutique, selon la revendication 1.

19. Le 2-(1,2-dipalmitoyl-sn-glycéro-3-hydroxy-phosphoryloxy)-éthylamide de l'acide 3,4-dicarboxy-3-hydroxy-butyrique ou un sel acceptable pour l'usage pharmaceutique de ce composé, selon la revendication 1.

20. Un composé selon la revendication 1, choisi dans le groupe consistant en le L-lysine-2-[1,2-dipalmitoyl-sn-glycéro-3-hydroxyphosphoryloxy]-éthylamide, le $N^{alpha}$-benzyloxycarbonyl-$N^{epsilon}$-tert.-butyloxycarbonyl-L-lysine-2-(1,2-dipalmitoyl-sn-glycéro-3-hydroxyphosphoryloxy)-éthylamide, le N-Boc-L-tyrosine-2-(1,2-dipalmitoyl-sn-glycéro-3-hydroxyphosphoryloxy)-éthylamide, l'ester alpha-benzylique-2-(1,2-dipalmitoyl-sn-glycéro-3-hydroxyphosphoryloxy)-éthylamide de l'acide N-benzyloxycarbonyl-L-aspartique, le bêta-2-(1,2-dipalmitoyl-sn-glycéro-3-hydroxyphosphoryloxy)-éthylamide de l'acide L-aspartique, le N-benzyloxycarbonyl-L-asparagine-alpha-2-(1,2-dilauroyl-sn-glycéro-3-hydroxyphosphoryloxy)-éthylamide, le L-asparagine-alpha-2-(1,2-dilauroyl-sn-glycéro-3-hydroxyphosphoryloxy)-éthylamide, le N-Boc-D-alanine-2-(1,2-dipalmitoyl-sn-glycéro-3-hydroxyphosphoryloxy)-éthylamide, le D-alanine-2-(1,2-dipalmitoyl-sn-glycéro-3-hydroxyphosphoryloxy)-éthylamide, le N-Boc-D-alanine-2-(1,2-dipalmitoyl-sn-glycéro-3-hydroxyphosphoryloxy)-éthylamide, le D-alanine-2-(1,2-dipalmitoyl-sn-glycéro-3-hydroxyphosphoryloxy)-éthylamide, le $N^{alpha}$, $N^{im}$-di-Boc-L-histidine-2-(1,2-didecanoyl-sn-glycero-3-hydroxyphosphoryloxy)-éthylamide, le L-histidine-2-(1,2-didécanoyl-sn-glycéro-3-hydroxyphosphoryloxy)-éthylamide, l'ester gamma-tert.-butylique-alpha-2-(1,3-dipalmitoyl-glycéro-2-hydroxyphosphoryloxy)-éthylamide de l'acide N-benzyloxycarbonyl-L-glutamique, l'alpha-2-(1,3-dipalmitoyl-glycéro-2-hydroxyphosphoryloxy)-éthylamide de l'acide L-glutamique, l'ester alpha-tert.-butylique-gamma-2-(1,3-dilauroyl-glycéro-2-hydroxyphosphoryloxy)-éthylamide de l'acide N-benzyloxycarbonyl-L-glutamique, le gamma-2-(1,3-dilauroyl-glycéro-2-hydroxyphosphoryloxy)-éthylamide de l'acide L-glutamique, l'ester gamma-méthylique-alpha-2-(1,2-dipalmitoyl-sn-glycéro-3-hydroxyphosphoryloxy)-éthylamide de l'acide N-benzyloxycarbonyl-L-glutamique, l'ester gamma-méthylique-alpha-2-(1,2-dipalmitoyl-sn-glycéro-3-hydroxyphosphoryloxy)-éthylamide de l'acide L-glutamique, le N-Boc-tyrosine-2-(1-palmitoyloxypropyl-3-oxyhydroxyphosphoryloxy)-éthylamide, le L-tyrosine-2-(1-palmitoyloxy-propyl-3-oxyhydroxyphosphoryloxy)-éthylamide, le N-Boc-L-sérine-2-(1,2-dihexadécyl-sn-glycéro-3-hydroxyphosphoryloxy)-éthylamide, le L-sérine-2-(1,2-dihexadécyl-sn-glycéro-3-hydroxyphosphoryloxy)éthylamide, le N-Boc-L-proline-2-(1,2-didécanoyl-sn-glycéro-3-hydroxyphosphoryloxy)-éthylamide, le L-proline-2-(1,2-didécanoyl-sn-glycéro-3-hydroxyphosphoryloxy)-éthylamide, la N-acétyl-L-leucyl-O-(1,3-dilauroyl-glycéro-2-hydroxyphosphoryl)-L-sérine, la N-Boc-L-leucyl-O-(1,2-dioléoyl-sn-glycéro-3-hydroxyphosphoryl)-L-sérine, la L-leucine-O-(1,2-dioléoyl-sn-glycéro-3-hydroxyphosphoryl)-L-sérine ou un sel de l'un de ces composés acceptable pour l'usage pharmaceutique.

21. Dérivés phosphatidyliques de formule Ia

```
              O   W
              ‖   |
R1a-T-Y-O-P-O-CH                    (Ia)
              |   |
              OH  Z
```

dans laquelle

$R^{1a}$ représente un groupe alcanoyle en $C_2$-$C_{22}$ qui peut être substitué par un groupe carboxy, un groupe propénoyle, 2-méthylpropénoyle, benzoyle, le radical acyle d'un acide alpha-aminocarboxylique dont le groupe alpha-amino peut être substitué par un groupe alkyle inférieur, alcanoyle inférieur, (alcoxy inférieur)carbonyle ou benzyloxycarbonyle, ou le radical acyle d'un acide bêta-aminocarboxylique ou d'un acide alphaou bêta-hydroxycarboxylique,

T représente le groupe NH non substitué ou substitué par un groupe alkyle inférieur, ou l'oxygène,

Y représente un groupe diméthylène non substitué ou substitué par un groupe carboxy libre, éthérifié ou amidé,

W représente l'hydrogène et

Z un groupe 1,2-dihydroxyéthyle, 2-hydroxyéthyle ou hydroxyméthyle dans lequel l'un au moins des groupes hydroxy est estérifié par un acide carboxylique aliphatique en $C_8$-$C_{30}$ ou éthérifié par un alcool aliphatique en $C_8$-$C_{30}$, ou bien W et Z représentent chacun un groupe hydroxyméthyle qui est estérifié par un acide carboxylique aliphatique en $C_8$-$C_{30}$ ou éthérifié par un alcool aliphatique en $C_8$-$C_{30}$, l'expression "inférieur" s'appliquant à des groupes qui contiennent jusqu'à 7 atomes de carbone inclus, et leurs sels acceptables pour l'usage pharmaceutique pour l'utilisation dans un procédé pour le traitement thérapeutique de l'organisme humain ou animal.

22. Le L-alanine-2-(1,2-dipalmitoyl-sn-glycéro-3-hydroxyphosphoryloxy)-éthylamide ou un sel acceptable pour l'usage pharmaceutique de ce composé, selon la revendication 21, pour l'utilisation dans un procédé pour le traitement thérapeutique de l'organisme humain ou animal.

23. Un composé de formule I selon l'une des revendications 1 à 20, ou un sel d'un tel composé acceptable pour l'usage pharmaceutique, pour l'utilisation dans un procédé pour le traitement thérapeutique de l'organisme humain ou animal.

24. Composition pharmaceutique contenant en tant que substance active une quantité efficace pour la prophylaxie ou la thérapie d'une infection virale, d'un composé de formule I selon l'une des revendications 1 à 20 ou d'un sel d'un tel composé acceptable pour l'usage pharmaceutique, avec une quantité notable d'un véhicule pharmaceutique.

25. Utilisation d'un composé de formule Ia

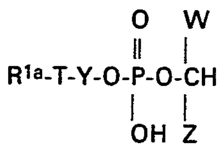

(Ia)

dans laquelle

$R^{1a}$ représente un groupe alcanoyle en $C_2$-$C_{22}$ qui peut être substitué par un groupe carboxy, un groupe alcénoyle en $C_3$-$C_{18}$, un groupe benzoyle, le radical acyle d'un acide alpha-aminocarboxylique dont le groupe alpha-amino peut être substitué par un groupe alkyle inférieur, alcanoyle inférieur, (alcoxy inférieur)-carbonyle ou benzyloxycarbonyle, ou le radical acyle d'un acide bêta-aminocarboxylique ou d'un acide alpha- ou bêta-hydroxycarboxylique,

T représente le groupe NH non substitué ou substitué par un groupe alkyle inférieur, ou l'oxygène,

Y représente un groupe diméthylène non substitué ou substitué par un groupe carboxy libre, éthérifié ou amidé,

W représente l'hydrogène et

Z un groupe 1,2-dihydroxyéthyle, 2-hydroxyéthyle ou hydroxyméthyle dans lequel l'un au moins des groupes hydroxy est estérifié par un acide carboxylique aliphatique en $C_8$-$C_{30}$ ou éthérifié par un alcool aliphatique en $C_8$-$C_{30}$, ou bien W et Z représentent chacun un groupe hydroxyméthyle estérifié par un acide carboxylique aliphatique en $C_8$-$C_{30}$ ou éthérifié par un alcool aliphatique en $C_8$-$C_{30}$, l'expression "inférieur" s'appliquant à des groupes qui contiennent jusqu'à 7 atomes de carbone inclus, ou d'un sel d'un tel composé acceptable pour l'usage pharmaceutique, pour la préparation d'un médicament pour l'utilisation pour la prophylaxie ou la thérapie d'une infection virale.

26. Utilisation d'un composé de formule I selon l'une des revendications 1 à 20, ou d'un sel d'un tel composé acceptable pour l'usage pharmaceutique pour la préparation d'un médicament pour l'utilisation pour la prophylaxie ou la thérapie d'une infection virale.

27. Procédé de préparation d'un composé de formule I

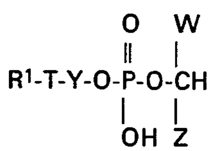

(I)

dans laquelle

$R^1$ représente un groupe alcanoyle en $C_3$-$C_{14}$, benzoyle, le radical acyle d'un acide alpha-aminocarboxylique autre que la glycine, la L-alanine et leurs dérivés à groupe amino substitué, et dont le groupe alpha-amino peut être substitué par un groupe alcanoyle inférieur, (alcoxy inférieur)-carbonyle ou benzyloxycarbonyle, ou le radical acyle d'un acide bêta-aminocarboxylique ou d'un acide alpha- ou bêta-hydroxycarboxylique,

T représente le groupe NH non substitué ou substitué par un groupe alkyle inférieur, ou l'oxygène,

Y représente un groupe diméthylène non substitué ou substitué par un groupe carboxy libre, éthérifié ou amidé,

W représente l'hydrogène et

Z un groupe 1,2-dihydroxyéthyle, 2-hydroxyéthyle ou hydroxyméthyle dans lequel l'un au moins des groupes hydroxy est estérifié par un acide carboxylique aliphatique en $C_8$-$C_{30}$ ou éthérifié par un alcool aliphatique en $C_8$-$C_{30}$, ou bien W et Z représentent chacun un groupe hydroxyméthyle estérifié par un acide aliphatique carboxylique en $C_8$-$C_{30}$ ou éthérifié par un alcool aliphatique en $C_8$-$C_{30}$, l'expression "inférieur" s'appliquant à des groupes qui contiennent jusqu'à 7 atomes de carbone inclus, ou d'un sel d'un tel composé, caractérisé en ce que:

a) on fait réagir un composé de formule II

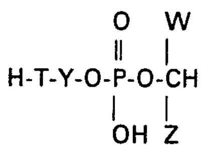

(II)

dans laquelle les symboles ont les significations indiquées ci-dessus, les groupes fonctionnels présents dans un composé de formule II, à l'exception du groupe participant à la réaction, étant à l'état protégé lorsque c'est nécessaire, ou un dérivé réactif d'un tel composé, avec un composé de formule III

$R^1$ - OH (III)

dans laquelle $R^1$ a les significations indiquées ci-dessus, les groupes fonctionnels présents dans un composé de formule III, à l'exception du groupe participant à la réaction, étant lorsque c'est nécessaire à l'état protégé, ou avec un dérivé réactif d'un acide carboxylique et d'un tel composé, et on élimine les groupes protecteurs présents, ou bien

b) on fait réagir un composé de formule IV

FIG00/25

dans laquelle w est égal à 0 ou 1 et $R^1$, T et Y ont les significations indiquées ci-dessus, les groupes fonctionnels présents dans un composé de formule IV, à l'exception du groupe participant à la réaction, étant à l'état protégé lorsque c'est nécessaire, ou un dérivé réactif d'un tel composé, avec un composé de formule V

FIG00/25

dans laquelle W et Z ont les significations indiquées ci-dessus, les groupes fonctionnels présents dans un composé de formule V, à l'exception du groupe participant à la réaction, étant à l'état protégé lorsque c'est nécessaire, et m est égal à 1 lorsque, dans l'autre réactif de formule IV, w est égal à 0, ou m est égal à 0 lorsque w est égal à 1, ou avec un dérivé réactif d'un composé de formule V, et on élimine les groupes protecteurs présents, ou bien

c) on oxyde un composé de formule VI

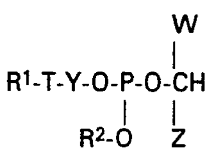

(VI)

dans laquelle $R^2$ représente l'hydrogène ou un groupe protecteur et les autres symboles ont les significations indiquées ci-dessus, les groupes fonctionnels présents dans un composé de formule VI, à l'exception du groupe participant à la réaction, étant à l'état protégé lorsque c'est nécessaire, ou bien un tautomère d'un composé de formule VI, à l'aide d'un agent oxydant, et on élimine les groupes protecteurs présents, ou bien

d) on hydrolyse un composé de formule VII

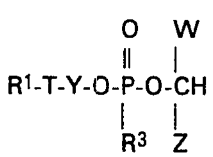

(VII)

dans laquelle $R^3$ représente un halogène et les autres symboles ont les significations indiquées ci-dessus, les groupes fonctionnels présents dans un composé de formule VII, à l'exception du groupe participant à la réaction, étant à l'état protégé lorsque c'est nécessaire, et on élimine les groupes protecteurs présents, ou bien

e) on part d'un composé de formule VIII

```
        O   W'
        ‖   |
R1-T-Y-O-P-O-CH                    (VIII)
        |   |
        OH  Z'
```

dans laquelle W' représente l'hydrogène et Z' un groupe 1,2-dihydroxyéthyle, 2-hydroxyéthyle, hydroxyméthyle ou 1,2-dihydroxyéthyle dans lequel l'un des deux groupes hydroxy est estérifié par un acide carboxylique aliphatique en $C_8$-$C_{30}$ ou éthérifié par un alcool aliphatique en $C_8$-$C_{30}$, ou bien l'un des symboles W' et Z' représente un groupe hydroxyméthyle et l'autre un groupe hydroxyméthyle libre ou un groupe hydroxyméthyle estérifié par un acide carboxylique aliphatique en $C_8$-$C_{30}$ ou éthérifié par un alcool aliphatique en $C_8$-$C_{30}$, et $R^1$, T et Y ont les significations indiquées ci-dessus, les groupes fonctionnels présents dans un composé de formule VIII, à l'exception du groupe participant à la réaction, étant à l'état protégé lorsque c'est nécessaire, ou d'un dérivé réactif d'un tel composé, on l'estérifie par un acide carboxylique aliphatique en $C_8$-$C_{30}$ ou un dérivé réactif d'un tel acide, ou bien on l'éthérifie par un alcool aliphatique en $C_8$-$C_{30}$ ou un dérivé réactif d'un tel alcool, et on élimine les groupes protecteurs présents, ou bien

f) on fait réagir un composé de formule IX

```
      O   W
      ‖   |
X-Y-O-P-O-CH                       (IX)
      |   |
      OH  Z
```

dans laquelle X représente un groupe éliminable nucléophile et les autres symboles ont les significations indiquées ci-dessus, les groupes fonctionnels présents dans un composé de formule IX, à l'exception du groupe participant à la réaction, étant à l'état protégé lorsque c'est nécessaire, avec un composé de formule X

$R^1$-T-H (X)

dans laquelle les symboles ont les significations indiquées ci-dessus, les groupes fonctionnels présents dans un composé de formule X, à l'exception du groupe participant à la réaction, étant à l'état protégé lorsque c'est nécessaire, ou avec un dérivé réactif d'un tel composé, et on élimine les groupes protecteurs présents, ou bien

g) dans un composé de formule I dans laquelle les symboles ont les significations indiquées ci-dessus, au moins un groupe fonctionnel d'un composé de formule I devant être protégé par un groupe protecteur facile à éliminer, on élimine le ou les groupes protecteurs, ou bien

h) pour préparer un composé de formule I dans laquelle W représente l'hydrogène et Z un groupe 1,2-dihydroxyéthyle dans lequel le groupe 2-hydroxy est estérifié par un acide carboxylique aliphatique en $C_8$-$C_{30}$, on fait réagir un composé de formule I dans laquelle W représente l'hydrogène et Z un groupe 1,2-dihydroxyéthyle dans lequel les deux groupes hydroxy sont estérifiés par un acide carboxylique aliphatique en $C_8$-$C_{30}$, avec une enzyme convenant pour l'élimination toposélective du radical estérifiant le groupe 1-hydroxy, et après exécution de l'une des variantes opératoires a) à h) ci-dessus, on convertit lorsque c'est nécessaire, pour la préparation d'un sel, un composé de formule I en un sel, ou bien pour convertir un composé de formule I ou l'un de ses sels en un autre composé de formule I ou en l'un de ses sels, on acyle un groupe amino ou hydroxy contenu dans le groupe $R^1$ ou bien on estérifie ou on amide un groupe carboxyle contenu dans le groupe $R^1$ ou dans le groupe Y.

**Revendications** pour l'Etat Contractant: AT

1. Procédé de préparation d'un composé de formule I

```
        O   W
        ‖   |
R1-T-Y-O-P-O-CH                    (I)
        |   |
        OH  Z
```

dans laquelle

$R^1$ représente un groupe alcanoyle en $C_3$-$C_{14}$, benzoyle, le radical acyle d'un acide alpha-aminocarboxylique autre que la glycine, la L-alanine et leurs dérivés à groupe amino substitué, et dont le groupe alpha-amino peut être substitué par un groupe alcanoyle inférieur, (alcoxy inférieur)-carbonyle ou benzyloxycarbonyle, ou le radical acyle d'un acide bêta-aminocarboxylique ou d'un acide alpha- ou bêta-hydroxycarboxylique,

T représente le groupe NH non substitué ou substitué par un groupe alkyle inférieur, ou l'oxygène,

Y représente un groupe diméthylène non substitué ou substitué par un groupe carboxy libre, éthérifié ou amidé,

W représente l'hydrogène et

Z un groupe 1,2-dihydroxyéthyle, 2-hydroxyéthyle ou hydroxyméthyle dans lequel l'un au moins des groupes hydroxy est estérifié par un acide carboxylique aliphatique en $C_8$-$C_{30}$ ou éthérifié par un alcool aliphatique en $C_8$-$C_{30}$, ou bien W et Z représentent chacun un groupe hydroxyméthyle estérifié par un acide aliphatique carboxylique en $C_8$-$C_{30}$ ou éthérifié par un alcool aliphatique en $C_8$-$C_{30}$, l'expression "inférieur" s'appliquant à des groupes qui contiennent jusqu'à 7 atomes de carbone inclus, ou d'un sel d'un tel composé, caractérisé en ce que:

a) on fait réagir un composé de formule II

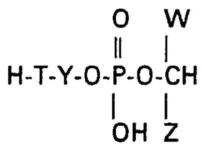

(II)

dans laquelle les symboles ont les significations indiquées ci-dessus, les groupes fonctionnels présents dans un composé de formule II, à l'exception du groupe participant à la réaction, étant à l'état protégé lorsque c'est nécessaire, ou un dérivé réactif d'un tel composé, avec un composé de formule III

$R^1$ - OH (III)

dans laquelle $R^1$ a les significations indiquées ci-dessus, les groupes fonctionnels présents dans un composé de formule III, à l'exception du groupe participant à la réaction, étant lorsque c'est nécessaire à l'état protégé, ou avec un dérivé réactif d'un acide carboxylique et d'un tel composé, et on élimine les groupes protecteurs présents, ou bien

b) on fait réagir un composé de formule IV

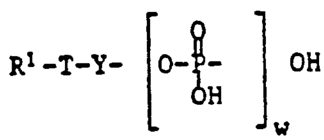

(IV)

dans laquelle

w est égal à 0 ou 1 et

$R^1$, T et Y ont les significations indiquées ci-dessus, les groupes fonctionnels présents dans un composé de formule IV, à l'exception du groupe participant à la réaction, étant à l'état protégé lorsque c'est nécessaire, ou un dérivé réactif d'un tel composé, avec un composé de formule V

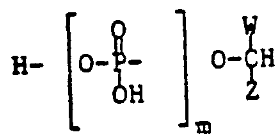

(V)

dans laquelle W et Z ont les significations indiquées ci-dessus, les groupes fonctionnels présents dans un composé de formule V, à l'exception du groupe participant à la réaction, étant à l'état protégé lorsque c'est nécessaire, et m est égal à 1 lorsque, dans l'autre réactif de formule IV, w est égal à 0, ou m est égal à 0 lorsque w est égal à 1, ou avec un dérivé réactif d'un composé de formule V, et on élimine les groupes protecteurs présents, ou bien

c) on oxyde un composé de formule VI

```
               W
               |
R1-T-Y-O-P-O-CH                                  (VI)
         |   |
      R2-O   Z
```

dans laquelle $R^2$ représente l'hydrogène ou un groupe protecteur et les autres symboles ont les significations indiquées ci-dessus, les groupes fonctionnels présents dans un composé de formule VI, à l'exception du groupe participant à la réaction, étant à l'état protégé lorsque c'est nécessaire, ou bien un tautomère d'un composé de formule VI, à l'aide d'un agent oxydant, et on élimine les groupes protecteurs présents, ou bien

d) on hydrolyse un composé de formule VII

```
          O   W
          ||  |
R1-T-Y-O-P-O-CH                                  (VII)
          |   |
          R3  Z
```

dans laquelle $R^3$ représente un halogène et les autres symboles ont les significations indiquées ci-dessus, les groupes fonctionnels présents dans un composé de formule VII, à l'exception du groupe participant à la réaction, étant à l'état protégé lorsque c'est nécessaire, et on élimine les groupes protecteurs présents, ou bien

e) on part d'un composé de formule VIII

```
          O   W'
          ||  |
R1-T-Y-O-P-O-CH                                  (VIII)
          |   |
          OH  Z'
```

dans laquelle W' représente l'hydrogène et Z' un groupe 1,2-dihydroxyéthyle, 2-hydroxyéthyle, hydroxyméthyle ou 1,2-dihydroxyéthyle dans lequel l'un des deux groupes hydroxy est estérifié par un acide carboxylique aliphatique en $C_8$-$C_{30}$ ou éthérifié par un alcool aliphatique en $C_8$-$C_{30}$, ou bien l'un des symboles W' et Z' représente un groupe hydroxyméthyle et l'autre un groupe hydroxyméthyle libre ou un groupe hydroxyméthyle estérifié par un acide carboxylique aliphatique en $C_8$-$C_{30}$ ou éthérifié par un alcool aliphatique en $C_8$-$C_{30}$, et $R^1$, T et Y ont les significations indiquées ci-dessus, les groupes fonctionnels présents dans un composé de formule VIII, à l'exception du groupe participant à la réaction, étant à l'état protégé lorsque c'est nécessaire, ou d'un dérivé réactif d'un tel composé, on l'estérifie par un acide carboxylique aliphatique en $C_8$-$C_{30}$ ou un dérivé réactif d'un tel acide, ou bien on l'éthérifie par un alcool aliphatique en $C_8$-$C_{30}$ ou un dérivé réactif d'un tel alcool, et on élimine les groupes protecteurs présents, ou bien

f) on fait réagir un composé de formule IX

```
      O   W
      ||  |
X-Y-O-P-O-CH                                     (IX)
      |   |
      OH  Z
```

dans laquelle X représente un groupe éliminable nucléophile et les autres symboles ont les significations indiquées ci-dessus, les groupes fonctionnels présents dans un composé de formule IX, à l'exception du groupe participant à la réaction, étant à l'état protégé lorsque c'est nécessaire, avec un composé de formule

$R^1$ - T - H (X)

dans laquelle les symboles ont les significations indiquées ci-dessus, les groupes fonctionnels présents dans un composé de formule X, à l'exception du groupe participant à la réaction, étant à l'état protégé lorsque c'est nécessaire, ou avec un dérivé réactif d'un tel composé, et on élimine les groupes protecteurs présents, ou bien

g) dans un composé de formule I dans laquelle les symboles ont les significations indiquées ci-dessus, au moins un groupe fonctionnel d'un composé de formule I devant être protégé par un groupe protecteur facile à éliminer, on élimine le ou les groupes protecteurs, ou bien

h) pour préparer un composé de formule I dans laquelle W représente l'hydrogène et Z un groupe 1,2-dihydroxyéthyle dans lequel le groupe 2-hydroxy est estérifié par un acide carboxylique aliphatique en $C_8$-$C_{30}$, on fait réagir un composé de formule I dans laquelle W représente l'hydrogène et Z un groupe 1,2-dihydroxyéthyle dans lequel les deux groupes hydroxy sont estérifiés par un acide carboxylique aliphatique en $C_8$-$C_{30}$, avec une enzyme convenant pour l'élimination toposélective du radical estérifiant le groupe 1-hydroxy, et après exécution de l'une des variantes opératoires a) à h) ci-dessus, on convertit lorsque c'est nécessaire, pour la préparation d'un sel, un composé de formule I en un sel, ou bien pour convertir un composé de formule I ou l'un de ses sels en un autre composé de formule I ou en l'un de ses sels, on acyle un groupe amino ou hydroxy contenu dans le groupe $R^1$ ou bien on estérifie ou on amide un groupe carboxyle contenu dans le groupe $R^1$ ou dans le groupe Y.

2. Procédé selon la revendication 1, caractérisé en ce que l'on choisit les composés de départ de manière à préparer un composé de formule I ou un sel d'un tel composé dans lequel $R^1$ représente le radical acyle d'un acide alpha-aminocarboxylique autre que la glycine, la L-alanine et leurs dérivés à groupe amino substitué, et dont le groupe alpha-amino est substitué par un groupe alcanoyle inférieur, (alcoxy inférieur)-carbonyle ou benzyloxycarbonyle.

3. Procédé selon la revendication 1 ou 2, caractérisé en ce que l'on choisit les composés de départ de manière à préparer un composé de formule I ou un sel d'un tel composé dans lequel W représente l'hydrogène et Z un groupe 1,2-dihydroxyéthyle dans lequel l'un au moins des groupes hydroxy est estérifié par un acide carboxylique aliphatique en $C_8$-$C_{30}$.

4. Procédé selon la revendication 1, caractérisé en ce que l'on choisit les composés de départ de manière à préparer un composé de formule I ou un sel d'un tel composé dans lequel $R^1$ représente un groupe alpha-aminoalcanoyle inférieur autre que le groupe glycyle, le groupe L-alanyle et leurs dérivés à groupe amino substitué, qui peut être substitué par un autre groupe amino, alcanoylamino inférieur, (alcoxy inférieur)-carbonylamino ou benzyloxycarbonylamino ou par un groupe guanidino, carboxy, phényloxycarbonyle, benzyloxycarbonyle, benzhydryloxycarbonyle, (alcoxy inférieur)-carbonyle, carbamoyle, hydroxy, alcoxy inférieur, alcanoyloxy inférieur, (alcoxy inférieur)-carbonyloxy, mercapto, alkylthio inférieur, (2-amino-2-carboxyéthyl)-dithio, phényle, 4-hydroxyphényle, imidazole-4-yle ou indole-3-yle, ou un groupe pyrrolidine-alpha-carbonyle non substitué ou substitué par un groupe hydroxy, alcoxy inférieur, alcanoyloxy inférieur ou (alcoxy inférieur)-carbonyloxy, T représente le groupe NH ou l'oxygène, Y représente un groupe diméthylène non substitué ou substitué par un groupe carboxy, (alcoxy inférieur)-carbonyle ou carbamoyle, W représente l'hydrogène et Z un groupe 1,2-dihydroxyéthyle dans lequel le groupe 2-hydroxy, seul, ou les deux groupes hydroxy, sont estérifiés par un acide alcanoïque ou alcénoïque en $C_{12}$-$C_{24}$, ou bien W et Z représentent chacun un groupe hydroxyméthyle estérifié par un acide alcanoïque ou alcénoïque en $C_{12}$-$C_{24}$.

5. Procédé selon la revendication 1 ou 3, caractérisé en ce que l'on choisit les composés de départ de manière à préparer un composé de formule I ou l'un de ses sels dans lequel $R^1$ représente le radical acyle, autre qu'un radical glycyle, L-alanyle et leurs dérivés à groupe amino substitué, d'un acide alcanoïque contenant jusqu'à 12 atomes de carbone substitué par un groupe alpha-amino et le cas échéant un autre groupe amino, un ou deux groupes carboxy, hydroxy, mercapto, alkylthio inférieurs, phényle, 4-hydroxyphényle, carbamoyle, guanidino, 3-indolyle, 4-imidazolyle ou (2-amino-2-carboxyéthyl)-dithio ou un radical benzoyle ou prolyle.

6. Procédé selon la revendication 1 ou 3, caractérisé en ce que l'on choisit les composés de départ de manière à préparer un composé de formule I ou un sel d'un tel composé dans lequel $R^1$ représente le radical acyle de l'un des vingt aminoacides rencontrés régulièrement dans les protéines, à l'exception de la glycine et de la L-alanine, ou le radical acyle de l'acide lactique ou de l'acide citrique.

7. Procédé selon la revendication 1, caractérisé en ce que l'on choisit les composés de départ de manière à préparer un composé de formule I ou un sel d'un tel composé dans lesquels $R^1$ représente un groupe benzoyle ou le radical acyle de la L-tyrosine, de la L-lysine, de l'acide L-aspartique, de la L-asparagine, de la L-histidine, de l'acide L-glutamique, de la L-sérine, de la L-proline, de la L-leucine ou de la D-alanine, les groupes amino présents dans ces radicaux acyle pouvant être substitués à l'azote par un groupe tert.-butyloxycarbonyle, benzyloxycarbonyle ou alcanoyle inférieur, et les groupes carboxy présents dans ces radicaux acyle pouvant être estérifiés par un alcanol inférieur, T représente le groupe NH, Y représente un groupe diméthylène non substitué ou substitué par un groupe carboxy ou (alcoxy inférieur)-carbonyle, W représente l'hydrogène et Z un groupe 1,2-dihydroxyéthyle, 2-hydroxyéthyle ou hydroxyméthyle dans lequel l'un au moins des groupes hydroxy est estérifié par un acide alcanoïque en $C_{10}$-$C_{18}$ ou un acide alcénoïque en $C_{18}$, ou bien W et Z représentent chacun un groupe hydroxyméthyle qui est estérifié par un acide alcanoïque en $C_{10}$-$C_{18}$ ou un alcide alcénoïque en $C_{18}$.

8. Procédé selon la revendication 7, caractérisé en ce que l'on choisit les composés de départ de manière à préparer un composé de formule I ou un sel d'un tel composé dans lesquels $R^1$ représente le radical acyle de la L-tyrosine, de la L-lysine, de l'acide L-aspartique, de la L-asparagine, de la L-histidine, de l'acide L-glutamique, de la L-sérine, de la L-proline, de la L-leucine ou de la D-alanine, les groupes amino présents dans ces radicaux acyle étant substitués à l'azote par un groupe tert.-butyloxycarbonyle, benzyloxycarbonyle ou alcanoyle inférieur.

9. Procédé selon la revendication 1, caractérisé en ce que l'on choisit les composés de départ de manière à préparer un composé de formule I ou un sel d'un tel composé dans lesquels $R^1$ représente le radical acyle de la L-valine, de la L-sérine, de la L-thréonine, de la L-cystéine, de la L-méthionine, de la L-tyrosine ou de l'acide

citrique, T représente le groupe NH, Y représente un groupe diméthylène non substitué ou substitué par un groupe carboxy, W représente l'hydrogène et Z un groupe 1,2-dihydroxyéthyle dans lequel les deux groupes hydroxy sont estérifiés par un acide alcanoïque ou alcénoïque à chaîne droite et à nombre pair d'atomes de carbone en $C_{16}$-$C_{24}$.

10. Procédé selon l'une des revendications 1 à 8, caractérisé en ce que l'on choisit les composés de départ de manière à préparer un composé de formule I ou un sel d'un tel composé dans lequel Y représente le groupe diméthylène substitué par un groupe carboxy ou (alcoxy inférieur)-carbonyle.

11. Procédé selon l'une des revendications 1, 2 et 4 à 8, caractérisé en ce que l'on choisit les composés de départ de manière à préparer un composé de formule I ou un sel d'un tel composé dans lequel W et Z représentent chacun un groupe hydroxyméthyle qui est estérifié par un acide alcénoïque en $C_{18}$ ou par un acide alcanoïque à chaîne droite en $C_{12}$-$C_{18}$.

12. Procédé selon l'une des revendications 1 à 11, caractérisé en ce que l'on choisit les composés de départ de manière à préparer un composé de formule I ou un sel d'un tel composé dans lequel T représente le groupe NH.

13. Procédé selon l'une des revendications 1 à 9, 11 et 12, caractérisé en ce que l'on choisit les composés de départ de manière à préparer un composé de formule I ou un sel d'un tel composé dans lequel Y représente le groupe diméthylène non substitué.

14. Procédé selon l'une des revendications 1 à 13, caractérisé en ce que l'on choisit les composés de départ de manière à préparer un sel d'un composé de formule I acceptable pour l'usage pharmaceutique.

15. Procédé selon l'une des revendications 1, 3, 4 et 10 à 13, caractérisé en ce que l'on choisit les composés de départ de manière à préparer un composé de formule I ou un sel d'un tel composé acceptable pour l'usage pharmaceutique dans lequel $R^1$ représente le radical acyle de la L-tyrosine ou d'une N-(alcoxy inférieur)-carbonyl- ou d'une N-(alcanoyle inférieur)-L-tyrosine.

16. Procédé selon l'une des revendications 1, 3, 4 et 10 à 13, caractérisé en ce que l'on choisit les composés de départ de manière à préparer un composé de formule I ou un sel d'un tel composé acceptable pour l'usage pharmaceutique dans lequel $R^1$ représente le radical acyle de la L-leucine ou d'une N-(alcoxy inférieur)-carbonyl- ou N-(alcanoyle inférieur)-L-leucine.

17. Procédé selon l'une des revendications 1, 3, 4 et 10 à 13, caractérisé en ce que l'on choisit les composés de départ de manière à préparer un composé de formule I ou un sel d'un tel composé acceptable pour l'usage pharmaceutique dans lequel $R^1$ représente le radical acyle de la L-lysine ou d'une $N^{alpha}$-benzyloxycarbonyl-$N^{epsilon}$-(alcoxy inférieur)-carbonyl-L-lysine.

18. Procédé selon la revendication 1, caractérisé en ce que l'on choisit les composés de départ de manière à préparer le L-tyrosine-2-(1,2-dipalmitoyl-sn-glycéro-3-hydroxyphosphoryloxy)-éthylamide ou un sel de ce composé acceptable pour l'usage pharmaceutique.

19. Procédé selon la revendication 1, caractérisé en ce que l'on choisit les composés de départ de manière à préparer le 2-(1,2-dipalmitoyl-sn-glycéro-3-hydroxyphosphoryloxy)-éthylamide de l'acide 3,4-dicarboxy-3-hydroxy-butyrique ou un sel de ce composé acceptable pour l'usage pharmaceutique.

20. Procédé selon la revendication 1, caractérisé en ce que l'on choisit les composés de départ de manière à préparer un composé choisi dans le groupe consistant en le L-lysine-2-[1,2-dipalmitoyl-sn-glycéro-3-hydroxyphosphoryloxy]-éthylamide, le $N^{alpha}$-benzyloxy-carbonyl-$N^{epsilon}$-tert.-butyloxycarbonyl-L-lysine-2-(1,2-dipalmitoyl-sn-glycéro-3-hydroxyphosphoryloxy)-éthylamide, le N-Boc-L-tyrosine-2-(1,2-dipalmitoyl-sn-glycéro-3-hydroxyphosphoryloxy)-éthylamide, l'ester alpha-benzylique-2-(1,2-dipalmitoyl-sn-glycéro-3-hydroxyphosphoryloxy)-éthylamide de l'acide N-benzyloxycarbonyl-L-aspartique, le bêta-2-(1,2-dipalmitoyl-sn-glycéro-3-hydroxyphosphoryloxy)-éthylamide de l'acide L-aspartique, le N-benzyloxycarbonyl-L-asparagine-alpha-2-(1,2-dilauroyl-sn-glycéro-3-hydroxyphosphoryloxy)-éthylamide, le L-asparagine-alpha-2-(1,2-dilauroyl-sn-glycéro-3-hydroxyphosphoryloxy)-éthylamide, le N-Boc-D-alanine-2-(1,2-dipalmitoyl-sn-glycéro-3-hydroxyphosphoryloxy)-éthylamide, le D-alanine-2-(1,2-dipalmitoyl-sn-glycéro-3-hydroxyphosphoryloxy)-éthylamide, le $N^{alpha}$,$N^{im}$-di-Boc-L-histidine-2-(1,2-didécanoyl-sn-glycéro-3-hydroxyphosphoryloxy)-éthylamide, le L-histidine-2-(1,2-didécanoyl-sn-glycéro-3-hydroxyphosphoryloxy)-éthylamide, l'ester gamma-tert.-butylique-alpha-2-(1,3-dipalmitoyl-glycéro-2-hydroxyphosphoryloxy)-éthylamide de l'acide N-benzyloxycarbonyl-L-glutamique, l'alpha-2-(1,3-dipalmitoyl-glycéro-2-hydroxyphosphoryloxy)-éthylamide de l'acide L-glutamique, l'ester alpha-tert.-butylique-gamma-2-(1,3-dilauroyl-glycéro-2-hydroxyphosphoryloxy)-éthylamide de l'acide N-benzyloxycarbonyl-L-glutamique, le gamma-2-(1,3-dilauroyl-glycéro-2-hydroxyphosphoryloxy)-éthylamide de l'acide L-glutamique, l'ester gamma-méthylique-alpha-2-(1,2-dipalmitoyl-sn-glycéro-3-hydroxyphosphoryloxy)-éthylamide de l'acide N-benzyloxycarbonyl-L-glutamique, l'ester gamma-méthylique-alpha-2-(1,2-dipalmitoyl-sn-glycéro-3-hydroxyphosphoryloxy)éthylamide de l'acide L-glutamique, le N-Boc-tyrosine-2-(1-palmitoyloxy-propyl-3-oxyhydroxyphosphoryloxy)-éthylamide, le L-tyrosine-2-(1-palmitoyloxy-propyl-3-oxyhydroxyphosphoryloxy)-éthylamide, le N-Boc-L-sérine-2-(1,2-dihexadécyl-sn-glycéro-3-hydroxyphosphoryloxy)-éthylamide, le L-sérine-2-(1,2-dihexadécyl-sn-glycéro-3-hydroxyphosphoryloxy)-éthylamide, le N-Boc-L-proline-2-(1,2-didécanoyl-sn-glycéro-3-hydroxyphosphoryloxy)-éthylamide, le L-proline-2-(1,2-didécanoyl-sn-glycéro-3-hydroxyphosphoryloxy)-éthylamide, la N-acétyl-L-leucyl-O-(1,3-dilauroylglycéro-2-hydroxyphosphoryl)-L-sérine, la N-Boc-L-leucyl-O-(1,2-dioléoyl-sn-glycéro-3-hydroxyphosphoryl)-L-sérine, la L-leucine-O-(1,2-dioléoyl-sn-glycéro-3-hydroxyphosphoryl)-L-sérine ou un sel de l'un de ces composés acceptable pour l'usage pharmaceutique.